# EUROPEAN PATENT SPECIFICATION

(11) **EP 3 079 682 B1**
(45) Date of publication and mention of the grant of the patent: **28.10.2026**
(21) Application number: 14869351.8
(22) Date of filing: 12.12.2014
(51) Int. Cl.: A61K 31/165, A61P 35/00, A61K 31/44, A61K 31/4436, A61K 45/06, A61K 31/4745, A61K 31/496, C07D 401/04, C07D 409/04, C07D 409/14, A61K 31/4439, C07D 413/04, A61P 35/02

(54) **METHODS TO TREAT LYMPHOPLASMACYTIC LYMPHOMA**
VERFAHREN ZUR BEHANDLUNG EINES LYMPHOPLASMAZYTISCHEN LYMPHOMS
PROCÉDÉ POUR TRAITER UN LYMPHOME LYMPHOPLASMOCYTAIRE

(30) Priority: 13.12.2013 US 201361915684 P; 13.08.2014 US 201462036917 P
(43) Date of publication of application: 19.10.2016
(73) Proprietor: Dana-Farber Cancer Institute, Inc., Boston, MA 02215 (US)
(72) Inventor: TREON, Steven P., Jamaica Plain, MA 02130 (US); BUHRLAGE, Sara Jean, Somerville, MA 02144 (US); GRAY, Nathanael, Boston, MA 02130 (US); TAN, Li, Boston, MA 02115 (US); YANG, Guang, Natick, MA 01760 (US)
(74) Representative: Elkington and Fife LLP
(86) International application number: PCT/US2014/070167
(87) International publication number: WO 2015/089481

(56) References cited:
- WO-A1-2006/067446
- WO-A1-2008/144253
- WO-A1-2012/161877
- WO-A2-2006/067445
- WO-A2-2008/039218
- WO-A2-2012/170976
- US-A1- 2007 060 619
- US-A1- 2007 072 862
- US-A1- 2008 269 215
- US-A1- 2013 040 949
- US-A1- 2013 040 949
- US-B1- 6 455 520
- US-B1- 6 821 965
- CHOI YONGMUN ET AL: "Discovery and structural analysis of Eph receptor tyrosine kinase inhibitors", BIOORGANIC & MEDICINAL CHEMISTRY LETTERS, vol. 19, no. 15, 13 May 2009 (2009-05-13), pages 4467 - 4470, XP029120740, ISSN: 0960-894X, DOI: 10.1016/J.BMCL.2009.05.029
- LYNE P D ET AL: "Identification of amidoheteroaryls as potent inhibitors of mutant (V600E) B-Raf kinase with in vivo activity", BIOORGANIC & MEDICINAL CHEMISTRY LETTERS, PERGAMON, AMSTERDAM, NL, vol. 19, no. 3, 1 February 2009 (2009-02-01), pages 1026 - 1029, XP025925876, ISSN: 0960-894X, [retrieved on 20081015], DOI: 10.1016/J.BMCL.2008.10.053
- CHEN N ET AL: "Discovery of a potent and selective c-Kit inhibitor for the treatment of inflammatory diseases", BIOORGANIC & MEDICINAL CHEMISTRY LETTERS, PERGAMON, AMSTERDAM, NL, vol. 18, no. 14, 15 July 2008 (2008-07-15), pages 4137 - 4141, XP022852915, ISSN: 0960-894X, [retrieved on 20080528], DOI: 10.1016/J.BMCL.2008.05.089
- GERRIT J P DIJKGRAAF ET AL: "Small Molecule Inhibition of GDC-0449 Refractory Smoothened Mutants and Downstream Mechanisms of Drug Resistance", CANCER RESEARCH, AMERICAN ASSOCIATION FOR CANCER RESEARCH, US, vol. 71, no. 2, 15 January 2011 (2011-01-15), pages 435 - 444, XP002669434, ISSN: 0008-5472, [retrieved on 20101201], DOI: 10.1158/0008-5472.CAN-10-2876
- GUANG YANG ET AL: "A mutation in MYD88 (L265P) supports the survival of lymphoplasmacytic cells by activation of Bruton tyrosine kinase in Waldenström macroglobulinemia", BLOOD, vol. 122, no. 7, 8 July 2013 (2013-07-08), US, pages 1222 - 1232, XP055281747, ISSN: 0006-4971, DOI: 10.1182/blood-2012-12-475111

## Description

### RELATED APPLICATIONS

The present application claims priority under 35 U.S.C. § 119(e) to U.S. provisional patent applications, U.S.S.N. 62/036,917, filed August 13, 2014, and U.S.S.N. 61/915,684, filed December 13, 2013.

### GOVERNMENT SUPPORT

This invention was made with U.S. Government support under grants 5R01CA130876-05, 5P50CA090578-10, 5R01CA136851-04, 2R01CA136851-05, and 1R01CA172592-01A1 awarded by the National Cancer Institute. The U.S. Government has certain rights in the invention.

### BACKGROUND OF THE INVENTION

Waldenström's macroglobulinemia (WM) is a distinct clinicopathological entity resulting from the accumulation, predominantly in the bone marrow, of clonally related lymphoplasmacytic cells which secrete a monoclonal IgM protein. This condition is considered to correspond to lymphoplasmacytic lymphoma (LPL) as defined by the World Health Organization classification system. WM is a rare disorder, with fewer than 1,500 cases occurring in the United States annually. There is a 2- to 3-fold risk increase of developing WM in people with a personal history of autoimmune diseases with autoantibodies and particularly elevated risks associated with hepatitis, human immunodeficiency virus, and rickettsiosis (Arch. Intern. Med., 2008, 168(17), 1903-9). There is no single accepted treatment for WM, and there can be a marked variation in clinical outcome. Objective response rates are high (>80%) but complete response rates are low (0-15%) (Clin. Adv. Hematol. Oncol., 2009, 7(10), 677-81, 687-90). Thus, there is a need for effective treatment of WM.

Bioorg. Med. Chem. 2009, 19(15) 4467-4470 relates to compounds which potently inhibit Eph receptor tyrosine kinases, particularly EphB2. Bioorg. Med. Chem. 2009, 19(3) 1026-1029 relates to amidoheteroaryl compounds which were designed and synthesized as inhibitors of B-Raf kinase. US6821965B1 relates to amidobenzamide derivatives which are useful cytokine inhibitors. Bioorg. & Med Chem Lett., 2008, 18(14) 4137-4141 relates to potent and selective c-Kit inhibitor. American Association for Cancer Research, 2010, 71(2), 435-444 relates to small molecule inhibitor of GDC-0449 refractory smoothened mutants and downstream mechanisms of drug resistance. US2007072862A1 relates to compounds that are capable of modulating the activity of one or more kinase enzymes, thereby regulating various kinase-associated disorders including, without limitation, inflammation and autoimmune disease. WO2006067445 relates to chemical compounds, or pharmaceutically acceptable salts thereof, which possess colony stimulating factor 1 receptor (CSF-1R) kinase inhibitory activity. WO2006067446 relates to chemical compounds, or pharmaceutically acceptable salts thereof, which possess B-Raf inhibitory activity. US2008269215 relates to organic compounds useful for therapy and/or prophylaxis in a mammal, in particular to compounds that inhibit the hedgehog signaling pathway. Blood, 2013, 122(7), 1222-1232 shows a mutation in MYD88 (L265P) supports survival of lymphoplasmacytic cells by activation of Bruton tyrosine kinase in Waldenström macroglobulinemia. WO2008039218 relates to compounds that form covalent bonds with Bruton's tyrosine kinase (BtK). US20130040949A1 relates to novel compounds which are able to modulate b-raf kinases, and the use of such compounds in the treatment of various diseases, disorders or conditions.

### SUMMARY OF THE INVENTION

The present invention is set out in the appended claims.

The present invention is based on the discovery of compounds of the Formula (**I- a1**), (**I-a2**) or **(I-a3):** 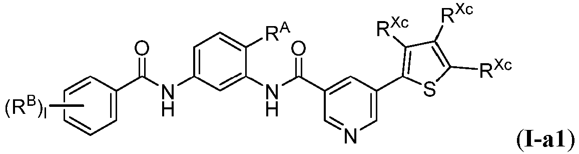 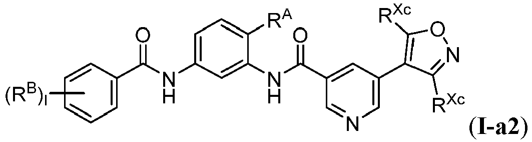 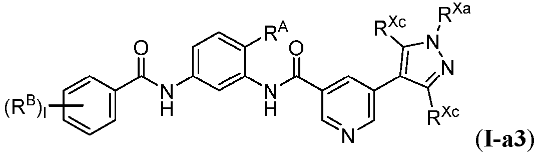 or a pharmaceutically acceptable salt, solvate, hydrate, polymorph, co-crystal, tautomer, or stereoisomer thereof, wherein R^{A}, R^{B}, R^{Xa}, R^{Xc} and 1 are defined herein, for the treatment of Waldenström's macroglobulinemia. The activity of these compounds was established by *in vitro* screening against several kinases *(e.g.,* BTK, HCK, TAK1, HPK1).

In certain embodiments, compounds of Formula (I-a) are of the formula: 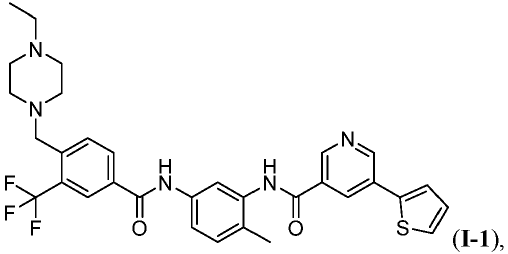 and pharmaceutically acceptable salts thereof.

The present invention is also based, at least in part, on the discovery that Waldenström's macroglobulinemia may be treated by administration of a compound of the invention to a subject in need thereof. The activity of these compounds was established by *in vitro* screening against several kinases *(e.g.,* BTK, HCK, TAK1, HPK1) that are involved in the regulation of aberrant cell growth, as well as cell-based screening against several cell lines (*e.g.*, BCWM.1, MWCL-1) that are disease state models of Waldenström's macroglobulinemia (Ditzel et al. Exp Hematol. 2007 Sep;35(9):1366-75; Hodge et al. Blood. 2011 May 12;117(19)).

A compound of the invention for use in treating B cell neoplasms of the group consisting of Hodgkin's lymphomas and most non-Hodgkin's lymphomas, such as diffuse large B cell lymphoma, Follicular lymphoma, mucosa-associated lymphatic tissue lymphoma (MALT), small cell lymphocytic lymphoma (overlaps with chronic lymphocytic leukemia), mantle cell lymphoma (MCL), Burkitt lymphoma, mediastinal large B cell lymphoma, nodal marginal zone B cell lymphoma (NMZL), splenic marginal zone lymphoma (SMZL), Intravascular large B-cell lymphoma, Primary effusion lymphoma, and Lymphomatoid granulomatosis.

The present invention is also based, at least in part, on pharmaceutical compositions comprising a compound of the invention and a pharmaceutically acceptable excipient. In certain embodiments, the pharmaceutical composition may be for use in treating a B cell neoplasm in a subject . In certain embodiments, the pharmaceutical composition may be for use in the treatment of Waldenström's macroglobulinemia in a subject in need thereof.

The present invention also provides kits comprising a container with a compound of the invention, or a pharmaceutical composition thereof. The kits may include a single dose or multiple doses of a compound described herein or a pharmaceutical composition thereof. The kits may be useful for modulating the activity of a kinase in a subject in need thereof. The kits may also be useful for treating and/or preventing in a subject in need thereof a condition associated with aberrant activity of a kinase. In certain embodiments, the kits further include instructions for using the kit *(e.g.,* for administering a compound described herein, or a pharmaceutical composition thereof).

The details of particular embodiments of the invention are set forth herein. Other features, objects, and advantages of the invention will be apparent from the Detailed Description, the Examples, and the Claims.

### DEFINITIONS

### Chemical Definitions

Definitions of specific functional groups and chemical terms are described in more detail below. The chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Organic Chemistry, Thomas Sorrell, University Science Books, Sausalito, 1999; Smith and March March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987.

Compounds described herein can comprise one or more asymmetric centers, and thus can exist in various stereoisomeric forms, *e.g.*, enantiomers and/or diastereomers. For example, the compounds described herein can be in the form of an individual enantiomer, diastereomer or geometric isomer, or can be in the form of a mixture of stereoisomers, including racemic mixtures and mixtures enriched in one or more stereoisomer. Isomers can be isolated from mixtures by methods known to those skilled in the art, including chiral high pressure liquid chromatography (HPLC) and the formation and crystallization of chiral salts; or preferred isomers can be prepared by asymmetric syntheses. See, for example, Jacques et al., Enantiomers, Racemates and Resolutions (Wiley Interscience, New York, 1981); Wilen et al., Tetrahedron 33:2725 (1977); Eliel, E.L. Stereochemistry of Carbon Compounds (McGraw-Hill, NY, 1962); and Wilen, S.H. Tables of Resolving Agents and Optical Resolutions p. 268 (E.L. Eliel, Ed., Univ. of Notre Dame Press, Notre Dame, IN 1972). The invention additionally encompasses compounds as individual isomers substantially free of other isomers, and alternatively, as mixtures of various isomers.

Unless otherwise stated, structures depicted herein are also meant to include compounds that differ only in the presence of one or more isotopically enriched atoms. For example, compounds having the present structures except for the replacement of hydrogen by deuterium or tritium, replacement of ¹⁹F with ¹⁸F, or the replacement of a carbon by a ¹³C- or ¹⁴C-enriched carbon are within the scope of the disclosure. Such compounds are useful, for example, as analytical tools or probes in biological assays.

When a range of values is listed, it is intended to encompass each value and sub-range within the range. For example "C₁₋₆ alkyl" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

The term "aliphatic," as used herein, refers to alkyl, alkenyl, alkynyl, and carbocyclic groups. Likewise, the term "heteroaliphatic" as used herein, refers to heteroalkyl, heteroalkenyl, heteroalkynyl, and heterocyclic groups.

The term "alkyl" refers to a radical of a straight-chain or branched saturated hydrocarbon group having from 1 to 10 carbon atoms ("C₁₋₁₀ alkyl"). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). In some embodiments, an alkyl group has 2 to 6 carbon atoms ("C₂₋₆ alkyl"). Examples of C₁₋₆ alkyl groups include methyl (C₁), ethyl (C₂), propyl (C₃) *(e.g.,* n-propyl, isopropyl), butyl (C₄) *(e.g.,* n-butyl, tert-butyl, sec-butyl, iso-butyl), pentyl (C₅) *(e.g.,* n-pentyl, 3-pentanyl, amyl, neopentyl, 3-methyl-2-butanyl, tertiary amyl), and hexyl (C₆) *(e.g.,* n-hexyl). Additional examples of alkyl groups include n-heptyl (C₇), and n-octyl (C₈). Unless otherwise specified, each instance of an alkyl group is independently unsubstituted (an "unsubstituted alkyl") or substituted (a "substituted alkyl") with one or more substituents (e.g., halogen, such as F). In certain embodiments, the alkyl group is an unsubstituted C₁₋₁₀ alkyl (such as unsubstituted C₁₋₆ alkyl, *e.g.,* -CH₃ (Me), unsubstituted ethyl (Et), unsubstituted propyl (Pr, *e.g.,* unsubstituted n-propyl (n-Pr), unsubstituted isopropyl (i-Pr)), unsubstituted butyl (Bu, *e.g.,* unsubstituted n-butyl (n-Bu), unsubstituted *tert-*butyl *(tert-*Bu or t-Bu), unsubstituted sec-butyl *(sec-*Bu*),* unsubstituted isobutyl (i-Bu)). In certain embodiments, the alkyl group is a substituted C₁₋₁₀ alkyl (such as substituted C₁₋₆ alkyl, *e.g.,* -CF₃, Bn).

As used herein, "haloalkyl" is a substituted alkyl group as defined herein wherein one or more of the hydrogen atoms are independently replaced by a halogen, *e.g.,* fluoro, bromo, chloro, or iodo. "Perhaloalkyl" is a subset of haloalkyl, and refers to an alkyl group wherein all of the hydrogen atoms are independently replaced by a halogen, *e.g.,* fluoro, bromo, chloro, or iodo. In certain embodiments, the haloalkyl moiety has 1 to 8 carbon atoms ("C₁₋₈ haloalkyl"). In certain embodiments, the haloalkyl moiety has 1 to 6 carbon atoms ("C₁₋₆ haloalkyl"). In certain embodiments, the haloalkyl moiety has 1 to 4 carbon atoms ("C₁₋₄ haloalkyl"). In certain embodiments, the haloalkyl moiety has 1 to 3 carbon atoms ("C₁₋₃ haloalkyl"). In certain embodiments, the haloalkyl moiety has 1 to 2 carbon atoms ("C₁₋₂ haloalkyl"). In certain embodiments, all of the haloalkyl hydrogen atoms are replaced with fluoro to provide a perfluoroalkyl group. In certain embodiments, all of the haloalkyl hydrogen atoms are replaced with chloro to provide a "perchloroalkyl" group. Examples of haloalkyl groups include -CF₃, - CF₂CF₃, -CF₂CF₂CF₃, -CCl₃, -CFCl₂, and -CF₂Cl.

As used herein, "heteroalkyl" refers to an alkyl group as defined herein which further includes at least one heteroatom (e.g., 1, 2, 3, or 4 heteroatoms) selected from oxygen, nitrogen, or sulfur within *(i.e.,* inserted between adjacent carbon atoms of) and/or placed at one or more terminal position(s) of the parent chain. In certain embodiments, a heteroalkyl group refers to a saturated group having from 1 to 10 carbon atoms and 1 or more heteroatoms within the parent chain ("heteroC₁₋₁₀ alkyl"). In certain embodiments, a heteroalkyl group is a saturated group having 1 to 9 carbon atoms and 1 or more heteroatoms within the parent chain ("heteroC₁₋₉ alkyl"). In certain embodiments, a heteroalkyl group is a saturated group having 1 to 8 carbon atoms and 1 or more heteroatoms within the parent chain ("heteroC₁₋₈ alkyl"). In certain embodiments, a heteroalkyl group is a saturated group having 1 to 7 carbon atoms and 1 or more heteroatoms within the parent chain ("heteroC₁₋₇ alkyl"). In certain embodiments, a heteroalkyl group is a saturated group having 1 to 6 carbon atoms and 1 or more heteroatoms within the parent chain ("heteroC₁₋₆ alkyl"). In certain embodiments, a heteroalkyl group is a saturated group having 1 to 5 carbon atoms and 1 or 2 heteroatoms within the parent chain ("heteroC₁₋₅ alkyl"). In certain embodiments, a heteroalkyl group is a saturated group having 1 to 4 carbon atoms and 1or 2 heteroatoms within the parent chain ("heteroC₁₋₄ alkyl"). In certain embodiments, a heteroalkyl group is a saturated group having 1 to 3 carbon atoms and 1 heteroatom within the parent chain ("heteroC₁₋₃ alkyl"). In certain embodiments, a heteroalkyl group is a saturated group having 1 to 2 carbon atoms and 1 heteroatom within the parent chain ("heteroC₁₋₂ alkyl"). In certain embodiments, a heteroalkyl group is a saturated group having 1 carbon atom and 1 heteroatom ("heteroC₁ alkyl"). In certain embodiments, a heteroalkyl group is a saturated group having 2 to 6 carbon atoms and 1 or 2 heteroatoms within the parent chain ("heteroC₂₋₆ alkyl"). Unless otherwise specified, each instance of a heteroalkyl group is independently unsubstituted (an "unsubstituted heteroalkyl") or substituted (a "substituted heteroalkyl") with one or more substituents. In certain embodiments, the heteroalkyl group is an unsubstituted heteroC₁₋₁₀ alkyl. In certain embodiments, the heteroalkyl group is a substituted heteroC₁₋₁₀ alkyl.

As used herein, "alkenyl" refers to a radical of a straight-chain or branched hydrocarbon group having from 2 to 10 carbon atoms and one or more carbon-carbon double bonds *(e.g.,* 1, 2, 3, or 4 double bonds). In certain embodiments, an alkenyl group has 2 to 9 carbon atoms ("C₂₋₉ alkenyl"). In certain embodiments, an alkenyl group has 2 to 8 carbon atoms ("C₂₋₈ alkenyl"). In certain embodiments, an alkenyl group has 2 to 7 carbon atoms ("C₂₋₇ alkenyl"). In certain embodiments, an alkenyl group has 2 to 6 carbon atoms ("C₂₋₆ alkenyl"). In certain embodiments, an alkenyl group has 2 to 5 carbon atoms ("C₂₋₅ alkenyl"). In certain embodiments, an alkenyl group has 2 to 4 carbon atoms ("C₂₋₄ alkenyl"). In certain embodiments, an alkenyl group has 2 to 3 carbon atoms ("C₂₋₃ alkenyl"). In certain embodiments, an alkenyl group has 2 carbon atoms ("C₂ alkenyl"). The one or more carbon-carbon double bonds can be internal (such as in 2-butenyl) or terminal (such as in 1-butenyl). Examples of C₂₋₄ alkenyl groups include ethenyl (C₂), 1-propenyl (C₃), 2-propenyl (C₃), 1-butenyl (C₄), 2-butenyl (C₄), and butadienyl (C₄). Examples of C₂₋₆ alkenyl groups include the aforementioned C₂₋₄ alkenyl groups as well as pentenyl (C₅), pentadienyl (C₅), and hexenyl (C₆). Additional examples of alkenyl include heptenyl (C₇), octenyl (C₈), and octatrienyl (C₈). Unless otherwise specified, each instance of an alkenyl group is independently unsubstituted (an "unsubstituted alkenyl") or substituted (a "substituted alkenyl") with one or more substituents. In certain embodiments, the alkenyl group is an unsubstituted C₂₋₁₀ alkenyl. In certain embodiments, the alkenyl group is a substituted C₂₋₁₀ alkenyl.

As used herein, "heteroalkenyl" refers to an alkenyl group as defined herein which further includes at least one heteroatom (*e.g*., 1, 2, 3, or 4 heteroatoms) selected from oxygen, nitrogen, or sulfur within *(i.e.,* inserted between adjacent carbon atoms of) and/or placed at one or more terminal position(s) of the parent chain. In certain embodiments, a heteroalkenyl group refers to a group having from 2 to 10 carbon atoms, at least one double bond, and 1 or more heteroatoms within the parent chain ("heteroC₂₋₁₀ alkenyl"). In certain embodiments, a heteroalkenyl group has 2 to 9 carbon atoms at least one double bond, and 1 or more heteroatoms within the parent chain ("heteroC₂₋₉ alkenyl"). In certain embodiments, a heteroalkenyl group has 2 to 8 carbon atoms, at least one double bond, and 1 or more heteroatoms within the parent chain ("heteroC₂₋₈ alkenyl"). In certain embodiments, a heteroalkenyl group has 2 to 7 carbon atoms, at least one double bond, and 1 or more heteroatoms within the parent chain ("heteroC₂₋₇ alkenyl"). In certain embodiments, a heteroalkenyl group has 2 to 6 carbon atoms, at least one double bond, and 1 or more heteroatoms within the parent chain ("heteroC₂₋₆ alkenyl"). In certain embodiments, a heteroalkenyl group has 2 to 5 carbon atoms, at least one double bond, and 1 or 2 heteroatoms within the parent chain ("heteroC₂₋₅ alkenyl"). In certain embodiments, a heteroalkenyl group has 2 to 4 carbon atoms, at least one double bond, and 1or 2 heteroatoms within the parent chain ("heteroC₂₋₄ alkenyl"). In certain embodiments, a heteroalkenyl group has 2 to 3 carbon atoms, at least one double bond, and 1 heteroatom within the parent chain ("heteroC₂₋₃ alkenyl"). In certain embodiments, a heteroalkenyl group has 2 to 6 carbon atoms, at least one double bond, and 1 or 2 heteroatoms within the parent chain ("heteroC₂₋₆ alkenyl"). Unless otherwise specified, each instance of a heteroalkenyl group is independently unsubstituted (an "unsubstituted heteroalkenyl") or substituted (a "substituted heteroalkenyl") with one or more substituents. In certain embodiments, the heteroalkenyl group is an unsubstituted heteroC₂₋₁₀ alkenyl. In certain embodiments, the heteroalkenyl group is a substituted heteroC₂₋₁₀ alkenyl. In an alkenyl group, a C=C double bond for which the stereochemistry is not specified (*e.g., -* CH=CHCH₃ or 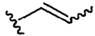 ) may be an (E)- or (Z)-double bond.

As used herein, "alkynyl" refers to a radical of a straight-chain or branched hydrocarbon group having from 2 to 10 carbon atoms and one or more carbon-carbon triple bonds *(e.g.,* 1, 2, 3, or 4 triple bonds) ("C₂₋₁₀ alkynyl"). In certain embodiments, an alkynyl group has 2 to 9 carbon atoms ("C₂₋₉ alkynyl"). In certain embodiments, an alkynyl group has 2 to 8 carbon atoms ("C₂₋₈ alkynyl"). In certain embodiments, an alkynyl group has 2 to 7 carbon atoms ("C₂₋₇ alkynyl"). In certain embodiments, an alkynyl group has 2 to 6 carbon atoms ("C₂₋₆ alkynyl"). In certain embodiments, an alkynyl group has 2 to 5 carbon atoms ("C₂₋₅ alkynyl"). In certain embodiments, an alkynyl group has 2 to 4 carbon atoms ("C₂₋₄ alkynyl"). In certain embodiments, an alkynyl group has 2 to 3 carbon atoms ("C₂₋₃ alkynyl"). In certain embodiments, an alkynyl group has 2 carbon atoms ("C₂ alkynyl"). The one or more carbon-carbon triple bonds can be internal (such as in 2-butynyl) or terminal (such as in 1-butynyl). Examples of C₂₋₄ alkynyl groups include, without limitation, ethynyl (C₂), 1-propynyl (C₃), 2-propynyl (C₃), 1-butynyl (C₄), and 2-butynyl (C₄). Examples of C₂₋₆ alkenyl groups include the aforementioned C₂₋₄ alkynyl groups as well as pentynyl (C₅), and hexynyl (C₆). Additional examples of alkynyl include heptynyl (C₇), and octynyl (C₈). Unless otherwise specified, each instance of an alkynyl group is independently unsubstituted (an "unsubstituted alkynyl") or substituted (a "substituted alkynyl") with one or more substituents. In certain embodiments, the alkynyl group is an unsubstituted C₂₋₁₀ alkynyl. In certain embodiments, the alkynyl group is a substituted C₂₋₁₀ alkynyl.

As used herein, "heteroalkynyl" refers to an alkynyl group as defined herein which further includes at least one heteroatom (*e.g.*, 1, 2, 3, or 4 heteroatoms) selected from oxygen, nitrogen, or sulfur within *(i.e.,* inserted between adjacent carbon atoms of) and/or placed at one or more terminal position(s) of the parent chain. In certain embodiments, a heteroalkynyl group refers to a group having from 2 to 10 carbon atoms, at least one triple bond, and 1 or more heteroatoms within the parent chain ("heteroC₂₋₁₀ alkynyl"). In certain embodiments, a heteroalkynyl group has 2 to 9 carbon atoms, at least one triple bond, and 1 or more heteroatoms within the parent chain ("heteroC₂₋₉ alkynyl"). In certain embodiments, a heteroalkynyl group has 2 to 8 carbon atoms, at least one triple bond, and 1 or more heteroatoms within the parent chain ("heteroC₂₋₈ alkynyl"). In certain embodiments, a heteroalkynyl group has 2 to 7 carbon atoms, at least one triple bond, and 1 or more heteroatoms within the parent chain ("heteroC₂₋₇ alkynyl"). In certain embodiments, a heteroalkynyl group has 2 to 6 carbon atoms, at least one triple bond, and 1 or more heteroatoms within the parent chain ("heteroC₂₋₆ alkynyl"). In certain embodiments, a heteroalkynyl group has 2 to 5 carbon atoms, at least one triple bond, and 1 or 2 heteroatoms within the parent chain ("heteroC₂₋₅ alkynyl"). In certain embodiments, a heteroalkynyl group has 2 to 4 carbon atoms, at least one triple bond, and 1or 2 heteroatoms within the parent chain ("heteroC₂₋₄ alkynyl"). In certain embodiments, a heteroalkynyl group has 2 to 3 carbon atoms, at least one triple bond, and 1 heteroatom within the parent chain ("heteroC₂₋₃ alkynyl"). In certain embodiments, a heteroalkynyl group has 2 to 6 carbon atoms, at least one triple bond, and 1 or 2 heteroatoms within the parent chain ("heteroC₂₋₆ alkynyl"). Unless otherwise specified, each instance of a heteroalkynyl group is independently unsubstituted (an "unsubstituted heteroalkynyl") or substituted (a "substituted heteroalkynyl") with one or more substituents. In certain embodiments, the heteroalkynyl group is an unsubstituted heteroC₂₋₁₀ alkynyl. In certain embodiments, the heteroalkynyl group is a substituted heteroC₂₋₁₀ alkynyl.

As used herein, "carbocyclyl" or "carbocyclic" refers to a radical of a non-aromatic cyclic hydrocarbon group having from 3 to 14 ring carbon atoms ("C₃₋₁₄ carbocyclyl") and zero heteroatoms in the non-aromatic ring system. In certain embodiments, a carbocyclyl group has 3 to 10 ring carbon atoms ("C₃₋₁₀ carbocyclyl"). In certain embodiments, a carbocyclyl group has 3 to 8 ring carbon atoms ("C₃₋₈ carbocyclyl"). In certain embodiments, a carbocyclyl group has 3 to 7 ring carbon atoms ("C₃₋₇ carbocyclyl"). In certain embodiments, a carbocyclyl group has 3 to 6 ring carbon atoms ("C₃₋₆ carbocyclyl"). In certain embodiments, a carbocyclyl group has 4 to 6 ring carbon atoms ("C₄₋₆ carbocyclyl"). In certain embodiments, a carbocyclyl group has 5 to 6 ring carbon atoms ("C₅₋₆ carbocyclyl"). In certain embodiments, a carbocyclyl group has 5 to 10 ring carbon atoms ("C₅₋₁₀ carbocyclyl"). Exemplary C₃₋₆ carbocyclyl groups include, without limitation, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), and cyclohexadienyl (C₆). Exemplary C₃₋₈ carbocyclyl groups include, without limitation, the aforementioned C₃₋₆ carbocyclyl groups as well as cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptanyl (C₇), and bicyclo[2.2.2]octanyl (C₈). Exemplary C₃₋₁₀ carbocyclyl groups include, without limitation, the aforementioned C₃₋₈ carbocyclyl groups as well as cyclononyl (C₉), cyclononenyl (C₉), cyclodecyl (C₁₀), cyclodecenyl (C₁₀), octahydro-1H-indenyl (C₉), decahydronaphthalenyl (C₁₀), and spiro[4.5]decanyl (C₁₀). As the foregoing examples illustrate, in certain embodiments, the carbocyclyl group is either monocyclic ("monocyclic carbocyclyl") or polycyclic (*e.g.*, containing a fused, bridged or spiro ring system such as a bicyclic system ("bicyclic carbocyclyl") or tricyclic system ("tricyclic carbocyclyl")) and can be saturated or can contain one or more carbon-carbon double or triple bonds. "Carbocyclyl" also includes ring systems wherein the carbocyclyl ring, as defined above, is fused with one or more aryl or heteroaryl groups wherein the point of attachment is on the carbocyclyl ring, and in such instances, the number of carbons continue to designate the number of carbons in the carbocyclic ring system. Unless otherwise specified, each instance of a carbocyclyl group is independently unsubstituted (an "unsubstituted carbocyclyl") or substituted (a "substituted carbocyclyl") with one or more substituents. In certain embodiments, the carbocyclyl group is an unsubstituted C₃₋₁₄ carbocyclyl. In certain embodiments, the carbocyclyl group is a substituted C₃₋₁₄ carbocyclyl.
In certain embodiments, "carbocyclyl" is a monocyclic, saturated carbocyclyl group having from 3 to 14 ring carbon atoms ("C₃₋₁₄ cycloalkyl"). In certain embodiments, a cycloalkyl group has 3 to 10 ring carbon atoms ("C₃₋₁₀ cycloalkyl"). In certain embodiments, a cycloalkyl group has 3 to 8 ring carbon atoms ("C₃₋₈ cycloalkyl"). In certain embodiments, a cycloalkyl group has 3 to 6 ring carbon atoms ("C₃₋₆ cycloalkyl"). In certain embodiments, a cycloalkyl group has 4 to 6 ring carbon atoms ("C₄₋₆ cycloalkyl"). In certain embodiments, a cycloalkyl group has 5 to 6 ring carbon atoms ("C₅₋₆ cycloalkyl"). In certain embodiments, a cycloalkyl group has 5 to 10 ring carbon atoms ("C₅₋₁₀ cycloalkyl"). Examples of C₅₋₆ cycloalkyl groups include cyclopentyl (C₅) and cyclohexyl (C₅). Examples of C₃₋₆ cycloalkyl groups include the aforementioned C₅₋₆ cycloalkyl groups as well as cyclopropyl (C₃) and cyclobutyl (C₄). Examples of C₃₋₈ cycloalkyl groups include the aforementioned C₃₋₆ cycloalkyl groups as well as cycloheptyl (C₇) and cyclooctyl (C₈). Unless otherwise specified, each instance of a cycloalkyl group is independently unsubstituted (an "unsubstituted cycloalkyl") or substituted (a "substituted cycloalkyl") with one or more substituents. In certain embodiments, the cycloalkyl group is an unsubstituted C₃₋₁₄ cycloalkyl. In certain embodiments, the cycloalkyl group is a substituted C₃₋₁₄ cycloalkyl.

As used herein, "heterocyclyl" or "heterocyclic" refers to a radical of a 3- to 14-membered non-aromatic ring system having ring carbon atoms and 1 to 4 ring heteroatoms, wherein each heteroatom is independently selected from nitrogen, oxygen, and sulfur ("3-14 membered heterocyclyl"). In heterocyclyl groups that contain one or more nitrogen atoms, the point of attachment can be a carbon or nitrogen atom, as valency permits. A heterocyclyl group can either be monocyclic ("monocyclic heterocyclyl") or polycyclic *(e.g.,* a fused, bridged or spiro ring system such as a bicyclic system ("bicyclic heterocyclyl") or tricyclic system ("tricyclic heterocyclyl")), and can be saturated or can contain one or more carbon-carbon double or triple bonds. Heterocyclyl polycyclic ring systems can include one or more heteroatoms in one or both rings. "Heterocyclyl" also includes ring systems wherein the heterocyclyl ring, as defined above, is fused with one or more carbocyclyl groups wherein the point of attachment is either on the carbocyclyl or heterocyclyl ring, or ring systems wherein the heterocyclyl ring, as defined above, is fused with one or more aryl or heteroaryl groups, wherein the point of attachment is on the heterocyclyl ring, and in such instances, the number of ring members continue to designate the number of ring members in the heterocyclyl ring system. Unless otherwise specified, each instance of heterocyclyl is independently unsubstituted (an "unsubstituted heterocyclyl") or substituted (a "substituted heterocyclyl") with one or more substituents. In certain embodiments, the heterocyclyl group is an unsubstituted 3-14 membered heterocyclyl. In certain embodiments, the heterocyclyl group is a substituted 3-14 membered heterocyclyl.

In certain embodiments, a heterocyclyl group is a 5-10 membered non-aromatic ring system having ring carbon atoms and 1-4 ring heteroatoms, wherein each heteroatom is independently selected from nitrogen, oxygen, and sulfur ("5-10 membered heterocyclyl"). In certain embodiments, a heterocyclyl group is a 5-8 membered non-aromatic ring system having ring carbon atoms and 1-4 ring heteroatoms, wherein each heteroatom is independently selected from nitrogen, oxygen, and sulfur ("5-8 membered heterocyclyl"). In certain embodiments, a heterocyclyl group is a 5-6 membered non-aromatic ring system having ring carbon atoms and 1-4 ring heteroatoms, wherein each heteroatom is independently selected from nitrogen, oxygen, and sulfur ("5-6 membered heterocyclyl"). In certain embodiments, the 5-6 membered heterocyclyl has 1-3 ring heteroatoms selected from nitrogen, oxygen, and sulfur. In certain embodiments, the 5-6 membered heterocyclyl has 1-2 ring heteroatoms selected from nitrogen, oxygen, and sulfur. In certain embodiments, the 5-6 membered heterocyclyl has 1 ring heteroatom selected from nitrogen, oxygen, and sulfur.

Exemplary 3-membered heterocyclyl groups containing 1 heteroatom include, without limitation, azirdinyl, oxiranyl, and thiiranyl. Exemplary 4-membered heterocyclyl groups containing 1 heteroatom include, without limitation, azetidinyl, oxetanyl, and thietanyl. Exemplary 5-membered heterocyclyl groups containing 1 heteroatom include, without limitation, tetrahydrofuranyl, dihydrofuranyl, tetrahydrothiophenyl, dihydrothiophenyl, pyrrolidinyl, dihydropyrrolyl, and pyrrolyl-2,5-dione. Exemplary 5-membered heterocyclyl groups containing 2 heteroatoms include, without limitation, dioxolanyl, oxathiolanyl and dithiolanyl. Exemplary 5-membered heterocyclyl groups containing 3 heteroatoms include, without limitation, triazolinyl, oxadiazolinyl, and thiadiazolinyl. Exemplary 6-membered heterocyclyl groups containing 1 heteroatom include, without limitation, piperidinyl, tetrahydropyranyl, dihydropyridinyl, and thianyl. Exemplary 6-membered heterocyclyl groups containing 2 heteroatoms include, without limitation, piperazinyl, morpholinyl, dithianyl, and dioxanyl. Exemplary 6-membered heterocyclyl groups containing 2 heteroatoms include, without limitation, triazinanyl. Exemplary 7-membered heterocyclyl groups containing 1 heteroatom include, without limitation, azepanyl, oxepanyl and thiepanyl. Exemplary 8-membered heterocyclyl groups containing 1 heteroatom include, without limitation, azocanyl, oxecanyl and thiocanyl. Exemplary bicyclic heterocyclyl groups include, without limitation, indolinyl, isoindolinyl, dihydrobenzofuranyl, dihydrobenzothienyl, tetrahydrobenzothienyl, tetrahydrobenzofuranyl, tetrahydroindolyl, tetrahydroquinolinyl, tetrahydroisoquinolinyl, decahydroquinolinyl, decahydroisoquinolinyl, octahydrochromenyl, octahydroisochromenyl, decahydronaphthyridinyl, decahydro-1,8-naphthyridinyl, octahydropyrrolo[3,2-b]pyrrole, indolinyl, phthalimidyl, naphthalimidyl, chromanyl, chromenyl, 1H-benzo[e][1,4]diazepinyl, 1,4,5,7-tetrahydropyrano[3,4-b]pyrrolyl, 5,6-dihydro-4H-furo[3,2-b]pyrrolyl, 6,7-dihydro-5H-furo[3,2-b]pyranyl, 5,7-dihydro-4H-thieno[2,3-c]pyranyl, 2,3-dihydro-1H-pyrrolo[2,3-b]pyridinyl, 2,3-dihydrofuro[2,3-b]pyridinyl, 4,5,6,7-tetrahydro-1H-pyrrolo[2,3-b]pyridinyl, 4,5,6,7-tetrahydrofuro[3,2-c]pyridinyl, 4,5,6,7-tetrahydrothieno[3,2-b]pyridinyl, and 1,2,3,4-tetrahydro-1,6-naphthyridinyl.

As used herein, "aryl" refers to a radical of a monocyclic or polycyclic (e.g., bicyclic or tricyclic) 4n+2 aromatic ring system (e.g., having 6, 10, or 14 π electrons shared in a cyclic array) having 6-14 ring carbon atoms and zero heteroatoms provided in the aromatic ring system ("C₆₋₁₄ aryl"). In certain embodiments, an aryl group has 6 ring carbon atoms ("C₆ aryl"; *e.g.,* phenyl). In certain embodiments, an aryl group has 10 ring carbon atoms ("C₁₀ aryl"; *e.g.,* naphthyl such as 1-naphthyl and 2-naphthyl). In certain embodiments, an aryl group has 14 ring carbon atoms ("C₁₄ aryl"; e.g., anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Unless otherwise specified, each instance of an aryl group is independently unsubstituted (an "unsubstituted aryl") or substituted (a "substituted aryl") with one or more substituents. In certain embodiments, the aryl group is an unsubstituted C₆₋₁₄ aryl. In certain embodiments, the aryl group is a substituted C₆₋₁₄ aryl.

"Aralkyl" is a subset of "alkyl" and refers to an alkyl group, as defined herein, substituted by an aryl group, as defined herein, wherein the point of attachment is on the alkyl moiety.

As used herein, "heteroaryl" refers to a radical of a 5-14 membered monocyclic or polycyclic *(e.g.,* bicyclic, tricyclic) 4n+2 aromatic ring system *(e.g.,* having 6, 10, or 14 π electrons shared in a cyclic array) having ring carbon atoms and 1-4 ring heteroatoms provided in the aromatic ring system, wherein each heteroatom is independently selected from nitrogen, oxygen, and sulfur ("5-14 membered heteroaryl"). In heteroaryl groups that contain one or more nitrogen atoms, the point of attachment can be a carbon or nitrogen atom, as valency permits. Heteroaryl polycyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused polycyclic (aryl/heteroaryl) ring system. Polycyclic heteroaryl groups wherein one ring does not contain a heteroatom (e.g., indolyl, quinolinyl, and carbazolyl) the point of attachment can be on either ring, *i.e.,* either the ring bearing a heteroatom *(e.g.,* 2-indolyl) or the ring that does not contain a heteroatom *(e.g.,* 5-indolyl).

In certain embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). In certain embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In certain embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In certain embodiments, the 5-6 membered heteroaryl has 1-3 ring heteroatoms selected from nitrogen, oxygen, and sulfur. In certain embodiments, the 5-6 membered heteroaryl has 1-2 ring heteroatoms selected from nitrogen, oxygen, and sulfur. In certain embodiments, the 5-6 membered heteroaryl has 1 ring heteroatom selected from nitrogen, oxygen, and sulfur. Unless otherwise specified, each instance of a heteroaryl group is independently unsubstituted (an "unsubstituted heteroaryl") or substituted (a "substituted heteroaryl") with one or more substituents. In certain embodiments, the heteroaryl group is an unsubstituted 5-14 membered heteroaryl. In certain embodiments, the heteroaryl group is a substituted 5-14 membered heteroaryl.

Exemplary 5-membered heteroaryl groups containing 1 heteroatom include, without limitation, pyrrolyl, furanyl, and thiophenyl. Exemplary 5-membered heteroaryl groups containing 2 heteroatoms include, without limitation, imidazolyl, pyrazolyl, oxazolyl, isoxazolyl, thiazolyl, and isothiazolyl. Exemplary 5-membered heteroaryl groups containing 3 heteroatoms include, without limitation, triazolyl, oxadiazolyl, and thiadiazolyl. Exemplary 5-membered heteroaryl groups containing 4 heteroatoms include, without limitation, tetrazolyl. Exemplary 6-membered heteroaryl groups containing 1 heteroatom include, without limitation, pyridinyl. Exemplary 6-membered heteroaryl groups containing 2 heteroatoms include, without limitation, pyridazinyl, pyrimidinyl, and pyrazinyl. Exemplary 6-membered heteroaryl groups containing 3 or 4 heteroatoms include, without limitation, triazinyl and tetrazinyl, respectively. Exemplary 7-membered heteroaryl groups containing 1 heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl. Exemplary tricyclic heteroaryl groups include, without limitation, phenanthridinyl, dibenzofuranyl, carbazolyl, acridinyl, phenothiazinyl, phenoxazinyl and phenazinyl.

"Heteroaralkyl" is a subset of "alkyl" and refers to an alkyl group, as defined herein, substituted by a heteroaryl group, as defined herein, wherein the point of attachment is on the alkyl moiety.

As used herein, the term "partially unsaturated" refers to a ring moiety that includes at least one double or triple bond. The term "partially unsaturated" is intended to encompass rings having multiple sites of unsaturation, but is not intended to include aromatic groups *(e.g.,* aryl or heteroaryl moieties) as herein defined.

As used herein, the term "saturated" refers to a ring moiety that does not contain a double or triple bond, *i.e.,* the ring contains all single bonds.

Affixing the suffix "-ene" to a group indicates the group is a divalent moiety, e.g., alkylene is the divalent moiety of alkyl, alkenylene is the divalent moiety of alkenyl, alkynylene is the divalent moiety of alkynyl, heteroalkylene is the divalent moiety of heteroalkyl, heteroalkenylene is the divalent moiety of heteroalkenyl, heteroalkynylene is the divalent moiety of heteroalkynyl, carbocyclylene is the divalent moiety of carbocyclyl, heterocyclylene is the divalent moiety of heterocyclyl, arylene is the divalent moiety of aryl, and heteroarylene is the divalent moiety of heteroaryl.

As understood from the above, alkyl, alkenyl, alkynyl, heteroalkyl, heteroalkenyl, heteroalkynyl, carbocyclyl, heterocyclyl, aryl, and heteroaryl groups, as defined herein, are, in certain embodiments, optionally substituted. Optionally substituted refers to a group which may be substituted or unsubstituted (e.g., "substituted" or "unsubstituted" alkyl, "substituted" or "unsubstituted" alkenyl, "substituted" or "unsubstituted" alkynyl, "substituted" or "unsubstituted" heteroalkyl, "substituted" or "unsubstituted" heteroalkenyl, "substituted" or "unsubstituted" heteroalkynyl, "substituted" or "unsubstituted" carbocyclyl, "substituted" or "unsubstituted" heterocyclyl, "substituted" or "unsubstituted" aryl or "substituted" or "unsubstituted" heteroaryl group). In general, the term "substituted" means that at least one hydrogen present on a group is replaced with a permissible substituent, e.g., a substituent which upon substitution results in a stable compound, *e.g.,* a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. The term "substituted" is contemplated to include substitution with all permissible substituents of organic compounds, and includes any of the substituents described herein that results in the formation of a stable compound. The present invention contemplates any and all such combinations in order to arrive at a stable compound. For purposes of this invention, heteroatoms such as nitrogen may have hydrogen substituents and/or any suitable substituent as described herein which satisfy the valencies of the heteroatoms and results in the formation of a stable moiety.

Exemplary carbon atom substituents include, but are not limited to, halogen, -CN, - NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, -C(=O)R^{aa}, -CO₂H, -CHO, -C(OR^{cc})₂, -CO₂R^{aa}, -OC(=O)R^{aa}, -OCO₂R^{aa}, - C(=O)N(R^{bb})₂, -OC(=O)N(R^{bb})₂, -NR^{bb}C(=O)R^{aa}, -NR^{bb}CO₂R^{aa}, -NR^{bb}C(=O)N(R^{bb})₂, - C(=NR^{bb})R^{aa}, -C(=NR^{bb})OR^{aa}, -OC(=NR^{bb})R^{aa}, -OC(=NR^{bb})OR^{aa}, -C(=NR^{bb})N(R^{bb})₂, - OC(=NR^{bb})N(R^{bb})₂, -NR^{bb}C(=NR^{bb})N(R^{bb})₂, -C(=O)NR^{bb}SO₂R^{a}ₐ, -NR^{bb}SO₂R^{aa}, -SO₂N(R^{bb})₂, -SO₂R^{aa}, -SO₂OR^{aa}, -OSO₂R^{aa}, -S(=O)R^{aa}, -OS(=O)R^{aa}, -Si(R^{aa})₃, -OSi(R^{aa})₃ -C(=S)N(R^{bb})₂, -C(=O)SR^{aa}, -C(=S)SR^{aa}, -SC(=S)SR^{aa}, -SC(=O)SR^{aa}, -OC(=O)SR^{aa}, -SC(=O)OR^{aa}, - SC(=O)R^{aa}, -P(=O)₂R^{aa}, -OP(=O)₂R^{aa}, -P(=O)(R^{aa})₂, -OP(=O)(R^{aa})₂, -OP(=O)(OR^{cc})₂, - P(=O)₂N(R^{bb})₂, -OP(=O)₂N(R^{bb})₂, -P(=O)(NR^{bb})₂, -OP(=O)(NR^{bb})₂, -NR^{bb}P(=O)(OR^{cc})₂, - NR^{bb}P(=O)(NR^{bb})₂, -P(R^{cc})₂, -P(R^{cc})₃, -OP(R^{cc})₂, -OP(R^{cc})₃, -B(R^{aa})₂, -B(OR^{cc})₂, - BR^{aa}(OR^{cc}), C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups;
or two geminal hydrogens on a carbon atom are replaced with the group =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb}, or =NOR^{cc};
each instance of R^{aa} is, independently, selected from C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀alkenyl, heteroC₂₋₁₀alkynyl, C₃₋₁₀ carbocyclyl, 3-14 membered heterocyclyl, C₆₋₁₄ aryl, and 5-14 membered heteroaryl, or two R^{aa} groups are joined to form a 3-14 membered heterocyclyl or 5-14 membered heteroaryl ring, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups;
each instance of R^{bb} is, independently, selected from hydrogen, -OH, -OR^{aa}, -N(R^{cc})₂, - CN, -C(=O)R^{aa}, -C(=O)N(R^{cc})₂, -CO₂R^{aa}, -SO₂R^{aa}, -C(=NR^{cc})OR^{aa}, -C(=NR^{cc})N(R^{cc})₂, - SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, - P(=O)₂R^{aa}, -P(=O)(R^{aa})₂, -P(=O)₂N(R^{cc})₂, -P(=O)(NR^{cc})₂, C₁₋₁₀ alkyl, C₁₋₁₀ perhaloalkyl, C₂₋₁₀ alkenyl, C₂₋₁₀ alkynyl, heteroC₁₋₁₀alkyl, heteroC₂₋₁₀alkenyl, heteroC₂₋₁₀alkynyl, C₃₋₁₀ carbocyclyl, 3-14 membered heterocyclyl, C₆₋₁₄ aryl, and 5-14 membered heteroaryl, or two R^{bb} groups are joined to form a 3-14 membered heterocyclyl or 5-14 membered heteroaryl ring, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups;
each instance of R^{cc} is, independently, selected from hydrogen, C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl, or two R^{cc} groups are joined to form a 3-14 membered heterocyclyl or 5-14 membered heteroaryl ring, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups;
each instance of R^{dd} is, independently, selected from halogen, -CN, -NO₂, -N₃, -SO₂H, -SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, -SSR^{ee}, - C(=O)R^{ee}, -CO₂H, -CO₂R^{ee}, -OC(=O)R^{ee}, -OCO₂R^{ee}, -C(=O)N(R)₂, -OC(=O)N(R^{ff})₂, - NR^{ff}C(=O)R^{ee}, -NR^{ff}CO₂R^{ee}, -NR^{ff}C(=O)N(R^{ff})₂, -C(=NR^{ff})OR^{ee}, -OC(=NR^{ff})R^{ee}, - OC(=NR^{ff})OR^{ee}, -C(=NR^{ff})N(R^{ff})₂, -OC(=NR^{ff})N(R^{ff})₂, -NR^{ff}C(=NR^{ff})N(R^{ff})₂,-NR^{ff}SO₂R^{ee}, - SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, -C(=S)N(R^{ff})₂, - C(=O)SR^{ee}, -C(=S)SR^{ee}, -SC(=S)SR^{ee}, -P(=O)₂R^{ee}, -P(=O)(R^{ee})₂, -OP(=O)(R^{ee})₂, - OP(=O)(OR^{ee})₂, C₁₋₆ alkyl, C₁₋₆ perhaloalkyl, C₂₋₆ alkenyl, C₂₋₆ alkynyl, heteroC₁₋₆alkyl, heteroC₂₋₆alkenyl, heteroC₂₋₆alkynyl, C₃₋₁₀ carbocyclyl, 3-10 membered heterocyclyl, C₆₋₁₀ aryl, 5-10 membered heteroaryl, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups, or two geminal R^{dd} substituents can be joined to form =O or =S;
each instance of R^{ee} is, independently, selected from C₁₋₆ alkyl, C₁₋₆ perhaloalkyl, C₂₋₆ alkenyl, C₂₋₆ alkynyl, heteroC₁₋₆ alkyl, heteroC₂₋₆alkenyl, heteroC₂₋₆ alkynyl, C₃₋₁₀ carbocyclyl, C₆₋₁₀ aryl, 3-10 membered heterocyclyl, and 3-10 membered heteroaryl, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups;
each instance of R^{ff} is, independently, selected from hydrogen, C₁₋₆ alkyl, C₁₋₆ perhaloalkyl, C₂₋₆ alkenyl, C₂₋₆ alkynyl, heteroC₁₋₆alkyl, heteroC₂₋₆alkenyl, heteroC₂₋₆alkynyl, C₃₋₁₀ carbocyclyl, 3-10 membered heterocyclyl, C₆₋₁₀ aryl and 5-10 membered heteroaryl, or two R^{ff} groups are joined to form a 3-10 membered heterocyclyl or 5-10 membered heteroaryl ring, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups; and
each instance of R^{gg} is, independently, halogen, -CN, -NO₂, -N₃, -SO₂H, -SO₃H, -OH, -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, - NH₂(C₁₋₆ alkyl)⁺X⁻, -NH₃⁺X⁻, -N(OC₁₋₆ alkyl)(C₁₋₆ alkyl), -N(OH)(C₁₋₆ alkyl), -NH(OH), - SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, -OC(=O)NH(C₁₋₆ alkyl), - NHC(=O)( C₁₋₆ alkyl), -N(C₁₋₆ alkyl)C(=O)( C₁₋₆ alkyl), -NHCO₂(C₁₋₆ alkyl), - NHC(=O)N(C₁₋₆ alkyl)₂, -NHC(=O)NH(C₁₋₆ alkyl), -NHC(=O)NH₂, -C(=NH)O(C₁₋₆ alkyl),-OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, -OC(NH)NH(C₁₋₆ alkyl), -OC(NH)NH₂, - NHC(NH)N(C₁₋₆ alkyl)₂, -NHC(=NH)NH₂, -NHSO₂(C₁₋₆ alkyl), -SO₂N(C₁₋₆ alkyl)₂, - SO₂NH(C₁₋₆ alkyl), -SO₂NH₂,-SO₂C₁₋₆ alkyl, -SO₂OC₁₋₆ alkyl, -OSO₂C₁₋₆ alkyl, -SOC₁₋₆ alkyl, -Si(C₁₋₆ alkyl)₃, -OSi(C₁₋₆ alkyl)₃ -C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ perhaloalkyl, C₂₋₆ alkenyl, C₂₋₆ alkynyl, heteroC₁₋₆alkyl, heteroC₂₋₆alkenyl, heteroC₂₋₆alkynyl, C₃₋₁₀ carbocyclyl, C₆₋₁₀ aryl, 3-10 membered heterocyclyl, 5-10 membered heteroaryl; or two geminal R^{gg} substituents can be joined to form =O or =S; wherein X⁻ is a counterion.

As used herein, the term "halo" or "halogen" refers to fluorine (fluoro, -F), chlorine (chloro, -Cl), bromine (bromo, -Br), or iodine (iodo, -I).

In certain embodiments, the substituent present on the nitrogen atom is an nitrogen protecting group (also referred to herein as an "amino protecting group"). Nitrogen protecting groups include, but are not limited to, -OH, -OR^{aa}, -N(R^{cc})₂, -C(=O)R^{aa}, -C(=O)N(R^{cc})₂, - CO₂R^{aa}, -SO₂R^{aa}, -C(=NR^{cc})R^{aa}, -C(=NR^{cc})OR^{aa}, -C(=NR^{cc})N(R^{cc})₂, -SO₂N(R^{cc})₂, -SO₂R^{cc}, - SO₂OR^{cc}, -SOR^{aa}, -C(=S)N(R^{cc})₂, -C(=O)SR^{cc}, -C(=S)SR^{cc}, C₁₋₁₀ alkyl *(e.g.,* alkyl, aralkyl, heteroaralkyl), C₂₋₁₀ alkenyl, C₂₋₁₀ alkynyl, C₁₋₁₀ heteroalkyl, C₂₋₁₀ heteroalkenyl, C₂₋₁₀ heteroalkynyl, C₃₋₁₀ carbocyclyl, 3-14 membered heterocyclyl, C₆₋₁₄ aryl, and 5-14 membered heteroaryl groups, wherein each alkyl, alkenyl, alkynyl, heteroalkyl, heteroalkenyl, heteroalkynyl, carbocyclyl, heterocyclyl, aralkyl, aryl, and heteroaryl is independently substituted with 0, 1, 2, 3, 4, or 5 R^{dd} groups, and wherein R^{aa}, R^{bb}, R^{cc} and R^{dd} are as described herein. Nitrogen protecting groups are well known in the art and include those described in detail in *Protecting Groups in Organic Synthesis,* T. W. Greene and P. G. M. Wuts, 3^{rd} edition, John Wiley & Sons, 1999.

For example, nitrogen protecting groups such as amide groups (e.g., -C(=O)R^{aa}) include, but are not limited to, formamide, acetamide, chloroacetamide, trichloroacetamide, trifluoroacetamide, phenylacetamide, 3-phenylpropanamide, picolinamide, 3-pyridylcarboxamide, N-benzoylphenylalanyl derivative, benzamide, p-phenylbenzamide, o-nitophenylacetamide, o-nitrophenoxyacetamide, acetoacetamide, (*N*'-dithiobenzyloxyacylamino)acetamide, 3-(p-hydroxyphenyl)propanamide, 3-(*o-*nitrophenyl)propanamide, 2-methyl-2-(o-nitrophenoxy)propanamide, 2-methyl-2-(o-phenylazophenoxy)propanamide, 4-chlorobutanamide, 3-methyl-3-nitrobutanamide, o-nitrocinnamide, N-acetylmethionine derivative, o-nitrobenzamide and o-(benzoyloxymethyl)benzamide.

Nitrogen protecting groups such as carbamate groups (e.g., -C(=O)OR^{aa}) include, but are not limited to, methyl carbamate, ethyl carbamante, 9-fluorenylmethyl carbamate (Fmoc), 9-(2-sulfo)fluorenylmethyl carbamate, 9-(2,7-dibromo)fluoroenylmethyl carbamate, 2,7-di-t-butyl-[9-(10,10-dioxo-10,10,10,10-tetrahydrothioxanthyl)]methyl carbamate (DBD-Tmoc), 4-methoxyphenacyl carbamate (Phenoc), 2,2,2-trichloroethyl carbamate (Troc), 2-trimethylsilylethyl carbamate (Teoc), 2-phenylethyl carbamate (hZ), 1-(1-adamantyl)-1-methylethyl carbamate (Adpoc), 1,1-dimethyl-2-haloethyl carbamate, 1,1-dimethyl-2,2-dibromoethyl carbamate (DB-*t*-BOC), 1,1-dimethyl-2,2,2-trichloroethyl carbamate (TCBOC), 1-methyl-1-(4-biphenylyl)ethyl carbamate (Bpoc), 1-(3,5-di-t-butylphenyl)-1-methylethyl carbamate (*t-Bumeoc),* 2-(2'- and 4'-pyridyl)ethyl carbamate (Pyoc), 2-*(N,N-*dicyclohexylcarboxamido)ethyl carbamate, t-butyl carbamate (BOC), 1-adamantyl carbamate (Adoc), vinyl carbamate (Voc), allyl carbamate (Alloc), 1-isopropylallyl carbamate (Ipaoc), cinnamyl carbamate (Coc), 4-nitrocinnamyl carbamate (Noc), 8-quinolyl carbamate, N-hydroxypiperidinyl carbamate, alkyldithio carbamate, benzyl carbamate (Cbz), *p-*methoxybenzyl carbamate (Moz), p-nitobenzyl carbamate, p-bromobenzyl carbamate, p-chlorobenzyl carbamate, 2,4-dichlorobenzyl carbamate, 4-methylsulfinylbenzyl carbamate (Msz), 9-anthrylmethyl carbamate, diphenylmethyl carbamate, 2-methylthioethyl carbamate, 2-methylsulfonylethyl carbamate, 2-(p-toluenesulfonyl)ethyl carbamate, [2-(1,3-dithianyl)]methyl carbamate (Dmoc), 4-methylthiophenyl carbamate (Mtpc), 2,4-dimethylthiophenyl carbamate (Bmpc), 2-phosphonioethyl carbamate (Peoc), 2-triphenylphosphonioisopropyl carbamate (Ppoc), 1,1-dimethyl-2-cyanoethyl carbamate, m-chloro-p-acyloxybenzyl carbamate, p-(dihydroxyboryl)benzyl carbamate, 5-benzisoxazolylmethyl carbamate, 2-(trifluoromethyl)-6-chromonylmethyl carbamate (Tcroc), m-nitrophenyl carbamate, 3,5-dimethoxybenzyl carbamate, o-nitrobenzyl carbamate, 3,4-dimethoxy-6-nitrobenzyl carbamate, phenyl(o-nitrophenyl)methyl carbamate, t-amyl carbamate, S-benzyl thiocarbamate, p-cyanobenzyl carbamate, cyclobutyl carbamate, cyclohexyl carbamate, cyclopentyl carbamate, cyclopropylmethyl carbamate, p-decyloxybenzyl carbamate, 2,2-dimethoxyacylvinyl carbamate, o-(N,N-dimethylcarboxamido)benzyl carbamate, 1,1-dimethyl-3-(*N*,*N*-dimethylcarboxamido)propyl carbamate, 1,1-dimethylpropynyl carbamate, di(2-pyridyl)methyl carbamate, 2-furanylmethyl carbamate, 2-iodoethyl carbamate, isoborynl carbamate, isobutyl carbamate, isonicotinyl carbamate, *p-(p* '-methoxyphenylazo)benzyl carbamate, 1-methylcyclobutyl carbamate, 1-methylcyclohexyl carbamate, 1-methyl-1-cyclopropylmethyl carbamate, 1-methyl-1-(3,5-dimethoxyphenyl)ethyl carbamate, 1-methyl-1-(p-phenylazophenyl)ethyl carbamate, 1-methyl-1-phenylethyl carbamate, 1-methyl-1-(4-pyridyl)ethyl carbamate, phenyl carbamate, p-(phenylazo)benzyl carbamate, 2,4,6-tri-t-butylphenyl carbamate, 4-(trimethylammonium)benzyl carbamate, and 2,4,6-trimethylbenzyl carbamate.

Nitrogen protecting groups such as sulfonamide groups *(e.g.,* -S(=O)₂R^{aa}) include, but are not limited to, p-toluenesulfonamide (Ts), benzenesulfonamide, 2,3,6,-trimethyl-4-methoxybenzenesulfonamide (Mtr), 2,4,6-trimethoxybenzenesulfonamide (Mtb), 2,6-dimethyl-4-methoxybenzenesulfonamide (Pme), 2,3,5,6-tetramethyl-4-methoxybenzenesulfonamide (Mte), 4-methoxybenzenesulfonamide (Mbs), 2,4,6-trimethylbenzenesulfonamide (Mts), 2,6-dimethoxy-4-methylbenzenesulfonamide (iMds), 2,2,5,7,8-pentamethylchroman-6-sulfonamide (Pmc), methanesulfonamide (Ms), β-trimethylsilylethanesulfonamide (SES), 9-anthracenesulfonamide, 4-(4',8'-dimethoxynaphthylmethyl)benzenesulfonamide (DNMBS), benzylsulfonamide, trifluoromethylsulfonamide, and phenacylsulfonamide.

Other nitrogen protecting groups include, but are not limited to, phenothiazinyl-(10)-acyl derivative, N'-p-toluenesulfonylaminoacyl derivative, N'-phenylaminothioacyl derivative, N-benzoylphenylalanyl derivative, N-acetylmethionine derivative, 4,5-diphenyl-3-oxazolin-2-one, N-phthalimide, N-dithiasuccinimide (Dts), N-2,3-diphenylmaleimide, *N*-2,5-dimethylpyrrole, *N*-1,1,4,4-tetramethyldisilylazacyclopentane adduct (STABASE), 5-substituted 1,3-dimethyl-1,3,5-triazacyclohexan-2-one, 5-substituted 1,3-dibenzyl-1,3,5-triazacyclohexan-2-one, 1-substituted 3,5-dinitro-4-pyridone, N-methylamine, N-allylamine, N-[2-(trimethylsilyl)ethoxy]methylamine (SEM), N-3-acetoxypropylamine, *N*-(1-isopropyl-4-nitro-2-oxo-3-pyroolin-3-yl)amine, quaternary ammonium salts, N-benzylamine, N-di(4-methoxyphenyl)methylamine, N-5-dibenzosuberylamine, N-triphenylmethylamine (Tr), *N-*[(4-methoxyphenyl)diphenylmethyl]amine (MMTr), N-9-phenylfluorenylamine (PhF), N-2,7-dichloro-9-fluorenylmethyleneamine, N-ferrocenylmethylamino (Fcm), N-2-picolylamino *N*'-oxide, N-1,1-dimethylthiomethyleneamine, N-benzylideneamine, *N-p-*methoxybenzylideneamine, N-diphenylmethyleneamine, *N-[(2-*pyridyl)mesityl]methyleneamine, *N*-(*N',N'*-dimethylaminomethylene)amine, *N,N'-*isopropylidenediamine, N-p-nitrobenzylideneamine, N-salicylideneamine, *N*-5-chlorosalicylideneamine, *N*-(5-chloro-2-hydroxyphenyl)phenylmethyleneamine, N-cyclohexylideneamine, N-(5,5-dimethyl-3-oxo-1-cyclohexenyl)amine, N-borane derivative, N-diphenylborinic acid derivative, N-[phenyl(pentaacylchromium- or tungsten)acyl]amine, N-copper chelate, N-zinc chelate, N-nitroamine, N-nitrosoamine, amine *N*-oxide, diphenylphosphinamide (Dpp), dimethylthiophosphinamide (Mpt), diphenylthiophosphinamide (Ppt), dialkyl phosphoramidates, dibenzyl phosphoramidate, diphenyl phosphoramidate, benzenesulfenamide, o-nitrobenzenesulfenamide (Nps), 2,4-dinitrobenzenesulfenamide, pentachlorobenzenesulfenamide, 2-nitro-4-methoxybenzenesulfenamide, triphenylmethylsulfenamide, and 3-nitropyridinesulfenamide (Npys).

Exemplary oxygen protecting groups include, but are not limited to, methyl, methoxylmethyl (MOM), methylthiomethyl (MTM), t-butylthiomethyl, (phenyldimethylsilyl)methoxymethyl (SMOM), benzyloxymethyl (BOM), *p-*methoxybenzyloxymethyl (PMBM), (4-methoxyphenoxy)methyl (p-AOM), guaiacolmethyl (GUM), t-butoxymethyl, 4-pentenyloxymethyl (POM), siloxymethyl, 2-methoxyethoxymethyl (MEM), 2,2,2-trichloroethoxymethyl, bis(2-chloroethoxy)methyl, 2-(trimethylsilyl)ethoxymethyl (SEMOR), tetrahydropyranyl (THP), 3-bromotetrahydropyranyl, tetrahydrothiopyranyl, 1-methoxycyclohexyl, 4-methoxytetrahydropyranyl (MTHP), 4-methoxytetrahydrothiopyranyl, 4-methoxytetrahydrothiopyranyl S,S-dioxide, 1-[(2-chloro-4-methyl)phenyl]-4-methoxypiperidin-4-yl (CTMP), 1,4-dioxan-2-yl, tetrahydrofuranyl, tetrahydrothiofuranyl, 2,3,3a,4,5,6,7,7a-octahydro-7,8,8-trimethyl-4,7-methanobenzofuran-2-yl, 1-ethoxyethyl, 1-(2-chloroethoxy)ethyl, 1-methyl-1-methoxyethyl, 1-methyl-1-benzyloxyethyl, 1-methyl-1-benzyloxy-2-fluoroethyl, 2,2,2-trichloroethyl, 2-trimethylsilylethyl, 2-(phenylselenyl)ethyl, t-butyl, allyl, p-chlorophenyl, p-methoxyphenyl, 2,4-dinitrophenyl, benzyl (Bn), p-methoxybenzyl, 3,4-dimethoxybenzyl, o-nitrobenzyl, p-nitrobenzyl, p-halobenzyl, 2,6-dichlorobenzyl, p-cyanobenzyl, p-phenylbenzyl, 2-picolyl, 4-picolyl, 3-methyl-2-picolyl N-oxido, diphenylmethyl, p,p '-dinitrobenzhydryl, 5-dibenzosuberyl, triphenylmethyl, α-naphthyldiphenylmethyl, p-methoxyphenyldiphenylmethyl, di(p-methoxyphenyl)phenylmethyl, tri(*p-*methoxyphenyl)methyl, 4-(4'-bromophenacyloxyphenyl)diphenylmethyl, 4,4',4"-tris(4,5-dichlorophthalimidophenyl)methyl, 4,4',4"-tris(levulinoyloxyphenyl)methyl, 4,4',4"-tris(benzoyloxyphenyl)methyl, 3-(imidazol-1-yl)bis(4',4"-dimethoxyphenyl)methyl, 1,1-bis(4-methoxyphenyl)-1'-pyrenylmethyl, 9-anthryl, 9-(9-phenyl)xanthenyl, 9-(9-phenyl-10-oxo)anthryl, 1,3-benzodithiolan-2-yl, benzisothiazolyl S,S-dioxido, trimethylsilyl (TMS), triethylsilyl (TES), triisopropylsilyl (TIPS), dimethylisopropylsilyl (IPDMS), diethylisopropylsilyl (DEIPS), dimethylthexylsilyl, t-butyldimethylsilyl (TBDMS), *t-*butyldiphenylsilyl (TBDPS), tribenzylsilyl, tri-p-xylylsilyl, triphenylsilyl, diphenylmethylsilyl (DPMS), *t*-butylmethoxyphenylsilyl (TBMPS), formate, benzoylformate, acetate, chloroacetate, dichloroacetate, trichloroacetate, trifluoroacetate, methoxyacetate, triphenylmethoxyacetate, phenoxyacetate, p-chlorophenoxyacetate, 3-phenylpropionate, 4-oxopentanoate (levulinate), 4,4-(ethylenedithio)pentanoate (levulinoyldithioacetal), pivaloate, adamantoate, crotonate, 4-methoxycrotonate, benzoate, p-phenylbenzoate, 2,4,6-trimethylbenzoate (mesitoate), methyl carbonate, 9-fluorenylmethyl carbonate (Fmoc), ethyl carbonate, 2,2,2-trichloroethyl carbonate (Troc), 2-(trimethylsilyl)ethyl carbonate (TMSEC), 2-(phenylsulfonyl) ethyl carbonate (Psec), 2-(triphenylphosphonio) ethyl carbonate (Peoc), isobutyl carbonate, vinyl carbonate, allyl carbonate, t-butyl carbonate (BOC), p-nitrophenyl carbonate, benzyl carbonate, p-methoxybenzyl carbonate, 3,4-dimethoxybenzyl carbonate, o-nitrobenzyl carbonate, p-nitrobenzyl carbonate, S-benzyl thiocarbonate, 4-ethoxy-1-napththyl carbonate, methyl dithiocarbonate, 2-iodobenzoate, 4-azidobutyrate, 4-nitro-4-methylpentanoate, o-(dibromomethyl)benzoate, 2-formylbenzenesulfonate, 2-(methylthiomethoxy)ethyl, 4-(methylthiomethoxy)butyrate, 2-(methylthiomethoxymethyl)benzoate, 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenylacetate, isobutyrate, monosuccinoate, (E)-2-methyl-2-butenoate, o-(methoxyacyl)benzoate, α-naphthoate, nitrate, alkyl *N,N,N',N'-*tetramethylphosphorodiamidate, alkyl N-phenylcarbamate, borate, dimethylphosphinothioyl, alkyl 2,4-dinitrophenylsulfenate, sulfate, methanesulfonate (mesylate), benzylsulfonate, and tosylate (Ts).

Sulfur protecting groups include, but are not limited to, -R^{aa}, -N(R^{bb})₂, -C(=O)SR^{aa}, -C(=O)R^{aa}, -CO₂R^{aa}, -C(=O)N(R^{bb})₂, -C(=NR^{bb})R^{aa}, -C(=NR^{bb})OR^{aa}, -C(=NR^{bb})N(R^{bb})₂,-S(=O)R^{aa}, -SO₂R^{aa}, -Si(R^{aa})₃, -P(R^{cc})₂, -P(R^{cc})₃, -P(=O)₂R^{aa}, -P(=O)(R^{aa})₂, -P(=O)(OR^{cc})₂, - P(=O)₂N(R^{bb})₂, and -P(=O)(NR^{bb})₂, wherein R^{aa}, R^{bb}, and R^{cc} are as described herein. Sulfur protecting groups are well known in the art and include those described in detail in Protecting Groups in Organic Synthesis, T. W. Greene and P. G. M. Wuts, 3rd edition, John Wiley & Sons, 1999.

As used herein, a "leaving group", or "LG", is a term understood in the art to refere to a molecular fragment that departs with a pair of electrons upon heterolytic bond cleavage, wherein the molecular fragment is an anion or neutral molecule. See, for example, Smith, March Advanced Organic Chemistry 6th ed. (501-502). Examples of suitable leaving groups include, but are not limited to, halides (such as chloride, bromide, or iodide), alkoxycarbonyloxy, aryloxycarbonyloxy, alkanesulfonyloxy, arenesulfonyloxy, alkyl-carbonyloxy (e.g., acetoxy), arylcarbonyloxy, aryloxy, methoxy, N,O-dimethylhydroxylamino, pixyl, haloformates, -NO₂, trialkylammonium, and aryliodonium salts.

These and other exemplary substituents are described in more detail in the Detailed Description, Examples, and Claims. The invention is not intended to be limited in any manner by the above exemplary listing of substituents.

### Other Definitions

The following definitions are more general terms used throughout the present application.

As used herein, the term "pharmaceutically acceptable salt" refers to those salts which are, within the scope of sound medical judgment, suitable for use in contact with the tissues of humans and lower animals without undue toxicity, irritation, and allergic response, and are commensurate with a reasonable benefit/risk ratio. Pharmaceutically acceptable salts are well known in the art. For example, Berge *et al.* describe pharmaceutically acceptable salts in detail in J. Pharmaceutical Sciences, 1977, 66, 1-19. Pharmaceutically acceptable salts of the compounds of this invention include those derived from suitable inorganic and organic acids and bases. Examples of pharmaceutically acceptable, nontoxic acid addition salts are salts of an amino group formed with inorganic acids, such as hydrochloric acid, hydrobromic acid, phosphoric acid, sulfuric acid, and perchloric acid or with organic acids, such as acetic acid, oxalic acid, maleic acid, tartaric acid, citric acid, succinic acid, or malonic acid or by using other methods known in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, and valerate salts. Salts derived from appropriate bases include alkali metal, alkaline earth metal, ammonium, and N⁺(C₁₋₄ alkyl)₄⁻ salts. Representative alkali or alkaline earth metal salts include sodium, lithium, potassium, calcium, and magnesium. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

The term "solvate" refers to forms of the compound, or a salt thereof, that are associated with a solvent, usually by a solvolysis reaction. This physical association may include hydrogen bonding. Conventional solvents include water, methanol, ethanol, acetic acid, DMSO, THF, and diethyl ether. Compounds of the invention may be prepared, *e.g.,* in crystalline form, and may be solvated. Suitable solvates include pharmaceutically acceptable solvates and further include both stoichiometric solvates and non-stoichiometric solvates. In certain instances, the solvate will be capable of isolation, for example, when one or more solvent molecules are incorporated in the crystal lattice of a crystalline solid. "Solvate" encompasses both solution-phase and isolable solvates. Representative solvates include hydrates, ethanolates, and methanolates.

The term "hydrate" refers to a compound that is associated with water. Typically, the number of the water molecules contained in a hydrate of a compound is in a definite ratio to the number of the compound molecules in the hydrate. Therefore, a hydrate of a compound may be represented, for example, by the general formula R·x H₂O, wherein R is the compound and wherein x is a number greater than 0. A given compound may form more than one type of hydrates, including, e.g., monohydrates (x is 1), lower hydrates (x is a number greater than 0 and smaller than 1, e.g., hemihydrates (R·0.5 H₂O)), and polyhydrates (x is a number greater than 1, e.g., dihydrates (R·2 H₂O) and hexahydrates (R·6 H₂O)).

The term "tautomers" refer to compounds that are interchangeable forms of a particular compound structure, and that vary in the displacement of hydrogen atoms and electrons. Thus, two structures may be in equilibrium through the movement of π electrons and an atom (usually H). For example, enols and ketones are tautomers because they are rapidly interconverted by treatment with either acid or base. Another example of tautomerism is the aci- and nitro- forms of phenylnitromethane, that are likewise formed by treatment with acid or base. Tautomeric forms may be relevant to the attainment of the optimal chemical reactivity and biological activity of a compound of interest.

It is also to be understood that compounds that have the same molecular formula but differ in the nature or sequence of bonding of their atoms or the arrangement of their atoms in space are termed "isomers." Isomers that differ in the arrangement of their atoms in space are termed "stereoisomers".

Stereoisomers that are not mirror images of one another are termed "diastereomers" and those that are non-superimposable mirror images of each other are termed "enantiomers". When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory *(i.e.,* as (+) or (-)-isomers respectively). A chiral compound can exist as either individual enantiomer or as a mixture thereof. A mixture containing equal proportions of the enantiomers is called a "racemic mixture".

The term "polymorphs" refers to a crystalline form of a compound (or a salt, hydrate, or solvate thereof). All polymorphs have the same elemental composition. Different crystalline forms usually have different X-ray diffraction patterns, infrared spectra, melting points, density, hardness, crystal shape, optical and electrical properties, stability, and solubility. Recrystallization solvent, rate of crystallization, storage temperature, and other factors may cause one crystal form to dominate. Various polymorphs of a compound can be prepared by crystallization under different conditions.

The term "prodrugs" refers to compounds that have cleavable groups and become by solvolysis or under physiological conditions the compounds of the invention, which are pharmaceutically active *in vivo.* Such examples include, but are not limited to, choline ester derivatives, and N-alkylmorpholine esters . Other derivatives of the compounds of this invention have activity in both their acid and acid derivative forms, but in the acid sensitive form often offers advantages of solubility, tissue compatibility, or delayed release in the mammalian organism (see, Bundgard, H., Design of Prodrugs, pp. 7-9, 21-24, Elsevier, Amsterdam 1985). Prodrugs include acid derivatives well known to practitioners of the art, such as, for example, esters prepared by reaction of the parent acid with a suitable alcohol, or amides prepared by reaction of the parent acid compound with a substituted or unsubstituted amine, or acid anhydrides, or mixed anhydrides. Simple aliphatic or aromatic esters, amides and anhydrides derived from acidic groups pendant on the compounds of this invention are particular prodrugs. In some cases it is desirable to prepare double ester type prodrugs such as (acyloxy)alkyl esters or ((alkoxycarbonyl)oxy)alkylesters. Particularly the C₁-C₈ alkyl, C₂-C₈ alkenyl, C₂-C₈ alkynyl, aryl, C₇-C₁₂ substituted aryl, and C₇-C₁₂ arylalkyl esters of the compounds of the invention.

### DETAILED DESCRIPTION OF THE INVENTION

In an effort to identify novel treatments for Waldenström's macroglobulinemia, *in vitro* screens were carried out against several kinases *(e.g.,* BTK, HCK, TAK1). These kinases are involved in the regulation of aberrant cell growth associated with this condition. Cell-based screening was also carried out in several disease state model lines of Waldenström's macroglobulinemia (e.g., BCWM.1, MWCL-1). Based on these screening efforts and subsequent lead optimization, compounds of of the invention were identified.

In one aspect, the present invention provides compounds of Formula (**I-a1**) , (I-a2) or (I-a3): 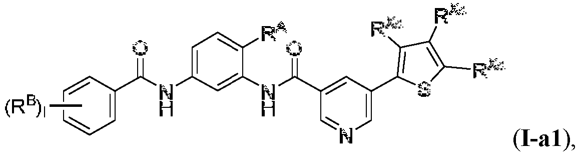 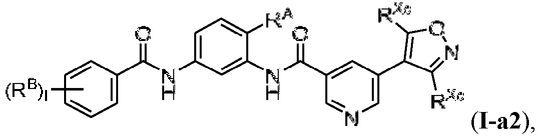 or 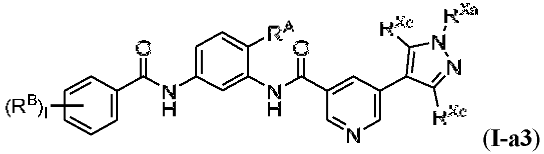 and pharmaceutically acceptable salts, solvates, hydrates, polymorphs, co-crystals, tautomers, or stereoisomers, and prodrugs thereof; wherein:
R^{A} issubstituted or unsubstituted C₁₋₆ alkyl;
each instance of R^{B} is independently selected from the group consisting of optionally substituted alkyl, optionally substituted heterocyclyl, and optionally substituted heteroaryl;
R^{Xa} is optionally substituted C₁₋₆ alkyl;
each instance of R^{Xc} is hydrogen; and
l is 1 or 2;
   carbon atom substituents are independently selected from the group consisting of halogen, -CN, -NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, -C(=O)R^{aa}, -CO₂H, -CHO, -C(OR^{cc})₂, -CO₂R^{aa}, - OC(=O)R^{aa}, -OCO₂R^{aa}, -C(=O)N(R^{bb})₂, -OC(=O)N(R^{bb})₂, -NR^{bb}C(=O)R^{aa}, -NR^{bb}CO₂R^{aa}, - NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa}, - C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa}, - NR^{bb}SO₂R^{aa}, -SO₂N(R^{bb})₂, -SO₂R^{aa}, -SO₂OR^{aa}, -OSO₂R^{aa}, -S(=O)R^{aa}, -OS(=O)R^{aa}, - Si(R^{aa})₃, -OSi(R^{aa})₃ -C(=S)N(R^{bb})₂, -C(=O)SR^{aa}, -C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)₂R^{aa}, -OP(=O)₂R^{aa}, -P(=O)(R^{aa})₂, - OP(=O)(R^{aa})₂, -OP(=O)(OR^{cc})₂, -P(=O)₂N(R^{bb})₂, -OP(=O)₂N(R^{bb})₂, -P(=O)(NR^{bb})₂, - OP(=O)(NR^{bb})₂, -NR^{bb}P(=O)(OR^{cc})₂, -NR^{bb}P(=O)(NR^{bb})₂, -P(R^{cc})₂, -P(R^{cc})₃, -OP(R^{cc})₂, - OP(R^{cc})₃, -B(R^{aa})₂, -B(OR^{cc})₂, -BR^{aa}(OR^{cc}), C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups;
or two geminal hydrogens on a carbon atom are replaced with the group =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb}, or =NOR^{cc};
nitrogen atom substituents are nitrogen protecting groups;
each instance of the nitrogen protecting group is independently -OH, -OR^{aa}, - N(R^{cc})₂, -C(=O)R^{aa}, -C(=O)N(R^{cc})₂, -CO₂R^{aa}, -SO₂R^{aa}, -C(=NR^{cc})R^{aa}, -C(=NR^{cc})OR^{aa}, - C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, - C(=S)SR^{cc}, C₁₋₁₀ alkyl, C₂₋₁₀ alkenyl, C₂₋₁₀ alkynyl, C₁₋₁₀ heteroalkyl, C₂₋₁₀ heteroalkenyl, C₂₋₁₀ heteroalkynyl, C₃₋₁₀ carbocyclyl, 3-14 membered heterocyclyl, C₆₋₁₄ aryl, or 5-14 membered heteroaryl, wherein each of the alkyl, alkenyl, alkynyl, heteroalkyl, heteroalkenyl, heteroalkynyl, carbocyclyl, heterocyclyl, aralkyl, aryl, and heteroaryl is independently substituted with 0, 1, 2, 3, 4, or 5 R^{dd} groups;
each instance of R^{aa} is, independently, selected from C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀alkenyl, heteroC₂₋₁₀alkynyl, C₃₋₁₀ carbocyclyl, 3-14 membered heterocyclyl, C₆₋₁₄ aryl, and 5-14 membered heteroaryl, or two R^{aa} groups are joined to form a 3-14 membered heterocyclyl or 5-14 membered heteroaryl ring, wherein each of the alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups;
each instance of R^{bb} is, independently, selected from hydrogen, -OH, -OR^{aa}, - N(R^{cc})₂, -CN, -C(=O)R^{aa}, -C(=O)N(R^{cc})₂, -CO₂R^{aa}, -SO₂R^{aa}, -C(=NR^{cc})OR^{aa}, - C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, - C(=S)SR^{cc}, -P(=O)₂R^{aa}, -P(=O)(R^{aa})₂, -P(=O)₂N(R^{cc})₂, -P(=O)(NR^{cc})₂, C₁₋₁₀ alkyl, C₁₋₁₀ perhaloalkyl, C₂₋₁₀ alkenyl, C₂₋₁₀ alkynyl, heteroC₁₋₁₀alkyl, heteroC₂₋₁₀alkenyl, heteroC₂₋₁₀alkynyl, C₃₋₁₀ carbocyclyl, 3-14 membered heterocyclyl, C₆₋₁₄ aryl, and 5-14 membered heteroaryl, or two R^{bb} groups are joined to form a 3-14 membered heterocyclyl or 5-14 membered heteroaryl ring, wherein each of the alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups;
each instance of R^{cc} is, independently, selected from hydrogen, C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl, or two R^{cc} groups are joined to form a 3-14 membered heterocyclyl or 5-14 membered heteroaryl ring, wherein each of the alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups;
each instance of R^{dd} is, independently, selected from halogen, -CN, -NO₂, -N₃, - SO₂H, -SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, - SSR^{ee}, -C(=O)R^{ee}, -CO₂H, -CO₂R^{ee}, -OC(=O)R^{ee}, -OCO₂R^{ee}, -C(=O)N(R^{ff})₂, - OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee}, - OC(=NR^{ff})R^{ee}, -OC(=NR^{ff})OR^{ee}, -C(=NR^{ff})N(R^{ff})₂, -OC(=NR^{ff})N(R^{ff})₂, - NR^{ff}C(=NR^{ff})N(R^{ff})₂,-NR^{ff}SO₂R^{ee}, -SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee}, -C(=S)SR^{ee}, -SC(=S)SR^{ee}, -P(=O)₂R^{ee}, - P(=O)(R^{ee})₂, -OP(=O)(R^{ee})₂, -OP(=O)(OR^{ee})₂, C₁₋₆ alkyl, C₁₋₆ perhaloalkyl, C₂₋₆ alkenyl, C₂₋₆ alkynyl, heteroC₁₋₆alkyl, heteroC₂₋₆alkenyl, heteroC₂₋₆alkynyl, C₃₋₁₀ carbocyclyl, 3-10 membered heterocyclyl, C₆₋₁₀ aryl, and 5-10 membered heteroaryl, wherein each of the alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups, or two geminal R^{dd} substituents are joined to form =O or =S;
each instance of R^{ee} is, independently, selected from C₁₋₆ alkyl, C₁₋₆ perhaloalkyl, C₂₋₆ alkenyl, C₂₋₆ alkynyl, heteroC₁₋₆ alkyl, heteroC₂₋₆alkenyl, heteroC₂₋₆ alkynyl, C₃₋₁₀ carbocyclyl, C₆₋₁₀ aryl, 3-10 membered heterocyclyl, and 3-10 membered heteroaryl, wherein each of the alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups;
each instance of R^{ff} is, independently, selected from hydrogen, C₁₋₆ alkyl, C₁₋₆ perhaloalkyl, C₂₋₆ alkenyl, C₂₋₆ alkynyl, heteroC₁₋₆alkyl, heteroC₂₋₆alkenyl, heteroC₂₋₆alkynyl, C₃₋₁₀ carbocyclyl, 3-10 membered heterocyclyl, C₆₋₁₀ aryl, and 5-10 membered heteroaryl, or two R^{ff} groups are joined to form a 3-10 membered heterocyclyl or 5-10 membered heteroaryl ring, wherein each of the alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups;
each instance of R^{gg} is, independently, halogen, -CN, -NO₂, -N₃, -SO₂H, -SO₃H, - OH, -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, -NH₂(C₁₋₆ alkyl)⁺X⁻, -NH₃⁺X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, - OC(=O)NH(C₁₋₆ alkyl), -NHC(=O)( C₁₋₆ alkyl), -N(C₁₋₆ alkyl)C(=O)( C₁₋₆ alkyl), - NHCO₂(C₁₋₆ alkyl), -NHC(=O)N(C₁₋₆ alkyl)₂, -NHC(=O)NH(C₁₋₆ alkyl), -NHC(=O)NH₂, - C(=NH)O(C₁₋₆ alkyl),-OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, -OC(NH)NH(C₁₋₆ alkyl), -OC(NH)NH₂, -NHC(NH)N(C₁₋₆ alkyl)₂, -NHC(=NH)NH₂, -NHSO₂(C₁₋₆ alkyl), - SO₂N(C₁₋₆ alkyl)₂, -SO₂NH(C₁₋₆ alkyl), -SO₂NH₂,-SO₂C₁₋₆ alkyl, -SO₂OC₁₋₆ alkyl, - OSO₂C₁₋₆ alkyl, -SOC₁₋₆ alkyl, -Si(C₁₋₆ alkyl)₃, -OSi(C₁₋₆ alkyl)₃ -C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ perhaloalkyl, C₂₋₆ alkenyl, C₂₋₆ alkynyl, heteroC₁₋₆alkyl, heteroC₂₋₆alkenyl, heteroC₂₋₆alkynyl, C₃₋₁₀ carbocyclyl, C₆₋₁₀ aryl, 3-10 membered heterocyclyl, or 5-10 membered heteroaryl; or two geminal R^{gg} substituents are joined to form =O or =S; and
X⁻ is a counterion.

In certain embodiments, the present invention provides compounds from the group consisting of: 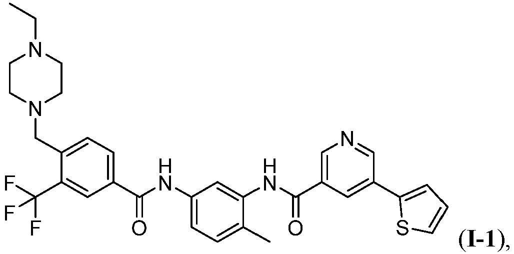 and pharmaceutically acceptable salts thereof.

In another aspect, the present invention provides the compounds of the invention for use in treating Waldenström's macroglobulinemia (WM) in a subject Also provided are the compounds of the invention for use in treating B cell neoplasms, preferably in combination with inhibitors of Bruton's tyrosine kinase (BTK), interleukin-1 receptor-associated kinase 1 (IRAK1), interleukin-1 receptor-associated kinase 4 (IRAK4), bone marrow on X chromosome kinase (BMX), phosphoinositide 3-kinase (PI3K), transforming growth factor b-activated kinase-1 (TAK1), and/or a Src family kinase. In certain embodiments, one or more compounds of the invention are used in combination with an inhibitor of the phosphoinositide 3-kinase delta isoform (PI3Kδ), even more preferably, Bruton's tyrosine kinase (BTK), interleukin-1 receptor-associated kinase 1 (IRAK1), interleukin-1 receptor-associated kinase 4 (IRAK4), and/or transforming growth factor b-activated kinase-1 (TAK1), and/or a Src family kinase. In certain embodiments, combinations of 2, 3, 4, 5, 6,7, 8, 9, 10, or more of the agents described herein are used for use in treating WM. In certain embodiments, the agents described herein are used in combination with kinase inhibitors such as inhibitors of Bruton's tyrosine kinase (BTK), interleukin-1 receptor-associated kinase 1 (IRAK1), interleukin-1 receptor-associated kinase 4 (IRAK4), bone marrow on X chromosome kinase (BMX), phosphoinositide 3-kinase (PI3K), and/or transforming growth factor b-activated kinase-1 (TAK1), and/or a Src family kinase, preferably Bruton's tyrosine kinase (BTK), interleukin-1 receptor-associated kinase 1 (IRAK1), interleukin-1 receptor-associated kinase 4 (IRAK4), and/or transforming growth factor b-activated kinase-1 (TAK1), and/or a Src family kinase.

Waldenstrom's macroglobulinemia (WM) is a distinct clinicopathological entity resulting from the accumulation, predominantly in the bone marrow, of clonally related lymphoplasmacytic cells which secrete a monoclonal IgM protein. This condition is considered to correspond to lymphoplasmacytic lymphoma (LPL) as defined by the World Health Organization classification system. Genetic factors play an important role in the pathogenesis of WM, with 25% of patients demonstrating a family history. IgM monoclonal gammopathy of unknown significance (MGUS) often precedes the development of WM.

As used herein, a B cell neoplasm includes both Hodgkin's lymphoma and non-Hodgkin's lymphomas. Classical Hodgkin's lymphoma (HL) includes various subtypes such as Nodular sclerosing HL, Mixed-cellularity subtype, Lymphocyte-rich or Lymphocytic predominance and Lymphocyte depleted. Examples of B cell non-Hodgkin's lymphomas include, but are not limited to, Waldenström's macroglobulinemia, diffuse large B cell lymphoma, follicular lymphoma, mucosa-associated lymphatic tissue lymphoma (MALT), small cell lymphocytic lymphoma (overlaps with chronic lymphocytic leukemia), mantle cell lymphoma (MCL), Burkitt lymphoma, mediastinal large B cell lymphoma, nodal marginal zone B cell lymphoma (NMZL), splenic marginal zone lymphoma (SMZL), intravascular large B-cell lymphoma, primary effusion lymphoma, and lymphomatoid granulomatosis.

In the present invention, the subject is administered a compound of Formula (**I-a1**), (**I-a2**) or (I-a3): 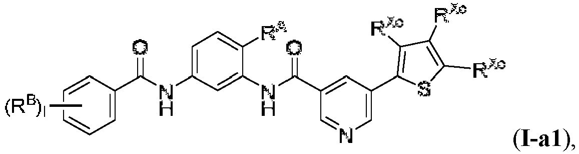 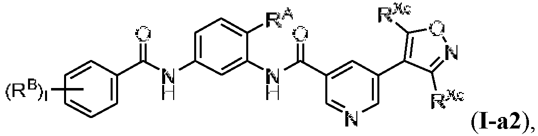 or 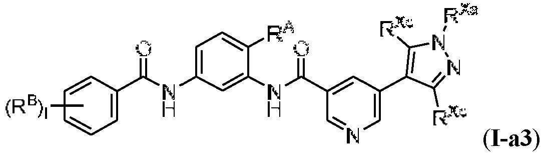 and pharmaceutically acceptable salts, solvates, hydrates, polymorphs, co-crystals, tautomers, or stereoisomers, and prodrugs thereof; wherein:
R^{A} issubstituted or unsubstituted C₁₋₆ alkyl ;
each instance of R^{B} is independently selected from the group consisting of optionally substituted alkyl, optionally substituted heterocyclyl and optionally substituted heteroaryl;
R^{Xa} is optionally substituted C₁₋₆ alkyl;
each instance of R^{Xc} is hydrogen; and
l is 1 or 2;
   carbon atom substituents are independently selected from the group consisting of halogen, -CN, -NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, -C(=O)R^{aa}, -CO₂H, -CHO, -C(OR^{cc})₂, -CO₂R^{aa}, - OC(=O)R^{aa}, -OCO₂R^{aa}, -C(=O)N(R^{bb})₂, -OC(=O)N(R^{bb})₂, -NR^{bb}C(=O)R^{aa}, -NR^{bb}CO₂R^{aa}, - NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa},-C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa},-NR^{bb}SO₂R^{aa}, -SO₂N(R^{bb})₂, -SO₂R^{aa}, -SO₂OR^{aa}, -OSO₂R^{aa}, -S(=O)R^{aa}, -OS(=O)R^{aa}, - Si(R^{aa})₃, -OSi(R^{aa})₃ -C(=S)N(R^{bb})₂, -C(=O)SR^{aa}, -C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)₂R^{aa}, -OP(=O)₂R^{aa}, -P(=O)(R^{aa})₂, - OP(=O)(R^{aa})₂, -OP(=O)(OR^{cc})₂, -P(=O)₂N(R^{bb})₂, -OP(=O)₂N(R^{bb})₂, -P(=O)(NR^{bb})₂, - OP(=O)(NR^{bb})₂, -NR^{bb}P(=O)(OR^{cc})₂, -NR^{bb}P(=O)(NR^{bb})₂, -P(R^{cc})₂, -P(R^{cc})₃, -OP(R^{cc})₂,-OP(R^{cc})₃, -B(R^{aa})₂, -B(OR^{cc})₂, -BR^{aa}(OR^{cc}), C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 **R^{dd}** groups;
or two geminal hydrogens on a carbon atom are replaced with the group =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb}, or =NOR^{cc};
nitrogen atom substituents are nitrogen protecting groups;
each instance of the nitrogen protecting group is independently -OH, -OR^{aa}, - N(R^{cc})₂, -C(=O)R^{aa}, -C(=O)N(R^{cc})₂, -CO₂R^{aa}, -SO₂R^{aa}, -C(=NR^{cc})R^{aa}, -C(=NR^{cc})OR^{aa}, - C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, - C(=S)SR^{cc}, C₁₋₁₀ alkyl, C₂₋₁₀ alkenyl, C₂₋₁₀ alkynyl, C₁₋₁₀ heteroalkyl, C₂₋₁₀ heteroalkenyl, C₂₋₁₀ heteroalkynyl, C₃₋₁₀ carbocyclyl, 3-14 membered heterocyclyl, C₆₋₁₄ aryl, or 5-14 membered heteroaryl, wherein each of the alkyl, alkenyl, alkynyl, heteroalkyl, heteroalkenyl, heteroalkynyl, carbocyclyl, heterocyclyl, aralkyl, aryl, and heteroaryl is independently substituted with 0, 1, 2, 3, 4, or 5 **R^{dd}** groups;
each instance of R^{aa} is, independently, selected from C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀alkenyl, heteroC₂₋₁₀alkynyl, C₃₋₁₀ carbocyclyl, 3-14 membered heterocyclyl, C₆₋₁₄ aryl, and 5-14 membered heteroaryl, or two R^{aa} groups are joined to form a 3-14 membered heterocyclyl or 5-14 membered heteroaryl ring, wherein each of the alkyl, alkenyl, alkynyl, heteroalkyl, heteroalkenyl, heteroalkynyl, carbocyclyl, heterocyclyl, aryl, and heteroaryl is independently substituted with 0, **1, 2,** 3, 4, or 5 **R^{dd}** groups;
each instance of R^{bb} is, independently, selected from hydrogen, -OH, -OR^{aa}, - N(R^{cc})₂, -CN, -C(=O)R^{aa}, -C(=O)N(R^{cc})₂, -CO₂R^{aa}, -SO₂R^{aa}, -C(=NR^{cc})OR^{aa}, - C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, - C(=S)SR^{cc}, -P(=O)₂R^{aa}, -P(=O)(R^{aa})₂, -P(=O)₂N(R^{cc})₂, -P(=O)(NR^{cc})₂, C₁₋₁₀ alkyl, C₁₋₁₀ perhaloalkyl, C₂₋₁₀ alkenyl, C₂₋₁₀ alkynyl, heteroC₁₋₁₀alkyl, heteroC₂₋₁₀alkenyl, heteroC₂₋₁₀alkynyl, C₃₋₁₀ carbocyclyl, 3-14 membered heterocyclyl, C₆₋₁₄ aryl, and 5-14 membered heteroaryl, or two R^{bb} groups are joined to form a 3-14 membered heterocyclyl or 5-14 membered heteroaryl ring, wherein each of the alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups;
each instance of R^{cc} is, independently, selected from hydrogen, C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl, or two R^{cc} groups are joined to form a 3-14 membered heterocyclyl or 5-14 membered heteroaryl ring, wherein each of the alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups;
each instance of R^{dd} is, independently, selected from halogen, -CN, -NO₂, -N₃, - SO₂H, -SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, - SSR^{ee}, -C(=O)R^{ee}, -CO₂H, -CO₂R^{ee}, -OC(=O)R^{ee}, -OCO₂R^{ee}, -C(=O)N(R^{ff})₂, - OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee}, - OC(=NR^{ff})R^{ee}, -OC(=NR^{ff})OR^{ee}, -C(=NR^{ff})N(R^{ff})₂, -OC(=NR^{ff})N(R^{ff})₂, - NR^{ff}C(=NR^{ff})N(R^{ff})₂,-NR^{ff}SO₂R^{ee}, -SO₂N(R^{ff})₂, -SO₂R^{ee}, -SO₂OR^{ee}, -OSO₂R^{ee}, - S(=O)R^{ee}, -Si(R^{ee})₃, -OSi(R^{ee})₃, -C(=S)N(R^{ff})₂, -C(=O)SR^{ee}, -C(=S)SR^{ee}, -SC(=S)SR^{ee}, - P(=O)₂R^{ee}, - P(=O)(R^{ee})₂, -OP(=O)(R^{ee})₂, -OP(=O)(OR^{ee})₂, C₁₋₆ alkyl, C₁₋₆ perhaloalkyl, C₂₋₆ alkenyl, C_{2- 6} alkynyl, heteroC₁₋₆alkyl, heteroC₂₋₆alkenyl, heteroC₂₋₆alkynyl, C₃₋₁₀ carbocyclyl, 3-10 membered heterocyclyl, C₆₋₁₀ aryl, and 5-10 membered heteroaryl, wherein each of the alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups, or two geminal R^{dd} substituents are joined to form =O or =S;
each instance of R^{ee} is, independently, selected from C₁₋₆ alkyl, C₁₋₆ perhaloalkyl, C_{2- 6} alkenyl, C₂₋₆ alkynyl, heteroC₁₋₆ alkyl, heteroC₂₋₆alkenyl, heteroC₂₋₆ alkynyl, C₃₋₁₀ carbocyclyl, C₆₋₁₀ aryl, 3-10 membered heterocyclyl, and 3-10 membered heteroaryl, wherein each of the alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups;
each instance of R^{ff} is, independently, selected from hydrogen, C₁₋₆ alkyl, C₁₋₆ perhaloalkyl, C₂₋₆ alkenyl, C₂₋₆ alkynyl, heteroC₁₋₆alkyl, heteroC₂₋₆alkenyl, heteroC₂₋₆alkynyl, C₃₋₁₀ carbocyclyl, 3-10 membered heterocyclyl, C₆₋₁₀ aryl, and 5-10 membered heteroaryl, or two R^{ff} groups are joined to form a 3-10 membered heterocyclyl or 5-10 membered heteroaryl ring, wherein each of the alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups;
each instance of R^{gg} is, independently, halogen, -CN, -NO₂, -N₃, -SO₂H, -SO₃H, - **OH,** -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, -NH₂(C₁₋₆ alkyl)⁺X, -NH₃ ⁺X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, - OC(=O)NH(C₁₋₆ alkyl), -NHC(=O)( C₁₋₆ alkyl), -N(C₁₋₆ alkyl)C(=O)( C₁₋₆ alkyl), - NHCO₂(C₁₋₆ alkyl), -NHC(=O)N(C₁₋₆ alkyl)₂, -NHC(=O)NH(C₁₋₆ alkyl), -NHC(=O)NH₂, C(=NH)O(C₁₋₆ alkyl),-OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, - OC(NH)NH(C₁₋₆ alkyl), -OC(NH)NH₂, -NHC(NH)N(C₁₋₆ alkyl)₂, -NHC(=NH)NH₂, -NHSO₂(C₁₋₆ alkyl), - SO₂N(C₁₋₆ alkyl)₂, -SO₂NH(C₁₋₆ alkyl), -SO₂NH₂,-SO₂C₁₋₆ alkyl, -SO₂OC₁₋₆ alkyl, - OSO₂C₁₋₆ alkyl, -SOC₁₋₆ alkyl, -Si(C₁₋₆ alkyl)₃, -OSi(C₁₋₆ alkyl)₃ -C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ perhaloalkyl, C₂₋₆ alkenyl, C₂₋₆ alkynyl, heteroC₁₋₆alkyl, heteroC₂₋₆alkenyl, heteroC₂₋₆alkynyl, C₃₋₁₀ carbocyclyl, C₆₋₁₀ aryl, 3-10 membered heterocyclyl, or 5- 10 membered heteroaryl; or two geminal R^{gg} substituents are joined to form =O or =S; and
X⁻ is a counterion.

In certain embodiments, the subject is administered a compound (**I-1**): 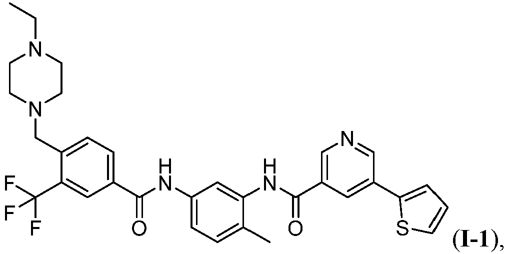 or a pharmaceutically acceptable salt thereof.

Compounds of Formula (**I-a1**), (I-a2) or (I-a3) include a phenyl ring substituted with one R^{A} group.

In certain embodiments, R^{A} is substituted C₁₋₆ alkyl. In certain embodiments, R^{A} is unsubstituted C₁₋₆ alkyl. In certain embodiments, R^{A} is methyl. In certain embodiments, R^{A} is ethyl. In certain embodiments, R^{A} is propyl. In certain embodiments, R^{A} is butyl.

Compounds of Formula (I-a1), (I-a2) or (I-a3) include a phenyl ring substituted with one or two R^{B} group. In certain embodiments, l is 1.

In certain embodiments, at least one R^{B} is substituted alkyl. In certain embodiments, at least one R^{B} is unsubstituted alkyl. In certain embodiments, at least one R^{B} is substituted C₁₋₆ alkyl. In certain embodiments, at least one R^{B} is unsubstituted C₁₋₆ alkyl. In certain embodiments, at least one R^{B} is methyl. In certain embodiments, at least one R^{B} is ethyl. In certain embodiments, at least one R^{B} is propyl. In certain embodiments, at least one R^{B} is 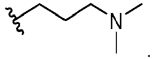 In certain embodiments, at least one R^{B} is 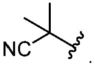 In certain embodiments, at least one R^{B} is butyl. In certain embodiments, at least one R^{B} is substituted heterocyclyl. In certain embodiments, at least one R^{B} is unsubstituted heterocyclyl. In certain embodiments, at least one R^{B} is substituted piperidine. In certain embodiments, at least one R^{B} is unsubstituted piperidine. In certain embodiments, at least one R^{B} substituted piperizine. In certain embodiments, at least one R^{B} unsubstituted piperizine. In certain embodiments, at least one R^{B} substituted pyrrolidine. In certain embodiments, at least one R^{B} unsubstituted pyrrolidine. In certain embodiments, at least one R^{B} is substituted morpholine. In certain embodiments, at least one R^{B} is unsubstituted morpholine. In certain embodiments, at least one R^{B} is substituted diazapane. In certain embodiments, at least one R^{B} is unsubstituted diazapane. In certain embodiments, at least one R^{B} is 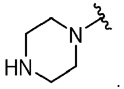 In certain embodiments, at least one R^{B} is 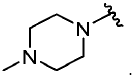 In certain embodiments, at least one R^{B} is 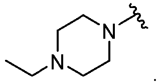 In certain embodiments, at least one R^{B} is 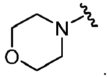 In certain embodiments, at least one R^{B} is substituted -(CH₂)(heterocyclyl). In certain embodiments, at least one R^{B} is unsubstituted - (CH₂)( heterocyclyl). In certain embodiments, at least one R^{B} is 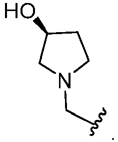 In certain embodiments, at least one R^{B} is 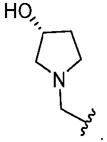 In certain embodiments, at least one R^{B} is 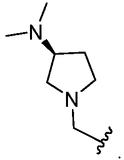 In certain embodiments, at least one R^{B} is 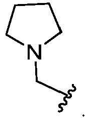 In certain embodiments, at least one R^{B} is 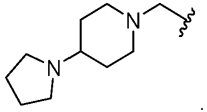 In certain embodiments, at least one R^{B} is 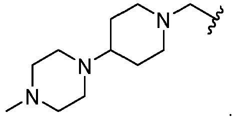 In certain embodiments, at least one R^{B} is 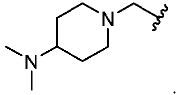 In certain embodiments, at least one R^{B} is 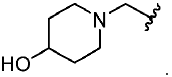 In certain embodiments, at least one R^{B} is 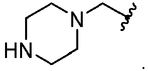 In certain embodiments, at least one R^{B} is 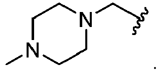 In certain embodiments, at least one R^{B} is 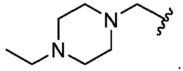 In certain embodiments, at least one R^{B} is 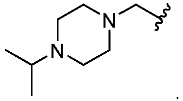 In certain embodiments, at least one R^{B} is 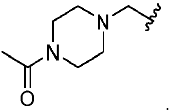 In certain embodiments, at least one R^{B} is 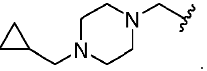 In certain embodiments, at least one R^{B} is 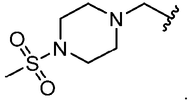 In certain embodiments, at least one R^{B} is 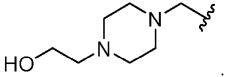 In certain embodiments, at least one R^{B} is 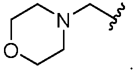 In certain embodiments, at least one R^{B} is 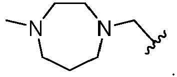 In certain embodiments, at least one R^{B} is substituted - (CH₂)₂(heterocyclyl). In certain embodiments, at least one R^{B} is unsubstituted - (CH₂)₂(heterocyclyl). In certain embodiments, at least one R^{B} is 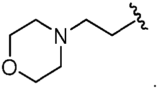 In certain embodiments, at least one R^{B} is substituted -(CH₂)₃(heterocyclyl). In certain embodiments, at least one R^{B} is unsubstituted -(CH₂)₃(heterocyclyl). In certain embodiments, at least one R^{B} is 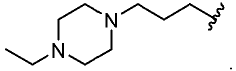 In certain embodiments, at least one R^{B} is substituted heteroaryl. In certain embodiments, at least one R^{B} is unsubstituted heteroaryl. In certain embodiments, at least one R^{B} is substituted pyridyl. In certain embodiments, at least one R^{B} is unsubstituted pyridyl. In certain embodiments, at least one R^{B} is substituted imidazole. In certain embodiments, at least one R^{B} is unsubstituted imidazole. In certain embodiments, at least one R^{B} is 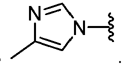 In certain embodiments, at least one R^{B} is

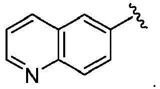

In certain embodiments, R^{B} is substituted or unsubstituted C₁₋₆ alkyl; and l is 1. In certain embodiments, R^{B} is substituted or unsubstituted C₁₋₆alkyl; l is 1; and R^{B} is *meta* to the point of attachment of the amide linker. In certain embodiments, R^{B} is substituted or unsubstituted C₁₋₆alkyl; l is 1; and R^{B} is *para* to the point of attachment of the amide linker. In certain embodiments, R^{B} is C₁₋₆alkyl substituted with one -CN group; and l is 1. In certain embodiments, R^{B} is C₁₋₆alkyl substituted with one -CN group; l is 1; and R^{B} is *meta* to the point of attachment of the amide linker. In certain embodiments, R^{B} is C₁₋₆alkyl substituted with one -CN group; l is 1; and R^{B} *is para* to the point of attachment of the amide linker. In certain embodiments, R^{B} is 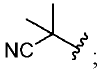 and l is 1. In certain embodiments, R^{B} is 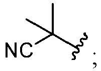 l is 1; and R^{B} is *meta* to the point of attachment of the amide linker. In certain embodiments, R^{B} is 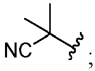 l is 1; and R^{B} is *para* to the point of attachment of the amide linker. In certain embodiments, R^{B} is substituted or unsubstituted -CH₂-(piperazinyl); and l is 1. In certain embodiments, R^{B} is substituted or unsubstituted -CH₂-(piperazinyl); l is 1; and R^{B} is *meta* to the point of attachment of the amide linker. In certain embodiments, R^{B} is substituted or unsubstituted -CH₂-(piperazinyl); l is 1; and R^{B} is *para* to the point of attachment of the amide linker. In certain embodiments, R^{B} is 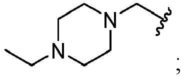 and l is 1. In certain embodiments, R^{B} is 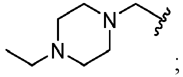 l is 1; and R^{B} is *meta* to the point of attachment of the amide linker. In certain embodiments, R^{B} is 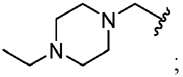 l is 1; and R^{B} is *para* to the point of attachment of the amide linker. In certain embodiments, R^{B} is substituted or unsubstituted imidazoyl; and l is 1. In certain embodiments, R^{B} is substituted or unsubstituted imidazoyl; l is 1; and R^{B} is *meta* to the point of attachment of the amide linker. In certain embodiments, R^{B} is substituted or unsubstituted imidazoyl; l is 1; and R^{B} is *para* to the point of attachment of the amide linker. In certain embodiments, R^{B} is 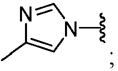 and l is 1. In certain embodiments, R^{B} is 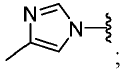 l is 1; and R^{B} is *meta* to the point of attachment of the amide linker. In certain embodiments, R^{B} is 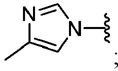 l is 1; and R^{B} is *para* to the point of attachment of the amide linker. In certain embodiments, R^{B} is substituted or unsubstituted piperazinyl; and l is 1. In certain embodiments, R^{B} is substituted or unsubstituted piperazinyl; l is 1; and R^{B} is *meta* to the point of attachment of the amide linker. In certain embodiments, R^{B} is substituted or unsubstituted piperazinyl; l is 1; and R^{B} is *para* to the point of attachment of the amide linker. In certain embodiments, R^{B} is 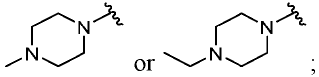 and l is 1. In certain embodiments, R^{B} is 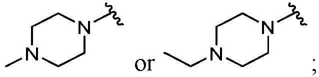 l is 1; and R^{B} is *meta* to the point of attachment of the amide linker. In certain embodiments, R^{B} is 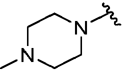 or 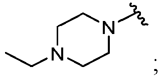 l is 1; and R^{B} *is para* to the point of attachment of the amide linker. In certain embodiments, R^{B} is substituted or unsubstituted morpholine; and l is 1. In certain embodiments, R^{B} is substituted or unsubstituted morpholine; l is 1; and R^{B} is *meta* to the point of attachment of the amide linker. In certain embodiments, R^{B} is substituted or unsubstituted morpholine; l is 1; and R^{B} is *para* to the point of attachment of the amide linker.

In certain embodiments, at least one R^{B} group is substituted or unsubstituted C₁₋₆ alkyl; and l is 2. In certain embodiments, at least one R^{B} group is substituted or unsubstituted C₁₋₆alkyl; l is 2; and at least one R^{B} is *meta* to the point of attachment of the amide linker. In certain embodiments, at least one R^{B} group is substituted or unsubstituted C₁₋₆alkyl; l is 2; and one R^{B} is *para* to the point of attachment of the amide linker. In certain embodiments, at least one R^{B} group is C₁₋₆alkyl substituted with one -CN group; and l is 2. In certain embodiments, at least one R^{B} group is C₁₋₆alkyl substituted with one -CN group; l is 2; and at least one R^{B} is *meta* to the point of attachment of the amide linker. In certain embodiments, at least one R^{B} group is C₁₋₆alkyl substituted with one -CN group; l is 2; and one R^{B} is *para* to the point of attachment of the amide linker. In certain embodiments, at least one R^{B} group is 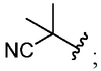 and l is 2. In certain embodiments, at least one R^{B} group is 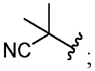 l is 2; and at least one R^{B} is *meta* to the point of attachment of the amide linker. In certain embodiments, at least one R^{B} group is 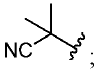 l is 2; and one R^{B} is *para* to the point of attachment of the amide linker. In certain embodiments, at least one R^{B} group is substituted or unsubstituted - CH₂-(piperazinyl); and l is 2. In certain embodiments, at least one R^{B} group is substituted or unsubstituted -CH₂-(piperazinyl); l is 2; and at least one R^{B} is *meta* to the point of attachment of the amide linker. In certain embodiments, at least one R^{B} group is substituted or unsubstituted -CH₂-(piperazinyl); l is 2; and one R^{B} is *para* to the point of attachment of the amide linker. In certain embodiments, at least one R^{B} group is 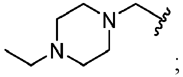 and l is 2. In certain embodiments, at least one R^{B} group is 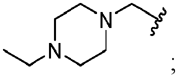 l is 2; and at least one R^{B} is *meta* to the point of attachment of the amide linker. In certain embodiments, at least one R^{B} group is 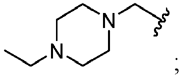 l is 2; and one R^{B} is *para* to the point of attachment of the amide linker In certain embodiments, at least one R^{B} group is substituted or unsubstituted imidazoyl; and l is 2. In certain embodiments, at least one R^{B} group is substituted or unsubstituted imidazoyl; l is 2; and at least one R^{B} is *meta* to the point of attachment of the amide linker. In certain embodiments, at least one R^{B} group is substituted or unsubstituted imidazoyl; l is 2; and one R^{B} is *para* to the point of attachment of the amide linker. In certain embodiments, at least one R^{B} group is 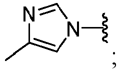 and l is 2. In certain embodiments, at least one R^{B} group is 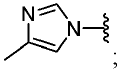 l is 2; and at least one R^{B} is *meta* to the point of attachment of the amide linker. In certain embodiments, at least one R^{B} group is 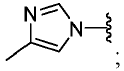 l is 2; and one R^{B} is *para* to the point of attachment of the amide linker. In certain embodiments, at least one R^{B} group is substituted or unsubstituted piperazinyl; and l is 2. In certain embodiments, at least one R^{B} group is substituted or unsubstituted piperazinyl; l is 2; and at least one R^{B} is *meta* to the point of attachment of the amide linker. In certain embodiments, at least one R^{B} group is substituted or unsubstituted piperazinyl; l is 2; and one R^{B} is *para* to the point of attachment of the amide linker. In certain embodiments, at least one R^{B} group is 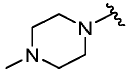 or 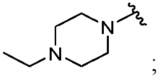 and l is 2. In certain embodiments, at least one R^{B} group is 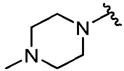 or 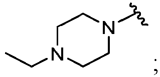 l is 2; and at least one R^{B} is *meta* to the point of attachment of the amide linker. In certain embodiments, at least one R^{B} group is 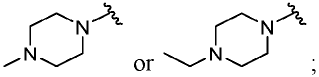 l is 2; and one R^{B} is *para* to the point of attachment of the amide linker. In certain embodiments, at least one R^{B} group is substituted or unsubstituted morpholine; and l is 2. In certain embodiments, at least one R^{B} group is substituted or unsubstituted morpholine; l is 2; and at least one R^{B} is *meta* to the point of attachment of the amide linker. In certain embodiments, at least one R^{B} group is substituted or unsubstituted morpholine; l is 2; and one R^{B} is *para* to the point of attachment of the amide linker. In certain embodiments, two R^{B} groups are substituted or unsubstituted morpholine; l is 2; and both R^{B} groups are *meta* to the point of attachment of the amide linker.

In compounds of Formula (I-a3), the pyridine ring is substituted with 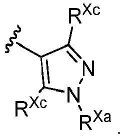 In certain embodiments,

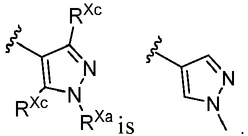

In compounds of Formula (I-a1), the pyridine ring is substituted with

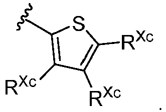

In compounds of Formula (I-a2), the pyridine ring is substituted with

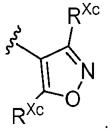

In compounds of Formula (I-a3), R^{Xa} is selected from the group consisting of substituted or unsubstituted C₁₋₆ alkyl.

In certain embodiments, R^{Xa} is substituted C₁₋₆ alkyl. In certain embodiments, R^{Xa} is substituted methyl. In certain embodiments, R^{Xa} is unsubstituted C₁₋₆ alkyl. In certain embodiments, R^{Xa} is methyl. In certain embodiments, R^{Xa} is ethyl. In certain embodiments, R^{Xa} is propyl. In certain embodiments, R^{Xa} is butyl.

In compounds of Formula (I-a1), (I-a2) or (I-a3), R^{Xc} is selected from the group consisting of hydrogen.

In the present invention, a compound of Formula (I-a1), (I-a2), or (I-a3): 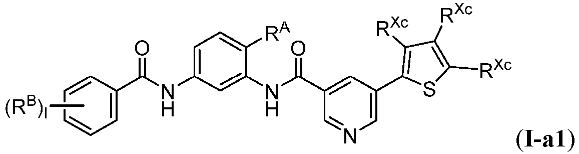 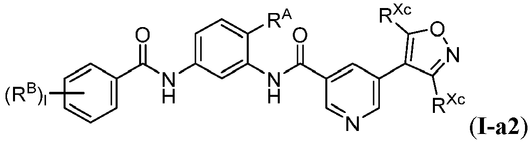 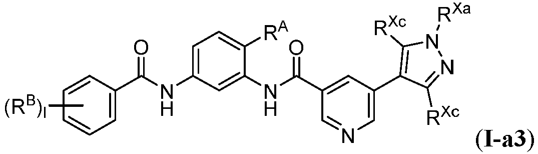 wherein R^{Xa}, R^{Xc}, R^{A}, R^{B}, and l are defined herein. In the present invention, R^{A} is substituted or unsubstituted C₁₋₆ alkyl. In certain embodiments, R^{A} is methyl. In certain embodiments, l is 1. In certain embodiments, l is 1; and R^{B} is *meta* to the point of attachment of the amide linker. In certain embodiments, l is 2. In certain embodiments, l is 2; and the two R^{B} groups are *meta* to the point of attachment of the amide linker. In certain embodiments, l is 2; one R^{B} group is *meta* to the point of attachment of the amide linker; and the second R^{B} group is *para* to the point of attachment of the amide linker. In certain embodiments, one R^{B} group is substituted or unsubstituted C₁₋₆ alkyl. In certain embodiments, one R^{B} group is C₁₋₆ alkyl substituted with one -CN group. In certain embodiments, one R^{B} group is 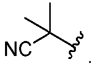 In certain embodiments, one R^{B} group is substituted or unsubstituted -CH₂-(piperazinyl). In certain embodiments, one R^{B} group is 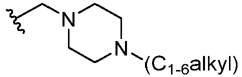 where the alkyl is optionally substituted. In certain embodiments, one R^{B} group is 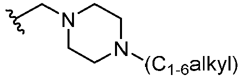 where the alkyl is unsubstituted. In certain embodiments, one R^{B} group is 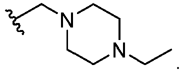 In certain embodiments, one R^{B} group is substituted or unsubstituted imidazoyl. In certain embodiments, one R^{B} group is 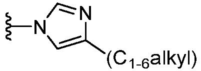 where the alkyl is optionally substituted. In certain embodiments, one R^{B} group is 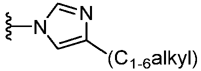 where the alkyl is unsubstituted. In certain embodiments, one R^{B} group is 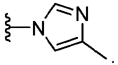 In certain embodiments, one R^{B} group is substituted or unsubstituted piperazinyl. In certain embodiments, one R^{B} group is 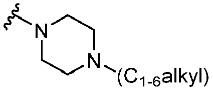 where there alkyl is optionally substituted. In certain embodiments, one R^{B} group is 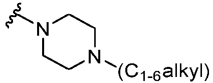 where there alkyl is unsubstituted. In certain embodiments, one R^{B} group is 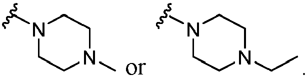 In certain embodiments, one R^{B} group is substituted or unsubstituted morpholine. In certain embodiments, two R^{B} groups are substituted or unsubstituted morpholine. In the present invention, R^{Xa} is substituted or unsubstituted C₁₋₆ alkyl. In certain embodiments, R^{Xa} is methyl or ethyl.

In certain embodiments, a compound of Formula (I-a1) (I-a2) or (I-a3): is a compound of Formula (I-a4)-(I-a12): 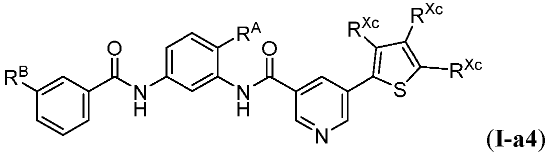 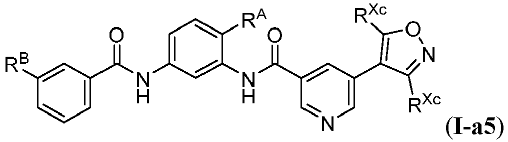 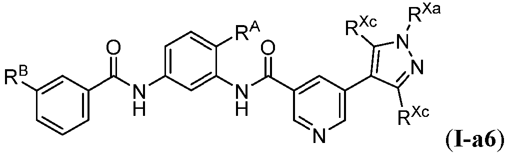 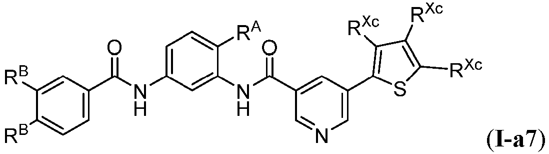 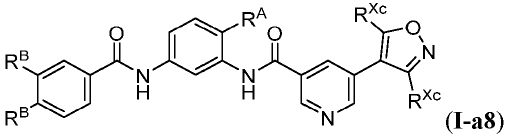 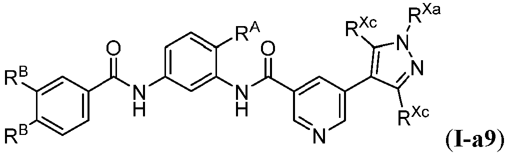 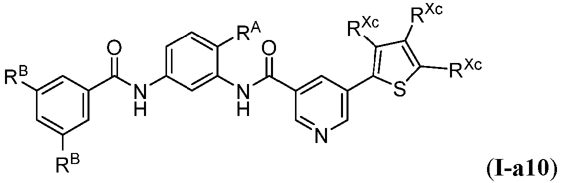 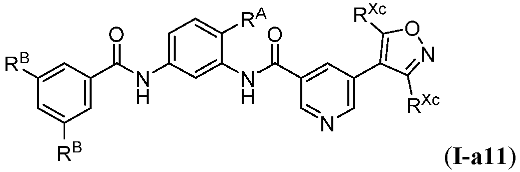 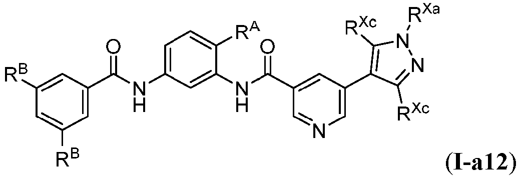 wherein R^{Xa}, R^{Xc}, R^{A}, and R^{B} are defined herein. In the present invention, R^{A} is substituted or unsubstituted C₁₋₆ alkyl. In certain embodiments, R^{A} is methyl. In certain embodiments, one R^{B} group is substituted or unsubstituted C₁₋₆ alkyl. In certain embodiments, one R^{B} group is C₁₋₆ alkyl substituted with one -CN group. In certain embodiments, one R^{B} group is 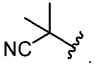 In certain embodiments, one R^{B} group is substituted or unsubstituted -CH₂-(piperazinyl). In certain embodiments, one R^{B} group is 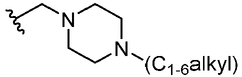 where the alkyl is optionally substituted. In certain embodiments, one R^{B} group is 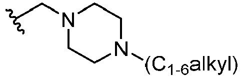 where the alkyl is unsubstituted. In certain embodiments, one R^{B} group is 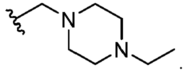 In certain embodiments, one R^{B} group is substituted or unsubstituted imidazoyl. In certain embodiments, one R^{B} group is 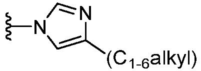 where the alkyl is optionally substituted. In certain embodiments, one R^{B} group is 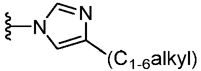 where the alkyl is unsubstituted. In certain embodiments, one R^{B} group is 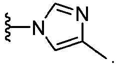 In certain embodiments, one R^{B} group is substituted or unsubstituted piperazinyl. In certain embodiments, one R^{B} group is 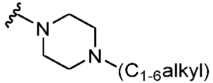 where there alkyl is optionally substituted. In certain embodiments, one R^{B} group is 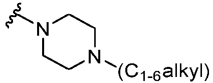 where there alkyl is unsubstituted. In certain embodiments, one R^{B} group is 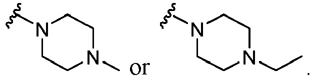 In certain embodiments, one R^{B} group is substituted or unsubstituted morpholine. In certain embodiments, two R^{B} groups are substituted or unsubstituted morpholine. In the present invention, all instances of R^{Xc} are hydrogen. In the present invention, R^{Xa} is substituted or unsubstituted C₁₋₆ alkyl. In certain embodiments, R^{Xa} is methyl or ethyl.

In certain embodiments, compounds of the present invention include those which:
- exhibit kinase inhibitory activity,
- exhibit the ability to inhibit transforming growth factor b-activated kinase-1 (TAK1), hemopoietic cell kinase (HCK) or both TAK1 and HCK,
- exhibit the ability to inhibit hematopoietic progenitor kinase 1 (HPK1, also known as mitogen-activated protein kinase kinase kinase kinase 1 or MAP4K1),
- exhibit the ability to inhibit Bruton's tyrosine kinase (BTK), v-src sarcoma (Schmidt-Ruppin A-2) viral oncogene homolog (SRC) family of kinases or both BTK and SRC,
- exhibit cytotoxic or growth inhibitory effect on WM cell lines maintained *in vitro* or in animal studies using a scientifically acceptable cancer cell xenograft model; and/or
- exhibit a therapeutic profile (e.g., optimum safety and curative effect) that is superior to existing chemotherapeutic agents.

As used herein "kinase" refers to a large class of enzymes which catalyze the transfer of the γ-phosphate from ATP to the hydroxyl group on the side chain of Ser/Thr or Tyr in proteins and peptides and are intimately involved in the control of various important cell functions, perhaps most notably: signal transduction, differentiation and proliferation. There are estimated to be about 2,000 distinct protein kinases in the human body and although each of these phosphorylates particular protein/peptide substrates, they all bind the same second substrate ATP in a highly conserved pocket. About 50% of the known oncogene products are protein tyrosine kinases PTKs and their kinase activity has been shown to lead to cell transformation.

In certain embodiments, the kinase to be inhibited is involved in the myeloid differentiation primary response gene (88) (MYD88) signaling pathway. For example, the kinase is Transforming growth factor b-activated kinase-1 (TAK1) TAK1 or Hemopoietic cell kinase (HCK). In certain embodiments, the compound of the invention inhibits TAK1, HCK, or both TAK1 and HCK.

Myeloid differentiation primary response gene (88) (MYD88) L265P is a widely expressed somatic mutation in WM patients that supports NF- NFκB signaling through stimulation of BTK, IRAK1/4, TAK1. MYD88 is an adaptor molecule for Toll-like receptors (TLR) with the exception of TLR-3 and interleukin-1 receptor (IL-1R) signaling. Following TLR or IL-1R stimulation, MYD88 is recruited to the activated receptor complex as a homodimer which then complexes with interleukin-1 receptor-associated kinase 4 (IRAK4) and activates IRAK1 and IRAK2. Tumor necrosis factor receptor associated factor 6 (TRAF6) is then activated by IRAK1 leading to NFκB activation *via* IκBα phosphorylation and TAK1 activation.

Transforming growth factor b-activated kinase-1 (TAK1; also known as MAP3K7) is a member of the serine/threonine protein kinase family. This kinase mediates the signaling transduction induced by TGF beta and morphogenetic protein (BMP), and controls a variety of cell functions including transcription regulation and apoptosis. TAK1 knockout is embryonic lethal to mice. Conditional knock-down of TAK1 in adult mice results in systemic inflammation, spenomegaly, degeneration in heart, kidneys and liver and increased proliferation and differentiation of myeloid progenitor cells. TAK1 is located downstream of Myd88, Bruton's tyrosine kinase (BTK), and interleukin-1 receptor-associated kinase (IRAK), and is being investigated for its role in innate immunity, inflammatory response, and Ras-dependent cancers.

Hemopoietic cell kinase (HCK) is a non-receptor tyrosine-protein kinase found in hematopoietic cells and is known to interact with Bruton's tyrosine kinase (BTK) upon activation by B cell receptors (Proc Natl Acad Sci USA. 1994 August 16; 91(17): 8152-8155). HCK transmits signals from cell surface receptors and plays an important role in the regulation of innate immune responses, including neutrophil, monocyte, macrophage and mast cell functions, phagocytosis, cell survival and proliferation, cell adhesion and migration. It acts downstream of receptors that bind the Fc region of immunoglobulins, such as FCGR1A and FCGR2A, but also CSF3R, PLAUR, the receptors for IFNG, IL2, IL6 and IL8, and integrins, such as ITGB1 and ITGB2. During the phagocytic process, it mediates mobilization of secretory lysosomes, degranulation, and activation of NADPH oxidase to bring about the respiratory burst. It also plays a role in the release of inflammatory molecules, promotes reorganization of the actin cytoskeleton and actin polymerization, and formation of podosomes and cell protrusions.

Hematopoietic progenitor kinase 1 (HPK1) is a hematopoietic cell-restricted member of the Ste20 serine/threonine kinase super family. HPK1 is also known as mitogen-activated protein kinase kinase kinase kinase 1 (MAP4K1). HPK1 is a tissue-specific upstream activator of the MEKK/JNK/SAPK signaling pathway. HPK1 diminishes T cell receptor (TCR) signaling activity and T cell proliferation by phosphorylating the adaptor protein SLP-76. Cytosolic HPK1 is recruited to the TCR complex, and its kinase activity is induced upon the engagement of the TCR. Overexpression of HPK1 suppresses TCR-induced activation of AP-1-dependent gene transcription in a kinase-dependent manner, suggesting that the kinase activity of HPK1 is required to inhibit the Erk MAPK pathway. This blockage of the Erk MAPK pathway is thought to be the inhibitory mechanism that negatively regulates TCR-induced IL-2 gene transcription *(*Immunol. Res. 2012, 54(1-3), 262-65). In certain embodiments, the compounds of Formula (I-a1), (I-a2) and (I-a3) inhibit HPK1.

In certain embodiments, the compounds of the invention are selective inhibitors of TAK1, HCK, or HPK1. The term "selective inhibitor" as used herein is understood to mean that in contrast to many kinase inhibitors of the prior art, the compounds do not act on a variety of kinases but act specifically on TAK1, HCK, or HPK1. In certain embodiments, the compounds of the invention inhibit one or more kinases in additon to TAK1, HCK, or HPK1 such as BTK or the SRC family of kinases. In certain embodiments of the invention, the specificity of the inhibitors is given by the IC₅₀ value. In some embodiments, the IC₅₀ value for a selective inhibitor is < 100 µM for TAK1, HCK, or HPK1, but >100 µM for other kinases.

The IC₅₀ value is defined as the concentration of inhibitor required to inhibit 50% of the kinase activity. In certain embodiments, the compounds of of the invention may exhibit IC₅₀ values < 100 µM. In certain other embodiments, the compounds exhibit IC₅₀ values < 50 µM. In certain other embodiments, the compounds exhibit IC₅₀ values < 40 µM. In certain other embodiments, the compounds exhibit IC₅₀ values < 30 µM. In certain other embodiments, the compounds exhibit IC₅₀ values < 20 µM. In certain other embodiments, the compounds exhibit IC₅₀ values < 10 µM. In certain other embodiments, the compounds exhibit IC₅₀ values < 7.5 µM. In certain embodiments, the compounds exhibit IC₅₀ values < 5 µM. In certain other embodiments, the compounds exhibit IC₅₀ values < 2.5 µM. In certain embodiments, the compounds exhibit IC₅₀ values < 1 µM. In certain embodiments, the compounds exhibit IC₅₀ values < 0.75 µM. In certain embodiments, the compounds exhibit IC₅₀ values < 0.5 µM. In certain embodiments, the compounds exhibit IC₅₀ values < 0.25 µM. In certain embodiments, the compounds exhibit IC₅₀ values < 0.1 µM. In certain other embodiments, the compounds exhibit IC₅₀ values < 75 nM. In certain other embodiments, the compounds exhibit IC₅₀ values < 50 nM. In certain other embodiments, the compounds exhibit IC₅₀ values < 25 nM. In certain other embodiments, the compounds exhibit IC₅₀ values < 10 nM. In other embodiments, the compounds exhibit IC₅₀ values < 7.5 nM. In other embodiments, the compounds exhibit IC₅₀ values < 5 nM.
In certain embodiments, the compounds Formula (I-a1), (I-a2) or (I-a3) inhibit HCK selectively. In some embodiments, this selective HCK inhibitor has an IC₅₀ value < 50 nM.

In certain embodiments, the compounds of Formula (I-a1), (I-a2) or (I-a3) inhibit both TAK1 and HCK. In certain embodiments, the compounds Formula (I-a1), (I-a2) or (I-a3) inhibit HPK1 selectively.

Also, provided are the compounds of the invention for use in treating B cell neoplasms, preferably wherein the compounds of the invention are administered in combination with inhibitors of Bruton's tyrosine kinase (BTK), interleukin-1 receptor-associated kinase 1 (IRAK1), interleukin-1 receptor-associated kinase 4 (IRAK4), bone marrow on X chromosome kinase (BMX), phosphoinositide 3-kinase (PI3K), transforming growth factor b-activated kinase-1 (TAK1), and/or a Src family kinase, even more preferably in combination with inhibitors of Bruton's tyrosine kinase (BTK), interleukin-1 receptor-associated kinase 1 (IRAK1), interleukin-1 receptor-associated kinase 4 (IRAK4), transforming growth factor b-activated kinase-1 (TAK1), or a Src family kinase. In certain embodiments, one or more compounds of the invention are used in combination with an inhibitor of the phosphoinositide 3-kinase delta isoform (PI3Kδ). In certain embodiments, combinations of 2, 3, 4, 5, 6,7, 8, 9, 10, or more of the agents described herein are used for treating WM. In certain embodiments, the agents described herein are used in combination with inhibitors of Bruton's tyrosine kinase (BTK), interleukin-1 receptor-associated kinase 1 (IRAK1), interleukin-1 receptor-associated kinase 4 (IRAK4), bone marrow on X chromosome kinase (BMX), phosphoinositide 3-kinase (PI3K), transforming growth factor b-activated kinase-1 (TAK1), and/or a Src family kinase, preferably in combination with inhibitors of Bruton's tyrosine kinase (BTK), interleukin-1 receptor-associated kinase 1 (IRAK1), interleukin-1 receptor-associated kinase 4 (IRAK4), transforming growth factor b-activated kinase-1 (TAK1), or a Src family kinase.

Bruton's tyrosine kinase (BTK) is a key signaling enzyme expressed in all hematopoietic cells types except T lymphocytes and natural killer cells. BTK plays an essential role in the B cell signaling pathway linking cell surface B cell receptor BCR stimulation to downstream intracellular responses. BTK is a key regulator of B cell development activation signaling and survival (Kurosaki, Curr. Op. Imm., 2000, 276-281; Schaeffer and Schwartzberg, Curr. Op. Imm., 2000, 282-288). In addition BTK plays a role in a number of other hematopoietic cell signaling pathways, e.g., Toll like receptor (TLR) and cytokine receptor-mediated TNF-α production in macrophages, IgE receptor (FcepsilonRI) signaling in mast cells, inhibition of Fas/APO-1 apoptotic signaling in B-lineage lymphoid cells, and collagen stimulated platelet aggregation. See *e.g.,* C.A. Jeffries, et al., J. Biol. Chem., 2003, 278, 26258-26264; N.J. Horwood, et al., J. Exp. Med., 2003, 197, 1603-1611; Iwaki et al., J. Biol. Chem., 2005, 280(48), 40261-40270; Vassilev et al., J. Biol. Chem., 1999, 274(3),1646-1656; and Quek et al., Curr. Biol., 1998, 8(20),1137-1140. Activated Btk interacts with MyD88 and TRIF, promoting the activation of MyD88-dependent and TRIF-dependent pathways *(*Nature Immunology, 2011, 12, 416-424).

BTK inhibitors are well-known in the art, and include, for example, ibrutinib and benzonaphthyridinones (see U.S. provisional patent application U.S.S.N. 61/716,273, filed October 19, 2012). Additional non-limiting examples of BTK inhibitors are disclosed in WO 1999/054286, WO 2013/010380, WO 2009/137596, WO 2011/029043, WO 2010/056875, WO 2000/056737, and WO 2013/067277.

IRAK1 and 4 are serine/threonine-protein kinases that play a critical role in initiating innate immune response against foreign pathogens. They are involved in Toll-like receptor (TLR) and IL-1R signaling pathways, and are rapidly recruited by MYD88 to the receptor-signaling complex upon TLR activation. Association with MYD88 leads to IRAK1 phosphorylation by IRAK4 and subsequent autophosphorylation and kinase activation of IRAK1 *(*Immunity, 1997, 7(6), 837-47). IRAK4-/- mice have abolished cellular responses to various IL-1 and TLR ligands and are severely impaired in their response to viral and bacterial challenges. IRAK1-/- mice show a similar but partial response.

IRAK1 and IRAK4 inhibitors are well-known in the art, and include, for example, those disclosed in WO 2003/030902, WO 2012/007375, G.M. Buckely et al., Biorg. Med. Chem. Lett., 2008, 18, 3211-3214, and G.M. Buckely et al., Biorg. Med. Chem. Lett., 2008, 18, 3656-3660, WO2013/074986, and U.S. provisional patent application, U.S.S.N. 61/727,640, filed November 16, 2012.

In certain embodiments, the IRAK4 inhibitor is 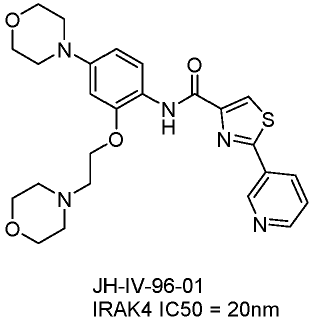 or its analogs.

"Bone Marrow on X chromosome" kinase (BMX, also termed ETK) is a non-receptor tyrosine kinase and is activated downstream of phosphatidylinositol-3 kinase (PI-3K) and v-src sarcoma (Schmidt-Ruppin A-2) viral oncogene homolog (SRC), but its substrates are unknown. Positional scanning peptide library screening revealed a marked preference for a priming phosphotyrosine (pY) in the -1 position. Potential substrates include multiple tyrosine kinases with kinase domain pYpY sites required for full activity. BMX has been found to phosphorylate residue Y577 of focal adhesion kinase (FAK) subsequent to Y576 phosphorylation by SRC. In addition, BMX loss by RNA interference and mouse embryonic fibroblasts (MEFs) from *Bmx* negative *(Bmx⁻)* mice displayed impaired FAK signaling. Insulin receptor (IR) phosphorylation similarly was decreased by BMX loss, as was hepatic IR phosphorylation in *Bmx⁻* mice. However, glucose tolerance was increased, reflecting a marked compensatory decrease in the activity of the AKT phosphatase PHLPP. These findings reveal a mechanism through which BMX functions as a central regulator of multiple kinase pathways.

BMX inhibitors are well-known in the art, and include, for example, those disclosed in U.S.S.N. 61/716,273 and 61/717,345. In certain embodiments, the BMX inhibitor is of formula: 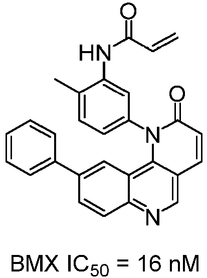 or an analog thereof.

Phosphatidylinositol 3-kinases (PI3-kinases or PI3Ks) are a family of enzymes involved in cellular functions such as cell growth, proliferation, differentiation, motility, survival and intracellular trafficking, which in turn are involved in cancer. PI3Ks are a family of related intracellular signal transducer enzymes capable of phosphorylating the 3 position hydroxyl group of the inositol ring of phosphatidylinositol (Ptdlns). Phosphatidylinositol 3-kinase is composed of an 85 kDa regulatory subunit and a 110 kDa catalytic subunit. The protein encoded by PI3KCA gene represents the catalytic subunit, which uses ATP to phosphorylate phosphatidylinositols (Ptdlns), Ptdins4P and Ptdins(4,5)P2. Of particular interest is the PI3K delta isoform, which is expressed in white blood cells and is mainly involved in the signaling, development, and survival of B cells.

PI3K inhibitors are well-known in the art, and include, for example, those disclosed in International PCT Publications WO 2013/088404, WO 2012/068096, and WO 2013/052699.

In certain embodiments, the PI3K inhibitor is of formula: 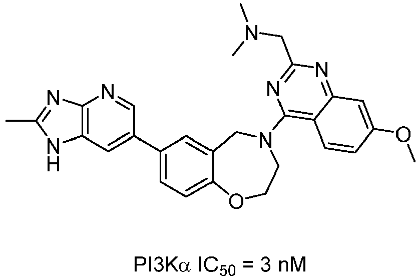 or an analog thereof.

Compounds of the invention may be combined with other kinase inhibitors to treat WM or other B cell neoplasms. In certain embodiments, a compound of the invention for use in treating WM or other B cell neoplasm, wherein the compound of the invention is administered with an inhibitor of Bruton's tyrosine kinase (BTK). In certain embodiments, a compound of the invention for use in treating WM or other B cell neoplasm, wherein the compound of the invention is administered with an inhibitor of interleukin-1 receptor-associated kinase 1 (IRAK1). In certain embodiments, a compound of the invention for use in treating WM or other B cell neoplasm, wherein the compound of the invention is administered with an inhibitor of phosphoinositide 3-kinase (PI3K). In certain embodiments, a compound of the invention for use in treating WM or other B cell neoplasm, wherein the compound of the invention is administered with an inhibitor of the phosphoinositide 3-kinase delta isoform (PI3Kδ). In certain embodiments, a compound of the invention for use in treating WM or other B cell neoplasm, wherein the compound of the invention is administered with two of any inhibitors of BTK, IRAK1, or PI3K. In certain embodiments, a compound of the invention for use in treating WM or other B cell neoplasm wherein the compound of the invention is administered with more than two of any inhibitors of BTK, IRAK1, or PI3K.

The BTK inhibitors, the IRAK1 inhibitors, the IRAK4 inhibitors, and/or the PI3K inhibitors can be administered to the subject simultaneously or sequentially.

A "subject" or "patient" to which administration is contemplated includes, any animal. In some embodiments, a subject includes but is not limited to, humans, commercially relevant mammals such as cattle, pigs, horses, sheep, goats, cats, and/or dogs), birds *(e.g.,* commercially relevant birds such as chickens, ducks, geese, and/or turkeys) and experimental animals (e.g., mice, rats, non-human primates). A subject in need of treatment is a subject identified as having a B cell neoplasm, *i.e.,* the subject has been diagnosed by a physician *(e.g.,* using methods well known in the art) as having a B cell neoplasm. In certain embodiments, the subject in need of treatment is a subject suspected of having or developing a B cell neoplasm, such as a subject presenting one or more symptoms indicative of a B cell neoplasm. The term "subject in need of treatment" further includes people who once had a B cell neoplasm but whose signs and/or symptoms have been ameliorated *(ie.,* their cancer is in remission). The one or more symptoms or clinical features of B cell neoplasms include, but are not limited to, asymptomatic localized or generalized peripheral lymphadenopathy, plasmacytic difference, bone marrow involvement, autoimmune thrombocytopenia, peripheral blood villous lymphocytes, end organ damage (hypercalcemia, renal insufficiency, bone lesions), recurrent infections, elevated creatine, hyperuricemia, and hypoalbunemia.

In certain embodiments, the subject is diagnosed as having Waldenström's macroglobulinemia (WM). The subject may present one or more signs, symptoms, or clinical features of WM including anemia, hyper-viscosity, neuropathy, coagulopathies, splenomegaly, hepatomegaly, adenopathy, and an IgM serum paraprotein. In certain embodiments, the subject is diagnosed as having WM on the basis that the subject has a mutation at position 38182641 of chromosome 3p22.2. In some embodiments, the mutation results in a single nucleotide change from T to C in the MYD88 gene. In some embodiments, the mutation results in an amino acid change from leucine to proline at position 265 in the MYD88 gene. The mutation may be detected in a biological sample obtained from the subject using any suitable method known in the art, including but not limited to, direct sequencing of nucleic acid molecules, HPLC analysis, DNA chip technologies, and mass spectroscopy. Non-limiting examples of the biological sample include bone marrow, lymph node, spleen, or blood.

The terms "administer," "administering," or "administration," as used he rein refers to implanting, absorbing, ingesting, injecting, or inhaling an inventive compound, or a pharmaceutical composition thereof.

As used herein, the terms "treatment," "treat," and "treating" refer to reversing, alleviating, delaying the onset of, or inhibiting the progress of a B cell neoplasm. In certain embodiments, treatment may be administered after one or more signs or symptoms have developed or have been observed. In other embodiments, treatment may be administered in the absence of signs or symptoms of the B cell neoplasm. For example, treatment may be administered to a susceptible individual prior to the onset of symptoms (e.g., in light of a history of symptoms and/or in light of genetic or other susceptibility factors). Treatment may also be continued after symptoms have resolved, for example, to delay or prevent recurrence.

An "effective amount" of compounds of the invention refers to an amount sufficient to elicit the desired biological response, *i.e.,* treating the B cell neoplasm. As will be appreciated by those of ordinary skill in this art, the effective amount of compounds of the invention may vary depending on such factors as the desired biological endpoint, the pharmacokinetics of the compound, the condition being treated, the mode of administration, and the age and health of the subject. An effective amount includes, but is not limited to, that amount necessary to slow, reduce, inhibit, ameliorate or reverse one or more signs and/or symptoms associated with a B cell neoplasm. In the treatment of Waldenström's macroglobulinemia, this may refer to a reduction in the levels of IgM serum paraprotein, reduction in anemia, reduction in hyper-viscosity, reduction in neuropathy, reduction in coagulopathies, reduction in splenomegaly, reduction in hepatomegaly, and reduction in adenopathy.

An effective amount of a compound may vary from about 0.001 mg/kg to about 1000 mg/kg in one or more dose administrations, for one or several days (depending on the mode of administration). In certain embodiments, the effective amount varies from about 0.001 mg/kg to about 1000 mg/kg, from about 0.01 mg/kg to about 750 mg/kg, from about 0.1 mg/kg to about 500 mg/kg, from about 1.0 mg/kg to about 250 mg/kg, from about 1.0 mg/kg to about 100 mg/kg, and from about 10.0 mg/kg to about 150 mg/kg.

One or more additional pharmaceutical agents, such as anti-cancer agents *(e.g.,* chemotherapeutics), anti-inflammatory agents, steroids, immunosuppressants, radiation therapy, or other agents, can be used in combination with the compounds of the invention in the treatment of a B cell neoplasm. The one or more additional pharmaceutical agents can be administered to the subject simultaneously or sequentially.

Exemplary chemotherapeutic agents include alkylating agents such as nitrogen mustards, ethylenimines, methylmelamines, alkyl sulfonates, nitrosuoureas, and triazenes; antimetabolites such as folic acid analogs, pyrimidine analogs, in particular fluorouracil and cytosine arabinoside, and purine analogs; natural products such as vinca alkaloids epipodophyllotoxins, antibiotics, enzymes, and biological response modifiers; and miscellaneous products such as platinum coordination complexes, anthracenedione, substituted urea such as hydroxyurea, methyl hydrazine derivatives, and adrenocorticoid suppressant.

Exemplary chemotherapeutic agents also include anthracycline antibiotics, actinomycin D, plicamycin, puromycin, gramicidin D, paclitaxel, colchicine, cytochalasin B, emetine, maytansine, amsacrine, cisplatin, carboplatin, mitomycin, altretamine, cyclophosphamide, lomustine, and carmustine.

In yet another aspect, the present invention provides pharmaceutical compositions comprising an effective amount of a compound of the invention, and pharmaceutically acceptable salts, solvates, hydrates, polymorphs, co-crystals, tautomers, stereoisomers, and prodrugs, and optionally a pharmaceutically acceptable excipient, for use in the treatment of a B cell neoplasm. In certain embodiments, provided by the invention are the compounds of the invention, and pharmaceutically acceptable salts and compositions thereof, for use in the treatment of a B cell neoplasm. In certain embodiments, the effective amount is a therapeutically effective amount. In certain embodiments, the therapeutically effective amount is an amount useful for the treatment and/or prevention of a B cell neoplasm. In certain embodiments, the B cell neoplasm is, but is not limited to, Hodgkin's lymphomas and most non-Hodgkins lymphomas, such as, diffuse large B cell lymphoma, Follicular lymphoma, Mucosa-Associated Lymphatic Tissue lymphoma (MALT), small cell lymphocytic lymphoma (overlaps with Chronic lymphocytic leukemia), Mantle cell lymphoma (MCL), Burkitt lymphoma, Mediastinal large B cell lymphoma, Waldenström's macroglobulinemia, Nodal marginal zone B cell lymphoma (NMZL), Splenic marginal zone lymphoma (SMZL), Intravascular large B-cell lymphoma, Primary effusion lymphoma and Lymphomatoid granulomatosis. An effective amount of a compound may vary from about 0.001 mg/kg to about 1000 mg/kg in one or more dose administrations, for one or several days (depending on the mode of administration). In certain embodiments, the effective amount varies from about 0.001 mg/kg to about 1000 mg/kg, from about 0.01 mg/kg to about 750 mg/kg, from about 0.1 mg/kg to about 500 mg/kg, from about 1.0 mg/kg to about 250 mg/kg, and from about 10.0 mg/kg to about 150 mg/kg.

Pharmaceutical compositions described herein can be prepared by any method known in the art of pharmacology. In general, such preparatory methods include the steps of bringing a compound of the invention (the "active ingredient") into association with a carrier or excipient, and/or one or more other accessory ingredients, and then, if necessary and/or desirable, shaping and/or packaging the product into a desired single- or multi-dose unit.

Pharmaceutical compositions can be prepared, packaged, and/or sold in bulk, as a single unit dose, and/or as a plurality of single unit doses. As used herein, a "unit dose" is a discrete amount of the pharmaceutical composition comprising a predetermined amount of the active ingredient. The amount of the active ingredient is generally equal to the dosage of the active ingredient which would be administered to a subject and/or a convenient fraction of such a dosage, such as, for example, one-half or one-third of such a dosage.

The pharmaceutical preparations of the present invention may include or be diluted into a pharmaceutically acceptable carrier. The term "pharmaceutically acceptable carrier" as used herein means one or more compatible fillers, diluents or other such substances, which are suitable for administration to a human or other mammal, such as a dog, cat, rat, mouse, or horse. The term "carrier" denotes an organic or inorganic ingredient, natural or synthetic, with which the active ingredient is combined to facilitate the application. The carriers are capable of being commingled with the preparations of the present invention, and with each other, in a manner such that there is no interaction which would substantially impair the desired pharmaceutical efficacy or stability. Carriers suitable for oral, subcutaneous, intravenous, intramuscular, *etc.* formulations can be found in Remington's Pharmaceutical Sciences, Mack Publishing Company, Easton, Pa.

The compounds and compositions provided herein can be administered by any route, including enteral *(e.g.,* oral), parenteral, intravenous, intramuscular, intra-arterial, intramedullary, intrathecal, subcutaneous, intraventricular, transdermal, interdermal, rectal, intravaginal, intraperitoneal, topical (as by powders, ointments, creams, and/or drops), mucosal, nasal, bucal, sublingual; by intratracheal instillation, bronchial instillation, and/or inhalation; and/or as an oral spray, nasal spray, and/or aerosol. Specifically contemplated routes are oral administration, intravenous administration (e.g., systemic intravenous injection), regional administration *via* blood and/or lymph supply, and/or direct administration to an affected site. In general, the most appropriate route of administration will depend upon a variety of factors including the nature of the agent *(e.g.,* its stability in the environment of the gastrointestinal tract), and/or the condition of the subject (e.g., whether the subject is able to tolerate oral administration).

The exact amount of a compound required to achieve an effective amount will vary from subject to subject, depending, for example, on species, age, and general condition of a subject, severity of the side effects or disorder, identity of the particular compound, mode of administration. The desired dosage can be delivered three times a day, two times a day, once a day, every other day, every third day, every week, every two weeks, every three weeks, or every four weeks. In certain embodiments, the desired dosage can be delivered using multiple administrations (e.g., two, three, four, five, six, seven, eight, nine, ten, eleven, twelve, thirteen, fourteen, or more administrations).

An effective amount may be included in a single dose *(e.g.,* single oral dose) or multiple doses (e.g., multiple oral doses). In certain embodiments, when multiple doses are administered to a subject or applied to a tissue or cell, any two doses of the multiple doses include different or substantially the same amounts of a compound described herein. In certain embodiments, when multiple doses are administered to a subject or applied to a tissue or cell, the frequency of administering the multiple doses to the subject or applying the multiple doses to the tissue or cell is three doses a day, two doses a day, one dose a day, one dose every other day, one dose every third day, one dose every week, one dose every two weeks, one dose every three weeks, or one dose every four weeks. In certain embodiments, the frequency of administering the multiple doses to the subject or applying the multiple doses to the tissue or cell is one dose per day. In certain embodiments, the frequency of administering the multiple doses to the subject or applying the multiple doses to the tissue or cell is two doses per day. In certain embodiments, the frequency of administering the multiple doses to the subject or applying the multiple doses to the tissue or cell is three doses per day. In certain embodiments, when multiple doses are administered to a subject or applied to a tissue or cell, the duration between the first dose and last dose of the multiple doses is one day, two days, four days, one week, two weeks, three weeks, one month, two months, three months, four months, six months, nine months, one year, two years, three years, four years, five years, seven years, ten years, fifteen years, twenty years, or the lifetime of the subject, tissue, or cell. In certain embodiments, the duration between the first dose and last dose of the multiple doses is three months, six months, or one year. In certain embodiments, the duration between the first dose and last dose of the multiple doses is the lifetime of the subject, tissue, or cell. In certain embodiments, a dose *(e.g.,* a single dose, or any dose of multiple doses) described herein includes independently between 0.1 µg and 1 µg, between 0.001 mg and 0.01 mg, between 0.01 mg and 0.1 mg, between 0.1 mg and 1 mg, between 1 mg and 3 mg, between 3 mg and 10 mg, between 10 mg and 30 mg, between 30 mg and 100 mg, between 100 mg and 300 mg, between 300 mg and 1,000 mg, or between 1 g and 10 g, inclusive, of a compound described herein. In certain embodiments, a dose described herein includes independently between 1 mg and 3 mg, inclusive, of a compound described herein. In certain embodiments, a dose described herein includes independently between 3 mg and 10 mg, inclusive, of a compound described herein. In certain embodiments, a dose described herein includes independently between 10 mg and 30 mg, inclusive, of a compound described herein. In certain embodiments, a dose described herein includes independently between 30 mg and 100 mg, inclusive, of a compound described herein.

In certain embodiments, an effective amount of a compound for administration one or more times a day to a 70 kg adult human may comprise about 0.0001 mg to about 3000 mg, about 0.0001 mg to about 2000 mg, about 0.0001 mg to about 1000 mg, about 0.001 mg to about 1000 mg, about 0.01 mg to about 1000 mg, about 0.1 mg to about 1000 mg, about 1 mg to about 1000 mg, about 1 mg to about 100 mg, about 10 mg to about 1000 mg, or about 100 mg to about 1000 mg, of a compound per unit dosage form.

In certain embodiments, the compound of the invention is administered at dosage levels sufficient to deliver from about 0.001 mg/kg to about 100 mg/kg, from about 0.01 mg/kg to about 50 mg/kg, preferably from about 0.1 mg/kg to about 40 mg/kg, preferably from about 0.5 mg/kg to about 30 mg/kg, from about 0.01 mg/kg to about 10 mg/kg, from about 0.1 mg/kg to about 10 mg/kg, and more preferably from about 1 mg/kg to about 25 mg/kg, of subject body weight per day, one or more times a day, to obtain the desired therapeutic effect.

It will be appreciated that dose ranges as described herein provide guidance for the administration of provided pharmaceutical compositions to an adult. The amount to be administered to, for example, a child or an adolescent can be determined by a medical practitioner or person skilled in the art and can be lower or the same as that administered to an adult.

The present invention is further illustrated by the following Example, which in no way should be construed as further limiting.

### Examples

In order that the invention described herein may be more fully understood, the following examples are set forth. The synthetic and biological examples described in this application are offered to illustrate the compounds, pharmaceutical compositions, and methods provided herein and are not to be construed in any way as limiting their scope.

### Example 1. Preparation of the Compounds

### Preparation of I-1

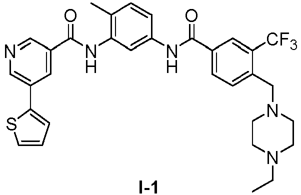

*N*-(5-(4-((4-ethylpiperazin-1-yl)methyl)-3-(trifluoromethyl)benzamido)-2-methylphenyl)-5-(thiophen-2-yl)nicotinamide (1-1): To a solution of 5-(thiophen-2-yl)nicotinic acid (205 mg, 1.0 mmol), DMAP (147 mg, 1.2 mmol), HATU (456 mg, 1.2 mmol) and *i*Pr₂NEt (440 uL, 2.5 mmol) in CH₂Cl₂ (5 mL) was added N-(3-amino-4-methylphenyl)-4-((4-ethylpiperazin-1-yl)methyl)-3-(trifluoromethyl)benzamide (420 mg, 1.0 mmol) and the resulting mixture was stirred at room temperature for 24 hours. The solution was filtered to remove solids, concentrated and purified by reverse phase HPLC to afford 485 mg (80%) of title compound as a white solid.

### Reference compound - Preparation of I-3

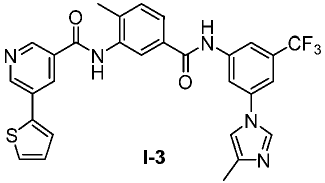

N-(2-methyl-5-((3-(4-methyl-1H-imidazol-1-yl)-5-(trifluoromethyl)phenyl)carbamoyl)phenyl)-5-(thiophen-2-yl)nicotinamide (I-3): To a solution of 5-(thiophen-2-yl)nicotinic acid (205 mg, 1.0 mmol), DMAP (147 mg, 1.2 mmol), HATU (456 mg, 1.2 mmol) and *i*Pr₂NEt (440 uL, 2.5 mmol) in CH₂Cl₂ (5 mL) was added 3-amino-4-methyl-N-(3-(4-methyl-1H-imidazol-1-yl)-5-(trifluoromethyl)phenyl)benzamide (375 mg, 1.0 mmol) and the resulting mixture was stirred at room temperature for 24 hours. The solution was filtered to remove solids, concentrated and purified by reverse phase HPLC to afford 425 mg (76%) of title compound as a white solid.

Reference compounds I-2, I-4, I-5, I-6 and I-7 were prepared similarly to I-3.

Characterization data for all final compounds is in the table below.

| **ID #** | **Structure** | **Name** | **¹H NMR and or MS (m/z)** |
|---|---|---|---|
| **I-1** | 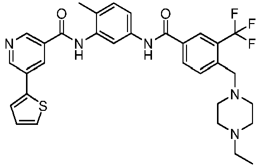 | N-(5-(4-((4-ethylpiperazin-1-yl)methyl)-3-(trifluoromethyl)benzami do)-2-methylphenyl)-5-(thiophen-2-yl)nicotinamide | ¹H NMR (600 MHz, TFA salt, DMSO) δ 10.57 (s, 1H), 10.33 (s, 1H), 9.50 (br, 1H), 9.17 (s, 1H), 9.10 (s, 1H), 8.59 (s, 1H), 8.37 (s, 1H), 8.32 (d, *J =* 8.4 Hz, 1H), 7.99 (d, *J =* 8.4 Hz, 1H), 7.94 (s, 1H), 7.84 (d, *J =* 3.6 Hz, 1H), 7.79 (d, *J* = 4.8, 1H), 7.68 (d, *J =* 3.6 Hz, 1H), 7.36 (d, *J =* 8.4 Hz, 1H), 7.31 (dd, *J =* 5.4, 3.6 Hz, 1H), 3.80 (s, 2H), 3.38 (m, 2H), 3.14 (q, *J* = 7.2 Hz, 1H), 3.01 (m, 2H), 2.94 (m, 2H), 2.45 (m, 2H), 2.25 (s, 3H), 1.22 (t, *J* = 7.2 Hz, 3H). MS (ESI) *m*/*z* 608 (M+H)⁺. |
| **I-2*** | 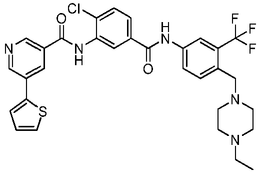 | N-(2-chloro-5-((4-((4-ethylpiperazin-1-yl)methyl)-3-(trifluoromethyl)phenyl) carbamoyl)phenyl)-5-(thiophen-2-yl)nicotinamide | ¹H NMR (600 MHz, TFA salt, DMSO) δ 10.65 (s, 1H), 10.64 (s, 1H), 9.54 (br, 1H), 9.13 (s,1H), 9.04 (s, 1H), 8.54 (s, 1H), 8.22 (s, 1H), 8. 21 (s, 1H), 8.10 (d, *J* = 8.4 Hz, 1H), 7.95 (d, J = 8.4 Hz, 1H), 7.79 (d, *J =* 8.4 Hz, 1H), 7.78 (d, *J =* 3.6 Hz, 1H), 7.73 *(d, J=* 4.8, 1H), 7.72 (d, *J =* 8.4 Hz, 1H), 7.24 (dd, *J =* 4.8, 3.6 Hz, 1H), 3.68 (s, 2H), 3.44 (m, 2H), 3.12 (m, 2H), 2.98 (m, 2H), 2.92 (m, 2H), 2.41 (m, 2H), 1.20 (t, *J =* 7.2 Hz, 3H). MS (ESI) *mlz* 628 (M+H)⁺. |
| **I-3*** | 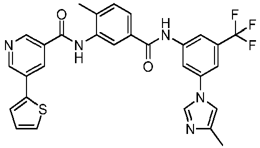 | N-(2-methyl-5-((3-(4-methyl-1H-imidazol-1-yl)-5-(trifluoromethyl)phenyl) carbamoyl)phenyl)-5-(thiophen-2-yl)nicotinamide | ¹H NMR (600 MHz, TFA salt, DMSO) δ 10.82 (s, 1H), 10.38 (s, 1H), 9.45 (br, 1H), 9.12 (s,1H), 9.05 (s, 1H), 8.55 (s, 1H), 8.52 (s, 1H), 8.23 (s, 1H), 8. 07 (s, 1H), 7.98 (s, 1H), 7.88 (d, *J =* 8.4 Hz, 1H), 7.77 (d, *J =* 3.6 Hz, 1H), 7.72 (d, *J =* 4.8 Hz, 1H), 7.52 (d, *J =* 8.4, 1H), 7.24 (dd, *J =* 4.8, 3.6 Hz, 1H), 2.36 (s, 3H), 2.33 (s, 3H). MS (ESI) *m*/*z* 562 (M+H)⁺. |
| **I-4*** | 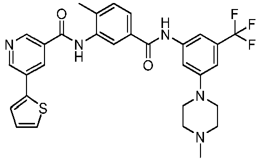 | N-(2-methyl-5-((3-(4-methylpiperazin-1-yl)-5-(trifluoromethyl)phenyl) carbamoyl)phenyl)-5-(thiophen-2-yl)nicotinamide | ¹H NMR (600 MHz, TFA salt, DMSO) δ 10.38 (s, 1H), 10.37 (s, 1H), 9.87 (br, 1H), 9.11 (s,1H), 9.04 (s, 1H), 8.53 (s, 1H), 8.02 (s, 1H), 7.84 (d, *J =* 7.8 Hz, 1H), 7.77 (d, *J =* 3.6 Hz, 1H), 7.70-7.75 (m, 3H), 7.48 (d, *J =* 8.4 Hz, 1H), 7.24 (dd, *J =* 4.8, 3.6 Hz, 1H), 7.06 (s, 1H), 3.91 (m, 2H), 3.54 (m, 2H), 3.16 (m, 2H), 3.05 (m, 2H), 2.86 (s, 3H), 2.35 (s, 3H). MS (ESI) *m*/*z* 580 (M+H)⁺. |
| **I-5*** | 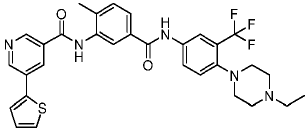 | N-(5-((4-(4-ethylpiperazin-1-yl)-3-(trifluoromethyl)phenyl) carbamoyl)-2-methylphenyl)-5-(thiophen-2-yl)nicotinamide | ¹H NMR (600 MHz, TFA salt, DMSO) δ 10.43 (s, 1H), 10.32 (s, 1H), 9.43 (br, 1H), 9.06 (s,1H), 8.99 (s, 1H), 8.47 (s, 1H), 8.13 (s, 1H), 8.08 (d, *J =* 8.4 Hz, 1H), 7.97 (s, 1H), 7.79 (d, *J =* 7.8 Hz, 1H), 7.72 (d, *J =* 4.8 Hz, 1H), 7.67 (d, *J =* 4.8 Hz, 1H), 7.52 (d, *J =* 8.4 Hz, 110), 7.52 (d, *J =* 7.8 Hz, 1H), 7.18 (dd, *J =* 4.8, 4.8 Hz, 1H), 3.51 (m, 4H), 3.18 (m, 2H), 3.00 (m, 4H), 2.29 (s, 3H), 1.18 (t, *J =* 7.2 Hz, 3H). MS (ESI) *m*/*z* 594 (M+H)⁺. |
| **I-6*** | 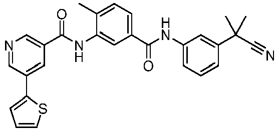 | N-(5-((3-(2-cyanopropan-2-yl)phenyl)carbamoyl)-2-methylphenyl)-5-(thiophen-2-yl)nicotinamide | ¹H NMR (600 MHz, TFA salt, DMSO) δ 10.32 (s, 1H), 10.28 (s, 1H), 9.06 (s,1H), 9.00 (s, 1H), 8.49 (s, 1H), 7.96 (s, 1H), 7.89 (s, 1H), 7.81 (s, 1H), 7.80 (d, *J =* 8.4 Hz, 1H), 7.72 (d, *J =* 3.6 Hz, 1H), 7.67 (d, *J =* 4.8 Hz, 1H), 7.42 (d, *J =* 8.4, 1H), 7.18 (m, 2H), 2.29 (s, 3H), 1.63 (s, 6H). MS (ESI) *m*/*z* 481 (M+H)⁺. |
| **I-7*** | 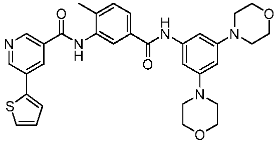 | N-(5-((3,5-dimorpholinophenyl)car bamoyl)-2-methylphenyl)-5-(thiophen-2-yl)nicotinamide | ¹H NMR (600 MHz, TFA salt, DMSO) δ 10.30 (s, 1H), 9.89 (s, 1H), 9.06 (s,1H), 8.99 (s, 1H), 8.48 (s, 1H), 7.93 (s, 1H), 7.75 (d, *J =* 7.8 Hz, 1H), 7.72 (d, *J =* 3.6 Hz, 1H), 7.64 (d, *J =* 4.8 Hz, 1H), 7.39 (d, *J =* 8.4 Hz, 1H), 7.18 (dd, *J =* 4.8, 3.6 Hz, 1H), 6.96 (s, 2H), 6.26 (s, 1H), 3.67 (m, 8H), 3.04 (m, 8H), 2.28 (s, 3H). MS (ESI) *m*/*z* 584 (M+H)⁺. |

| | | | |
|---|---|---|---|
| *reference compounds | | | |

### Example 2. Biological assays of the Compounds

### In vitro activity assays

The *in vitro* activity of the compounds described herein in inhibiting TAK1, HCK, and other kinases were obtained using an Invitrogen Select Screening assay as known in the art. The IC₅₀ values determined from this assay are shown below.

### Cell proliferation analysis

CellTiter-Glo^{®} Luminescent cell viability assay (Promega) was used to assess cell survival following treatment with the compounds described. Cells were seeded into 384 well plates with the EL406 Combination Washer Dispenser (BioTek Instruments, Inc.), and the compounds were injected into the cells culture media with the JANUS Automated Workstation (PerkinElmer Inc.). Cells were treated with a series diluted inhibitors (20~0.04 µM) for 72 hours at 37 °C. Luminescent measurement is performed using the 2104 Envision^{®} Multilabel Reader (PerkinElmer Inc.).

### Apoptosis analysis for primary patient bone marrow tumor cells

WM cells were treated with and without the compounds described herein. Cells were incubated at 37 °C with 0.01~4 uM of the compounds described herein. Apoptosis analysis was performed using Annexin V/Propidium iodide staining with the Apoptosis Detection Kit I (BD Pharmingen). 1×106/well cells were treated in 24 well plates for ~24 hours with inhibitors or corresponding controls. A minimum of 10,000 events were acquired using a BD^{™} FACSCanto II flow cytometer and analyzed with BD FACS DIVA Software.

### Results

A number of compounds described herein show inhibitory activity against TAK1, HCK, BTK and other kinases. Shown in Table 1 and 1a are exemplary *in vitro* IC₅₀ data of these compounds. Table 2 and 2a shows the *in vitro* EC₅₀ values of these compounds.

**Table 1.**

| **Structure** | **Compound ID** | **BTK IC₅₀ (nM)** | **HCK IC₅₀ (nM) Inv** | **TAK1 IC₅₀ (nM)** | **GCK IC₅₀ (nM)** |
|---|---|---|---|---|---|
| 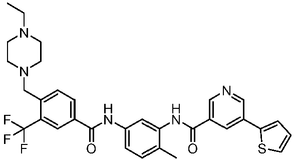 | **(I-1)** | 22.9 | 7.4 | 8210 | - |
| 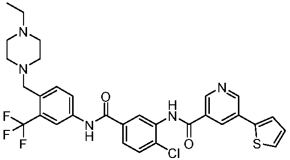 | **(I-2)*** | - | - | - | - |
| 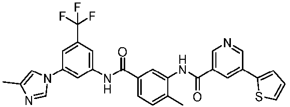 | **(I-3)*** | 196 | 62.2 | - | - |
| 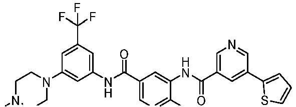 | **(I-4)*** | 459 | 22.8 | - | - |
| 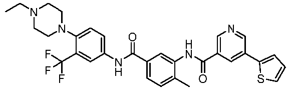 | **(I-5)*** | 3910 | 117 | > 10000 | - |
| 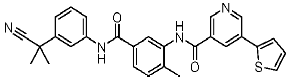 | **(I-6)*** | 1150 | 29.9 | > 3330 | - |
| 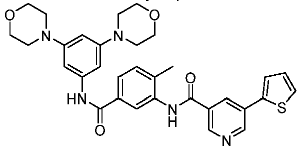 | **(I-7)*** | 3420 | 56.5 | - | - |

| | | | | | |
|---|---|---|---|---|---|
| *reference compounds | | | | | |

**Table 1a.**

| **Structure** | **Compound ID** | **BTK IC₅₀ (nM)** | **HCK IC₅₀ (nM) Inv** | **TAK1 IC₅₀ (nM)** |
|---|---|---|---|---|
| 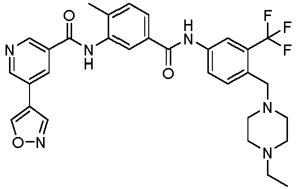 | **(I-8)*** | 69.7 | 5.33 | - |
| 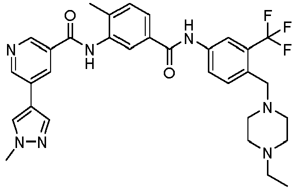 | **(I-9)*** | 25.3 | 2.43 | - |

| | | | | |
|---|---|---|---|---|
| *reference compounds | | | | |

**Table 2.**

| **Compound ID** | **BCWM.1 EC₅₀ (nM)** | **MWCL-1 EC₅₀ (nM)** | **RPCIWM-1 EC₅₀ (nM)** | **OCI-Ly3 EC₅₀ (nM)** | **Ramos EC₅₀ (nM)** | **OCI-Ly19 EC₅₀ (nM)** | **Mec1 EC₅₀ (nM)** |
|---|---|---|---|---|---|---|---|
| **(I-1)** | 38 | 459 | - | 2900 | 4150 | 552 | - |
| **(I-2)*** | 12 | 16 | 110 | 161 | 60 | 52 | - |
| **(I-3)*** | 141 | 355 | 725 | 411 | 555 | 508 | - |
| **(I-4)*** | - | - | - | - | - | - | - |
| **(I-5)*** | 365 | 1090 | 0.02 | 2160 | 989 | 1560 | - |
| **(I-6)*** | - | - | - | - | - | - | - |
| **(I-7)*** | 180 | 1760 | 4800 | 3540 | 2380 | 2970 | - |

| | | | | | | | |
|---|---|---|---|---|---|---|---|
| * reference compounds | | | | | | | |

**Table 2a.**

| **Compound ID** | **BCWM.1 EC₅₀ (nM)** | **MWCL-1 EC₅₀ (nM)** | **TMD8 EC₅₀ (nM)** | **OCI-Ly7 EC₅₀ (nM)** | **OCI-Ly3 EC₅₀ (nM)** | **Ramos EC₅₀ (nM)** | **OCI-Ly19 EC₅₀ (nM)** |
|---|---|---|---|---|---|---|---|
| **(I-8)*** | 2.7 | 15 | - | - | 76 | 329 | 56 |
| | < 1 | < 1 | | | 356 | | |
| **(I-9)*** | 542 | 294 | - | - | 5300 | 7470 | 2650 |
| | 305 | 281 | | | 356 | | |

| | | | | | | | |
|---|---|---|---|---|---|---|---|
| *reference compounds | | | | | | | |

### Kinative

The kinase selectivity of reference compound **I-6** was evaluated using a chemical proteomic approach named KiNativ which detects 260 kinases in A375 cells (ActivX Biosciences). To probe the intracellular targets of the compounds, A375 cells were incubated with the inhibitor at 1 µM final concentration and then looked for protection of labeling by an ATP-biotin probe that non-specifically labels conserved lysines on kinases and other nucleotide-dependent enzymes.

### Results

Table 3 shows that reference compound **I-6** inhibits a number of kinases at 1 µM, including Abl (>90%), FYN (55.7%), LYN (87.6%), and ZAK (>95%).

**Table 3.**

| **Kinase** | **Reference** | **Description** | **Sequence** | **SEQ ID NO:** | **Labeling Site** | **Compound 1-6 (1 µM)** |
|---|---|---|---|---|---|---|
| **ABL, ARG** | UniRef100_ P00519 | Proto-oncogene tyrosine-protein kinase ABL1 [Homo sapiens (Human)] | LMTGDTYTAHAGAK FPIK | 1 | Activation Loop | **>90** |
| **ACK** | UniRef100_ Q07912 | Activated CDC42 kinase 1 [Homo sapiens (Human)] | TVSVAVKCLKPDVLS QPEAMDDFIR | 2 | Lys1 | **-1.2** |
| **AMPKa1, AMPKa2** | UniRef100_ Q13131 | 5'-AMP-activated protein kinase catalytic subunit alpha-1 [Homo sapiens (Human)] | VAVKILNR | 3 | Lys1 | **15.3** |
| **ATR** | UniRef100_ Q13535 | Serine/threonine-protein kinase ATR [Homo sapiens (Human)] | FYIMMCKPK | 4 | ATP | **26.2** |
| **AurA** | UniRef100_ O14965 | Serine/threonine-protein kinase 6 [Homo sapiens (Human)] | DIKPENLLLGSAGEL K | 5 | Lys2 | **-21.6** |
| **AurB** | UniRef100_ Q96GD4 | Serine/threonine-protein kinase 12 [Homo sapiens (Human)] | SHFIVALKVLFK | 6 | Lys1 | **-94** |
| **BARK1** | UniRef100_ P25098 | Beta-adrenergic receptor kinase 1 n=1 Tax=Homo sapiens RepID=ARBK1_HUMAN | DLKPANILLDEHGHV R | 7 | Lys2 | **-1.2** |
| **BLK** | UniRef100_ P51451 | Tyrosine-protein kinase BLK n=2 Tax=Homo sapiens RepID=BLK_HUMAN | IIDSEYTAQEGAKFPI K | 8 | Activation Loop | **68.8** |
| **BRAF** | UniRef100_ P15056 | B-Raf proto-oncogene serine/threonine-protein kinase [Homo sapiens (Human)] | DLKSNNIFLHEDLTV K | 9 | Lys2 | **-20.3** |
| **BTK** | UniRef100_ Q06187 | Tyrosine-protein kinase BTK [Homo sapiens] | GQYDVAIKMIK | 10 | Lys1 | **7.8** |
| **BTK** | UniRef100_ Q06187 | Tyrosine-protein kinase BTK [Homo sapiens] | YVLDDEYTSSVGSK FPVR | 11 | Activation Loop | **-12.5** |
| **CaMK1d** | UniRef100_ Q8IU85 | Calcium/calmodulin-dependent protein kinase type 1D [Homo sapiens (Human)] | LFAVKCIPK | 12 | Lys1 | **-17.8** |
| **CaMK2a, CaMK2b, CaMK2d, CaMK2g** | UniRef100_ Q9UQM7 | Calcium/calmodulin-dependent protein kinase type II alpha chain [Homo sapiens (Human)] | DLKPENLLLASK | 13 | Lys2 | **0.7** |
| **CaMK2d** | UniRef100_ Q13557 | Calcium/calmodulin-dependent protein kinase type II delta chain n=2 Tax=Euarchontoglires ReplD=KCC2D_HUMAN | IPTGQEYAAKIINTKK | 14 | Lys1 | **-1.1** |
| **CaMK4** | UniRef100_ Q16566 | Calcium/calmodulin-dependent protein kinase type IV [Homo sapiens (Human)] | DLKPENLLYATPAPD APLK | 15 | Lys2 | **20.7** |
| **CDC2** | UniRef100_ P06493 | Cell division control protein 2 homolog [Homo sapiens (Human)] | DLKPQNLLIDDKGTI K | 16 | Lys2 | **-7.9** |
| **CDK11, CDK8** | UniRef100_ P49336 | Cell division protein kinase 8 [Homo sapiens (Human)] | DLKPANILVMGEGPE R | 17 | Lys2 | **20.7** |
| **CDK2** | UniRef100_ P24941 | Cell division protein kinase 2 [Homo sapiens (Human)] | DLKPQNLLINTEGAIK | 18 | Lys2 | **8.6** |
| **CDK5** | UniRef100_ Q00535 | Cell division protein kinase 5 [Homo sapiens (Human)] | DLKPQNLLINR | 19 | Lys2 | **9.3** |
| **CDK7** | UniRef100_ P50613 | Cell division protein kinase 7 [Homo sapiens (Human)] | DLKPNNLLLDENGVL K | 20 | Lys2 | **-6.8** |
| **CHK2** | UniRef100_ 096017 | Serine/threonine-protein kinase Chk2 [Homo sapiens (Human)] | VAIKIISK | 21 | Lys1 | **4.7** |
| **CK1a** | UniRef100_ P48729 | Casein kinase I isoform alpha [Homo sapiens (Human)] | DIKPDNFLMGIGR | 22 | Lys2 | **25.1** |
| **CK1g1** | UniRef100_ Q9HCP0 | Casein kinase I isoform gamma-1 [Homo sapiens (Human)] | DVKPENFLIGR | 23 | Lys2 | **14.4** |
| **CK1g2** | UniRef100_ P78368 | Casein kinase I isoform gamma-2 [Homo sapiens (Human)] | DVKPENFLVGRPGT K | 24 | Lys2 | **10.8** |
| **CSK** | UniRef100_ P41240 | Tyrosine-protein kinase CSK [Homo sapiens (Human)] | VSDFGLTKEASSTQ DTGKLPVK | 25 | Activation Loop | **23.3** |
| **DNAPK** | UniRef100_ P78527 | DNA-dependent protein kinase catalytic subunit [Homo sapiens (Human)] | EHPFLVKGGEDLR | 26 | ATP | **4.6** |
| **eEF2K** | UniRef100_ O00418 | Elongation factor 2 kinase [Homo sapiens (Human)] | YIKYNSNSGFVR | 27 | ATP | **12** |
| **Erk1** | UniRef100_ P27361 | Mitogen-activated protein kinase 3 [Homo sapiens (Human)] | DLKPSNLLINTTCDL K | 28 | Lys2 | **-3** |
| **Erk2** | UniRef100_ P28482 | Mitogen-activated protein kinase 1 [Homo sapiens] | DLKPSNLLLNTTCDL K | 29 | Lys2 | **-11** |
| **FER** | UniRef100_ P16591 | Proto-oncogene tyrosine-protein kinase FER n=2 Tax=Homo sapiens RepID=FER_HUMAN | TSVAVKTCKEDLPQ ELK | 30 | Lys1 | **-9.6** |
| **FGR** | UniRef100_ P09769 | Proto-oncogene tyrosine-protein kinase FGR [Homo sapiens (Human)] | LIKDDEYNPCQGSKF PIK | 31 | Activation Loop | **3.5** |
| **FRAP** | UniRef100_ P42345 | FKBP12-rapamycin complex-associated protein [Homo sapiens (Human)] | IQSIAPSLQVITSKQR PR | 32 | ATP | **-8.8** |
| **FYN, SRC, YES** | UniRef100_ P06241 | Proto-oncogene tyrosine-protein kinase Fyn n=2 Tax=Homo sapiens RepID=FYN_HUMAN | QGAKFPIKWTAPEA ALYGR | 33 | Activation Loop | **55.7** |
| **GCK** | UniRef100_ Q12851 | Mitogen-activated protein kinase kinase kinase kinase 2 n=1 Tax=Homo sapiens RepID=M4K2_HUMAN | DIKGANLLLTLQGDV K | 34 | Lys2 | **21.9** |
| **GSK3A** | UniRef100_ P49840 | Glycogen synthase kinase-3 alpha [Homo sapiens (Human)] | DIKPQNLLVDPDTAV LK | 35 | Lys2 | **17.3** |
| **GSK3B** | UniRef100_ P49841 | Glycogen synthase kinase-3 beta n=2 Tax=Homo sapiens RepID=GSK3B_HUMAN | DIKPQNLLLDPDTAV LK | 36 | Lys2 | **15.9** |
| **HPK1** | UniRef100_ Q92918 | Mitogen-activated protein kinase kinase kinase kinase 1 [Homo sapiens (Human)] | DKVSGDLVALKMVK | 37 | Lys1 | **7.5** |
| **IKKe** | UniRef100_ Q14164 | Inhibitor of nuclear factor kappa-B kinase epsilon subunit [Homo sapiens (Human)] | SGELVAVKVFNTTSY LRPR | 38 | Lys1 | **3.8** |
| **IKKe, TBK1** | UniRef100_ Q14164 | Inhibitor of nuclear factor kappa-B kinase epsilon subunit [Homo sapiens (Human)] | DIKPGNIMR | 39 | Lys2 | **24.8** |
| **ILK** | UniRef100_ Q13418 | Integrin-linked protein kinase 1 [Homo sapiens (Human)] | ISMADVKFSFQCPG R | 40 | Protein Kinase Domain | **17.2** |
| **IRAK4** | UniRef100_ Q9NWZ3 | Interleukin-1 receptor-associated kinase 4 [Homo sapiens (Human)] | DIKSANILLDEAFTAK | 41 | Lys2 | **-1.4** |
| **JAK1** | UniRef100_ P23458 | Tyrosine-protein kinase JAK1 n=1 Tax=Homo sapiens RepID=JAK1_HUMAN | QLASALSYLEDKDLV HGNVCTKNLLLAR | 42 | Protein Kinase Domain | **16.6** |
| **JAK1 domain2** | UniRef100_ P23458 | Tyrosine-protein kinase JAK1 n=1 Tax=Homo sapiens RepID=JAK1_HUMAN | YDPEGDNTGEQVAV KSLKPESGGNHIADL KK | 43 | Lys1 | **3.3** |
| **JNK1, JNK2, JNK3** | UniRef100_ P45983 | Mitogen-activated protein kinase 8 [Homo sapiens (Human)] | DLKPSNIVVK | 44 | Lys2 | **11.7** |
| **KHS1** | UniRef100_ Q9Y4K4 | Mitogen-activated protein kinase kinase kinase kinase 5 [Homo sapiens (Human)] | NVHTGELAAVKIIK | 45 | Lys1 | **-21.8** |
| **LCK** | UniRef100_ P06239 | Proto-oncogene tyrosine-protein kinase LCK n=2 Tax=Homo sapiens RepID=LCK_HUMAN | EGAKFPIKWTAPEAI NYGTFTIK | 46 | Activation Loop | **82.1** |
| **LKB1** | UniRef100_ Q15831 | Serine/threonine-protein kinase 11 [Homo sapiens (Human)] | DIKPGNLLLTTGGTL K | 47 | Lys2 | **4.9** |
| **LOK** | UniRef100_ O94804 | Serine/threonine-protein kinase 10 [Homo sapiens (Human)] | DLKAGNVLMTLEGDI R | 48 | Lys2 | **23.9** |
| **LYN** | UniRef100_ P07948 | Tyrosine-protein kinase Lyn n=1 Tax=Homo sapiens RepID=LYN_HUMAN | VAVKTLKPGTMSVQ AFLEEANLMK | 49 | Lys1 | **87.6** |
| **LYN** | UniRef100_ P07948 | Tyrosine-protein kinase Lyn n=1 Tax=Homo sapiens RepID=LYN_HUMAN | EGAKFPIKWTAPEAI NFGCFTIK | 50 | Activation Loop | **68.5** |
| **MAP2K1** | UniRef100_ Q02750 | Dual specificity mitogen-activated protein kinase kinase 1 n=4 Tax=Eutheria RepID=MP2K1_HUMAN | IMHRDVKPSNILVNS R | 51 | Lys2 | **10.8** |
| **MAP2K1, MAP2K2** | UniRef100_ Q02750 | Dual specificity mitogen-activated protein kinase kinase 1 n=4 Tax=Eutheria RepID=MP2K1_HUMAN | DVKPSNILVNSR | 52 | Lys2 | **4.1** |
| **MAP2K3** | UniRef100_ P46734 | Dual specificity mitogen-activated protein kinase kinase 3 [Homo sapiens (Human)] | DVKPSNVLINK | 53 | Lys2 | **4.9** |
| **MAP2K4** | UniRef100_ P45985 | Dual specificity mitogen-activated protein kinase kinase 4 [Homo sapiens (Human)] | DIKPSNILLDR | 54 | Lys2 | **-14.6** |
| **MAP2K5** | UniRef100_ Q13163 | Dual specificity mitogen-activated protein kinase kinase 5 n=1 Tax=Homo sapiens RepID=MP2K5_HUMAN | DVKPSNMLVNTR | 55 | Lys2 | **28.7** |
| **MAP2K6** | UniRef100_ P52564 | Dual specificity mitogen-activated protein kinase kinase 6 [Homo sapiens (Human)] | DVKPSNVLINALGQV K | 56 | Lys2 | **9.4** |
| **MAP2K7** | UniRef100_ 014733 | Dual specificity mitogen-activated protein kinase kinase 7 [Homo sapiens (Human)] | DVKPSNILLDER | 57 | Lys2 | **-3** |
| **MAP3K1** | UniRef100_ Q13233 | Mitogen-activated protein kinase kinase kinase 1 n=1 Tax=Homo sapiens RepID=M3K1_HUMAN | DVKGANLLIDSTGQR | 58 | Lys2 | **9.8** |
| **MAP3K2, MAP3K3** | UniRef100_ Q9Y2U5 | Mitogen-activated protein kinase kinase kinase 2 n=3 Tax=Homo sapiens RepID=M3K2_HUMAN | DIKGANILR | 59 | Lys2 | **-15.3** |
| **MAP3K4** | UniRef100_ Q9Y6R4 | Mitogen-activated protein kinase kinase kinase 4 [Homo sapiens (Human)] | DIKGANIFLTSSGLIK | 60 | Lys2 | **6.5** |
| **MAP3K5** | UniRef100_ Q99683 | Mitogen-activated protein kinase kinase kinase 5 [Homo sapiens (Human)] | DIKGDNVLINTYSGV LK | 61 | Lys2 | **16.2** |
| **MARK1, MARK2** | UniRef100_ Q7KZI7 | Serine/threonine-protein kinase MARK2 [Homo sapiens (Human)] | EVAVKIIDK | 62 | Lys1 | **-4.9** |
| **MARK2, MARK3** | UniRef100_ P27448 | MAP/microtubule affinity-regulating kinase 3 [Homo sapiens (Human)] | DLKAENLLLDADMNI K | 63 | Lys2 | **17.2** |
| **MARK3** | UniRef100_ P27448 | MAP/microtubule affinity-regulating kinase 3 [Homo sapiens (Human)] | EVAIKIIDKTQLNPTS LQK | 64 | Lys1 | **-2.2** |
| **MAST3** | UniRef100_ O60307 | Microtubule-associated serine/threonine-protein kinase 3 [Homo sapiens (Human)] | DLKPDNLLITSLGHIK | 65 | Lys2 | **18.9** |
| **MASTL** | UniRef100_ Q96GX5 | Microtubule-associated serine/threonine-protein kinase-like [Homo sapiens (Human)] | LYAVKVVK | 66 | Lys1 | **-3.7** |
| **MST1** | UniRef100_ Q13043 | Serine/threonine-protein kinase 4 [Homo sapiens (Human)] | ETGQIVAIKQVPVES DLQEIIK | 67 | Lys1 | **-26.3** |
| **MST2** | UniRef100_ Q13188 | Serine/threonine-protein kinase 3 [Homo sapiens (Human)] | ESGQVVAIKQVPVE SDLQEIIK | 68 | Lys1 | **-6.1** |
| **MST3** | UniRef100_ Q9Y6E0 | Serine/threonine-protein kinase 24 [Homo sapiens (Human)] | DIKAANVLLSEHGEV K | 69 | Lys2 | **-0.4** |
| **MST4, YSK1** | UniRef100_ O00506 | Serine/threonine-protein kinase 25 [Homo sapiens (Human)] | DIKAANVLLSEQGDV K | 70 | Lys2 | **22** |
| **NDR1** | UniRef100_ Q15208 | Serine/threonine-protein kinase 38 [Homo sapiens (Human)] | DTGHVYAMKILR | 71 | Lys1 | **-10.5** |
| **NDR2** | UniRef100_ Q9Y2H1 | Serine/threonine-protein kinase 38-like [Homo sapiens (Human)] | DIKPDNLLLDAK | 72 | Lys2 | **-12.5** |
| **NEK3** | UniRef100_ P51956 | Serine/threonine-protein kinase Nek3 [Homo sapiens (Human)] | SKNIFLTQNGK | 73 | Activation Loop | **10** |
| **NEK6, NEK7** | UniRef100_ Q9HC98 | Serine/threonine-protein kinase Nek6 [Homo sapiens (Human)] | DIKPANVFITATGVV K | 74 | Lys2 | **-3.7** |
| **NEK7** | UniRef100_ Q8TDX7 | Serine/threonine-protein kinase Nek7 [Homo sapiens (Human)] | AACLLDGVPVALKK | 75 | Lys1 | **10.7** |
| **NEK9** | UniRef100_ Q8TD19 | Serine/threonine-protein kinase Nek9 n=1 Tax=Homo sapiens RepID=NEK9_HUMAN | DIKTLNIFLTK | 76 | Lys2 | **-0.3** |
| **p38a** | UniRef100_ Q16539 | Mitogen-activated protein kinase 14 n=3 Tax=Eutheria RepID=MK14_HUMAN | QELNKTIWEVPER | 77 | Protein Kinase Domain | **85.1** |
| **p38d, p38g** | UniRef100_ P53778 | Mitogen-activated protein kinase 12 [Homo sapiens (Human)] | DLKPGNLAVNEDCE LK | 78 | Lys2 | **17.8** |
| **p70S6K** | UniRef100_ P23443 | Ribosomal protein S6 kinase 1 (EC 2.7.1.37) (S6K) (S6K1) (70 kDa ribosomal protein S6 kinase 1) (p70 S6 kinase alpha) (p70(S6K)-alpha) [Homo sapiens (Human)] | DLKPENIMLNHQGH VK | 79 | Lys2 | **7.4** |
| **PFTAIRE1** | UniRef100_ 094921 | Serine/threonine-protein kinase PFTAIRE-1 n=1 Tax=Homo sapiens RepID=PFTK1_HUMAN | LVALKVIR | 80 | Lys1 | **-31.3** |
| **PI4KB** | UniRef100_ Q9UBF8 | Phosphatidylinositol 4-kinase beta n=2 Tax=Homo sapiens RepID=PI4KB_HUMAN | VPHTQAVVLNSKDK | 81 | ATP | **-15.1** |
| **PIK3C3** | UniRef100_ Q8NEB9 | Phosphatidylinositol 3-kinase catalytic subunit type 3 [Homo sapiens (Human)] | TEDGGKYPVIFKHG DDLR | 82 | ATP | **-23.1** |
| **PIK3CB** | UniRef100_ P42338 | Phosphatidylinositol-4,5-bisphosphate 3-kinase catalytic subunit beta isoform [Homo sapiens (Human)] | VFGEDSVGVIFKNG DDLR | 83 | ATP | **2.8** |
| **PIK3CD** | UniRef100_ O00329 | Phosphatidylinositol-4,5-bisphosphate 3-kinase catalytic subunit delta isoform [Homo sapiens (Human)] | VNWLAHNVSKDNR Q | 84 | ATP | **-6.8** |
| **PIP4K2A** | UniRef100_ P48426 | Phosphatidylinositol-4-phosphate 5-kinase type II alpha (EC 2.7.1.68) (1-phosphatidylinositol-4-phosphate 5-kinase 2-alpha) (PtdIns(4)P-5- kinase isoform 2-alpha) (PIP5KII-alpha) (Diphosphoinositide kinase 2- alpha) (PtdIns(4)P-5-kinase B isoform) (PIP5KI | AKELPTLKDNDFINE GQK | 85 | ATP | **12.8** |
| **PIP4K2C** | UniRef100_ Q8TBX8 | Phosphatidylinositol-5-phosphate 4-kinase type-2 gamma n=1 Tax=Homo sapiens RepID=PI42C_HUMAN | TLVIKEVSSEDIADM HSNLSNYHQYIVK | 86 | ATP | **12.3** |
| **PIP5K3** | UniRef100_ Q9Y2I7 | FYVE finger-containing phosphoinositide kinase (EC 2.7.1.68) (1- phosphatidylinositol-4-phosphate 5-kinase) (Phosphatidylinositol-3- phosphate 5-kinase type III) (PIP5K) (PtdIns(4)P-5-kinase) [Homo sapiens (Human)] | GGKSGAAFYATEDD RFILK | 87 | ATP | **17.8** |
| **PITSLRE** | UniRef100_ P21127 | PITSLRE serine/threonine-protein kinase CDC2L1 [Homo sapiens (Human)] | DLKTSNLLLSHAGIL K | 88 | Lys2 | **1.9** |
| **PKCi** | UniRef100_ P41743 | Protein kinase C iota type [Homo sapiens (Human)] | DLKLDNVLLDSEGHI K | 89 | Lys2 | **-17.5** |
| **PKD1, PKD2** | UniRef100_ Q15139 | Serine/threonine-protein kinase D1 n=1 Tax=Homo sapiens RepID=KPCD1_HUMAN | NIVHCDLKPENVLLA SADPFPQVK | 90 | Lys2 | **-2.5** |
| **PKD2** | UniRef100_ Q9BZL6 | Protein kinase D2 [Homo sapiens (Human)] | DVAVKVIDK | 91 | Lys1 | **-17.8** |
| **PKD3** | UniRef100_ O94806 | Protein kinase D3 [Homo sapiens (Human)] | NIVHCDLKPENVLLA SAEPFPQVK | 92 | Lys2 | **-4.5** |
| **PKN1** | UniRef100_ Q16512 | Protein kinase N1 [Homo sapiens (Human)] | VLLSEFRPSGELFAI KALK | 93 | Lys1 | **-35.2** |
| **PKR** | UniRef100_ P19525 | Interferon-induced, double-stranded RNA-activated protein kinase [Homo sapiens (Human)] | DLKPSNIFLVDTK | 94 | Lys2 | **3.2** |
| **PLK1** | UniRef100_ P53350 | Serine/threonine-protein kinase PLK1 [Homo sapiens (Human)] | CFEISDADTKEVFAG KIVPK | 95 | Lys1 | **-5.4** |
| **PYK2** | UniRef100_ Q14289 | Protein tyrosine kinase 2 beta [Homo sapiens (Human)] | YIEDEDYYKASVTR | 96 | Activation Loop | **5.4** |
| **ROCK1, ROCK2** | UniRef100_ O75116 | Rho-associated protein kinase 2 [Homo sapiens (Human)] | DVKPDNMLLDK | 97 | Lys2 | **12.7** |
| **RSK1 domain1** | UniRef100_ Q15418 | Ribosomal protein S6 kinase alpha 1 [Homo sapiens (Human)] | DLKPENILLDEEGHIK LTDFGLSKEAIDHEK | 98 | Lys2 | **33.3** |
| **RSK1 domain2** | UniRef100_ Q15418 | Ribosomal protein S6 kinase alpha 1 [Homo sapiens (Human)] | DLKPSNILYVDESGN PECLR | 99 | Lys2 | **-22.9** |
| **RSK2 domain1** | UniRef100_ P51812 | Ribosomal protein S6 kinase alpha 3 [Homo sapiens (Human)] | DLKPENILLDEEGHIK LTDFGLSKESIDHEK | 100 | Lys2 | **5** |
| **RSK2 domain2** | UniRef100_ P51812 | Ribosomal protein S6 kinase alpha 3 [Homo sapiens (Human)] | DLKPSNILYVDESGN PESIR | 101 | Lys2 | **-15.8** |
| **SGK3** | UniRef100_ Q96BR1 | Serine/threonine-protein kinase Sgk3 [Homo sapiens (Human)] | FYAVKVLQK | 102 | Lys1 | **-7.2** |
| **SLK** | UniRef100_ Q9H2G2 | CTCL tumor antigen se20-9 [Homo sapiens (Human)] | DLKAGNILFTLDGDIK | 103 | Lys2 | **-19.5** |
| **STLKS** | UniRef100_ Q7RTN6 | STE20-related adaptor protein [Homo sapiens (Human)] | YSVKVLPWLSPEVL QQNLQGYDAK | 104 | Activation Loop | **-17.8** |
| **STLK6** | UniRef100_ Q9C0K7 | Serine/threonine-protein kinase ALS2CR2 [Homo sapiens (Human)] | SIKASHILISGDGLVT LSGLSHLHSLVK | 105 | Lys2 | **44.8** |
| **SYK** | UniRef100_ P43405 | Tyrosine-protein kinase SYK [Homo sapiens (Human)] | ISDFGLSKALR | 106 | Activation Loop | **-7.9** |
| **SYK** | UniRef100_ P43405 | Tyrosine-protein kinase SYK [Homo sapiens (Human)] | TVAVKILK | 107 | Lys1 | **-38.5** |
| **TAO1, TAO3** | UniRef100_ Q9H2K8 | Serine/threonine-protein kinase TAO3 [Homo sapiens (Human)] | DIKAGNILLTEPGQV K | 108 | Lys2 | **11.9** |
| **TAO2** | UniRef100_ Q9UL54 | Serine/threonine-protein kinase TAO2 n=2 Tax=Homo sapiens RepID=TAOK2_HUMAN | DVKAGNILLSEPGLV K | 109 | Lys2 | **33.4** |
| **TEC** | UniRef100_ P42680 | Tyrosine-protein kinase Tec n=2 Tax=Homo sapiens RepID=TEC_HUMAN | YVLDDQYTSSSGAK FPVK | 110 | Activation Loop | **-28.9** |
| **TLK1** | UniRef100_ Q9UKI8 | Serine/threonine-protein kinase tousled-like 1 [Homo sapiens (Human)] | YLNEIKPPIIHYDLKP GNILLVDGTACGEIK | 111 | Lys2 | **3.9** |
| **TLK2** | UniRef100_ Q86UE8 | Serine/threonine-protein kinase tousled-like 2 [Homo sapiens (Human)] | YLNEIKPPIIHYDLKP GNILLVNGTACGEIK | 112 | Lys2 | **-18.8** |
| **ULK3** | UniRef100_ Q6PHR2 | Unc-51-like kinase 3 [Homo sapiens (Human)] | EVVAIKCVAK | 113 | Lys1 | **-21.5** |
| **ZAK** | UniRef100_ Q9NYL2 | Mitogen-activated protein kinase kinase kinase MLT [Homo sapiens (Human)] | WISQDKEVAVKK | 114 | Lys1 | **>95** |
| **ZAP70** | UniRef100_ P43403 | Tyrosine-protein kinase ZAP-70 [Homo sapiens (Human)] | ISDFGLSKALGADDS YYTAR | 115 | Activation Loop | **7.5** |
| **ZC1/HGK, ZC2/TNIK, ZC3/MINK** | UniRef100_ 095819 | Mitogen-activated protein kinase kinase kinase kinase 4 [Homo sapiens (Human)] | DIKGQNVLLTENAEV K | 116 | Lys2 | **27** |

### EQUIVALENTS AND SCOPE

In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

Furthermore, the invention encompasses all variations, combinations, and permutations in which one or more limitations, elements, clauses, and descriptive terms from one or more of the listed claims is introduced into another claim. For example, any claim that is dependent on another claim can be modified to include one or more limitations found in any other claim that is dependent on the same base claim. Where elements are presented as lists, *e.g.,* in Markush group format, each subgroup of the elements is also disclosed, and any element(s) can be removed from the group. It should it be understood that, in general, where the invention, or aspects of the invention, is/are referred to as comprising particular elements and/or features, certain embodiments of the invention or aspects of the invention consist, or consist essentially of, such elements and/or features. For purposes of simplicity, those embodiments have not been specifically set forth *in haec verba* herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments of the invention, to the tenth of the unit of the lower limit of the range, unless the context clearly dictates otherwise.

In addition, any particular embodiment of the present invention that falls within the prior art may be explicitly excluded from any one or more of the claims. Because such embodiments are deemed to be known to one of ordinary skill in the art, they may be excluded even if the exclusion is not set forth explicitly herein. Any particular embodiment of the invention can be excluded from any claim, for any reason, whether or not related to the existence of prior art.

Those skilled in the art will recognize or be able to ascertain using no more than routine experimentation many equivalents to the specific embodiments described herein. The scope of the present embodiments described herein is not intended to be limited to the above Description, but rather is as set forth in the appended claims.

## Claims

1. A compound of Formula **(I-a1), (I-a2)** or **(I-a3):** 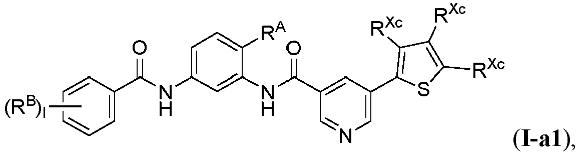 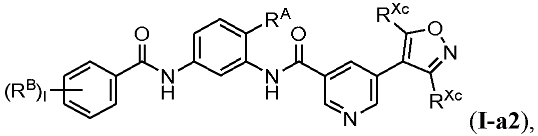 or 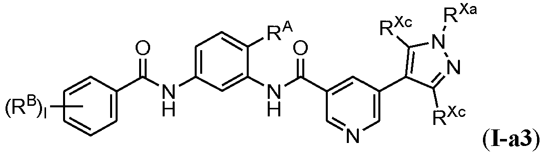
or a pharmaceutically acceptable salt, solvate, hydrate, polymorph, co-crystal, tautomer, or stereoisomer thereof;
wherein:
R^{A} is substituted or unsubstituted C₁₋₆ alkyl;
each instance of R^{B} is independently selected from the group consisting of optionally substituted alkyl, optionally substituted heterocyclyl, and optionally substituted heteroaryl;
R^{Xa} is optionally substituted C₁₋₆ alkyl;
each instance of R^{Xc} is hydrogen; and
l is 1 or 2;
carbon atom substituents are independently selected from the group consisting of halogen, -CN, -NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, -C(=O)R^{aa}, -CO₂H, -CHO, -C(OR^{cc})₂, -CO₂R^{aa}, - OC(=O)R^{aa}, -OCO₂R^{aa}, -C(=O)N(R^{bb})₂, -OC(=O)N(R^{bb})₂, -NR^{bb}C(=O)R^{aa}, -NR^{bb}CO₂R^{aa}, - NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa},-C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa},-NR^{bb}SO₂R^{aa}, -SO₂N(R^{bb})₂, -SO₂R^{aa}, -SO₂OR^{aa}, -OSO₂R^{aa}, -S(=O)R^{aa}, -OS(=O)R^{aa}, - Si(R^{aa})₃, -OSi(R^{aa})₃ -C(=S)N(R^{bb})₂, -C(=O)SR^{aa}, -C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)₂R^{aa}, -OP(=O)₂R^{aa}, -P(=O)(R^{aa})₂, - OP(=O)(R^{aa})₂, -OP(=O)(OR^{cc})₂, -P(=O)₂N(R^{bb})₂, -OP(=O)₂N(R^{bb})₂, -P(=O)(NR^{bb})₂, - OP(=O)(NR^{bb})₂, -NR^{bb}P(=O)(OR^{cc})₂, -NR^{bb}P(=O)(NR^{bb})₂, -P(R^{cc})₂, -P(R^{cc})₃, -OP(R^{cc})₂,-OP(R^{cc})₃, -B(R^{aa})₂, -B(OR^{cc})₂, -BR^{aa}(OR^{cc}), C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups;
or two geminal hydrogens on a carbon atom are replaced with the group =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb}, or =NOR^{cc};
nitrogen atom substituents are nitrogen protecting groups;
each instance of the nitrogen protecting group is independently -OH, -OR^{aa}, - N(R^{cc})₂, -C(=O)R^{aa}, -C(=O)N(R^{cc})₂, -CO₂R^{aa}, -SO₂R^{aa}, -C(=NR^{cc})R^{aa}, -C(=NR^{cc})OR^{aa}, - C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, - C(=S)SR^{cc}, C₁₋₁₀ alkyl, C₂₋₁₀ alkenyl, C₂₋₁₀ alkynyl, C₁₋₁₀ heteroalkyl, C₂₋₁₀ heteroalkenyl, C₂₋₁₀ heteroalkynyl, C₃₋₁₀ carbocyclyl, 3-14 membered heterocyclyl, C₆₋₁₄ aryl, or 5-14 membered heteroaryl, wherein each of the alkyl, alkenyl, alkynyl, heteroalkyl, heteroalkenyl, heteroalkynyl, carbocyclyl, heterocyclyl, aralkyl, aryl, and heteroaryl is independently substituted with 0, 1, 2, 3, 4, or 5 R^{dd} groups;
each instance of R^{aa} is, independently, selected from C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀alkenyl, heteroC₂₋₁₀alkynyl, C₃₋₁₀ carbocyclyl, 3-14 membered heterocyclyl, C₆₋₁₄ aryl, and 5-14 membered heteroaryl, or two R^{aa} groups are joined to form a 3-14 membered heterocyclyl or 5-14 membered heteroaryl ring, wherein each of the alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups;
each instance of R^{bb} is, independently, selected from hydrogen, -OH, -OR^{aa}, - N(R^{cc})₂, -CN, -C(=O)R^{aa}, -C(=O)N(R^{cc})₂, -CO₂R^{aa}, -SO₂R^{aa}, -C(=NR^{cc})OR^{aa}, - C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, - C(=S)SR^{cc}, -P(=O)₂R^{aa}, -P(=O)(R^{aa})₂, -P(=O)₂N(R^{cc})₂, -P(=O)(NR^{cc})₂, C₁₋₁₀ alkyl, C₁₋₁₀ perhaloalkyl, C₂₋₁₀ alkenyl, C₂₋₁₀ alkynyl, heteroC₁₋₁₀alkyl, heteroC₂₋₁₀alkenyl, heteroC₂₋₁₀akynyl, C₃₋₁₀ carbocyclyl, 3-14 membered heterocyclyl, C₆₋₁₄ aryl, and 5-14 membered heteroaryl, or two R^{bb} groups are joined to form a 3-14 membered heterocyclyl or 5-14 membered heteroaryl ring, wherein each of the alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups;
each instance of R^{cc} is, independently, selected from hydrogen, C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl, or two R^{cc} groups are joined to form a 3-14 membered heterocyclyl or 5-14 membered heteroaryl ring, wherein each of the alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups;
each instance of R^{dd} is, independently, selected from halogen, -CN, -NO₂, -N₃, - SO₂H, -SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, - SSR^{ee}, -C(=O)R^{ee}, -CO₂H, -CO₂R^{ee}, -OC(=O)R^{ee}, -OCO₂R^{ee}, -C(=O)N(R^{ff})₂, - OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee}, - OC(=NR^{ff})R^{ee}, -OC(=NR^{ff})OR^{ee}, -C(=NR^{ff})N(R^{ff})₂, -OC(=NR^{ff})N(R^{ff})₂, - NR^{ff}C(=NR^{ff})N(R^{ff})₂,-NR^{ff}SO₂R^{ee}, -SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee}, -C(=S)SR^{ee}, -SC(=S)SR^{ee}, -P(=O)₂R^{ee}, - P(=O)(R^{ee})₂, -OP(=O)(R^{ee})₂, -OP(=O)(OR^{ee})₂, C₁₋₆ alkyl, C₁₋₆ perhaloalkyl, C₂₋₆ alkenyl, C₂₋₆ alkynyl, heteroC₁₋₆alkyl, heteroC₂₋₆alkenyl, heteroC₂₋₆alkynyl, C₃₋₁₀ carbocyclyl, 3-10 membered heterocyclyl, C₆₋₁₀ aryl, and 5-10 membered heteroaryl, wherein each of the alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups, or two geminal R^{dd} substituents are joined to form =O or =S;
each instance of R^{ee} is, independently, selected from C₁₋₆ alkyl, C₁₋₆ perhaloalkyl, C₂₋₆ alkenyl, C₂₋₆ alkynyl, heteroC₁₋₆ alkyl, heteroC₂₋₆alkenyl, heteroC₂₋₆ alkynyl, C₃₋₁₀ carbocyclyl, C₆₋₁₀ aryl, 3-10 membered heterocyclyl, and 3-10 membered heteroaryl, wherein each of the alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups;
each instance of R^{ff} is, independently, selected from hydrogen, C₁₋₆ alkyl, C₁₋₆ perhaloalkyl, C₂₋₆ alkenyl, C₂₋₆ alkynyl, heteroC₁₋₆alkyl, heteroC₂₋₆alkenyl, heteroC₂₋₆alkynyl, C₃₋₁₀ carbocyclyl, 3-10 membered heterocyclyl, C₆₋₁₀ aryl, and 5-10 membered heteroaryl, or two R^{ff} groups are joined to form a 3-10 membered heterocyclyl or 5-10 membered heteroaryl ring, wherein each of the alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups;
each instance of R^{gg} is, independently, halogen, -CN, -NO₂, -N₃, -SO₂H, -SO₃H, - OH, -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, -NH₂(C₁₋₆ alkyl) ⁺X⁻, -NH₃⁺X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, - OC(=O)NH(C₁₋₆ alkyl), -NHC(=O)( C₁₋₆ alkyl), -N(C₁₋₆ alkyl)C(=O)( C₁₋₆ alkyl), - NHCO₂(C₁₋₆ alkyl), -NHC(=O)N(C₁₋₆ alkyl)₂, -NHC(=O)NH(C₁₋₆ alkyl), -NHC(=O)NH₂, - C(=NH)O(C₁₋₆ alkyl),-OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, -OC(NH)NH(C₁₋₆ alkyl), -OC(NH)NH₂, -NHC(NH)N(C₁₋₆ alkyl)₂, -NHC(=NH)NH₂, -NHSO₂(C₁₋₆ alkyl), - SO₂N(C₁₋₆ alkyl)₂, -SO₂NH(C₁₋₆ alkyl), -SO₂NH₂,-SO₂C₁₋₆ alkyl, -SO₂OC₁₋₆ alkyl, - OSO₂C₁₋₆ alkyl, -SOC₁₋₆ alkyl, -Si(C₁₋₆ alkyl)₃, -OSi(C₁₋₆ alkyl)₃ -C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ perhaloalkyl, C₂₋₆ alkenyl, C₂₋₆ alkynyl, heteroC₁₋₆alkyl, heteroC₂₋₆alkenyl, heteroC₂₋₆alkynyl, C₃₋₁₀ carbocyclyl, C₆₋₁₀ aryl, 3-10 membered heterocyclyl, or 5-10 membered heteroaryl; or two geminal R^{gg} substituents are joined to form =O or =S; and
X⁻ is a counterion.

2. The compound of claim 1, or a pharmaceutically acceptable salt, solvate, hydrate, polymorph, co-crystal, tautomer, or stereoisomer thereof, wherein R^{A} is methyl.

3. The compound of claim 1 or 2, wherein the compound is of the formula: 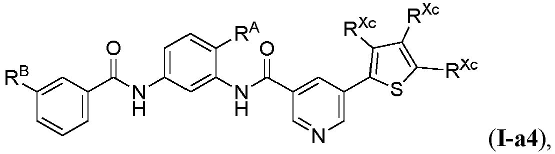 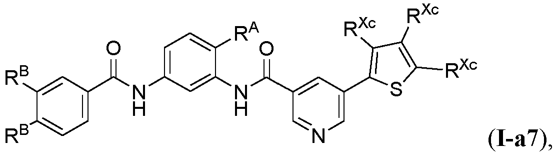 or 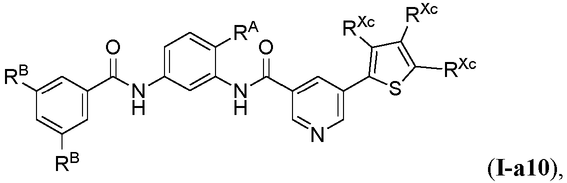 or a pharmaceutically acceptable salt, solvate, hydrate, polymorph, co-crystal, tautomer, or stereoisomer thereof.

4. The compound of claim 1 or 2, wherein the compound is of the formula: 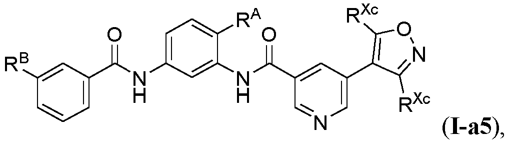 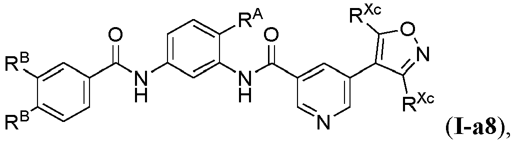 or 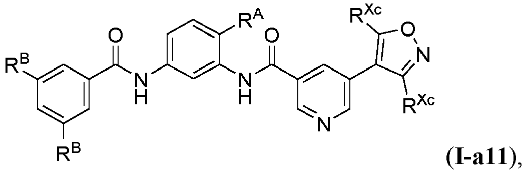 or a pharmaceutically acceptable salt, solvate, hydrate, polymorph, co-crystal, tautomer, or stereoisomer thereof.

5. The compound of claim 1 or 2, wherein the compound is of the formula: 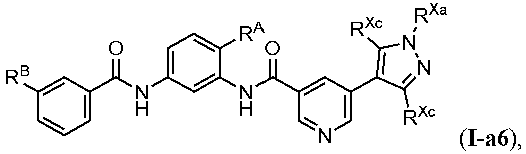 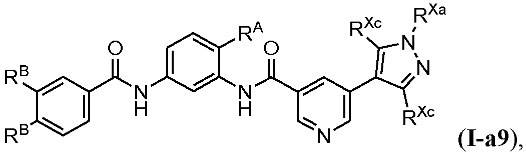 or 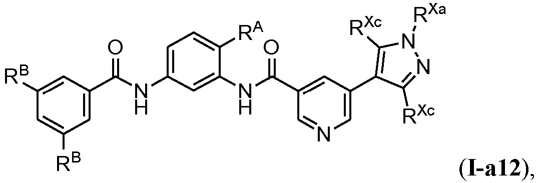 or a pharmaceutically acceptable salt, solvate, hydrate, polymorph, co-crystal, tautomer, or stereoisomer thereof.

6. The compound claim 1 or 2, or a pharmaceutically acceptable salt, solvate, hydrate, polymorph, co-crystal, tautomer, or stereoisomer thereof, wherein l is 1, and R^{B} is *meta* to the point of attachment of the amide linker; or
wherein l is 2, and two R^{B} groups are *meta* to the point of attachment of the amide linker, or one R^{B} group is *meta* to the point of attachment of the amide linker, and the second R^{B} group is *para* to the point of attachment of the amide linker.

7. The compound of any of claims 1-6, or a pharmaceutically acceptable salt, solvate, hydrate, polymorph, co-crystal, tautomer, or stereoisomer thereof, wherein one R^{B} group is substituted or unsubstituted C₁₋₆alkyl.

8. The compound of claim 7, or a pharmaceutically acceptable salt, solvate, hydrate, polymorph, co-crystal, tautomer, or stereoisomer thereof, wherein one R^{B} group is C₁₋₆alkyl substituted with one -CN group, preferably
wherein one R^{B} group is 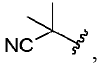 or
wherein one R^{B} group is substituted or unsubstituted -CH₂-(piperazinyl), preferably
wherein one R^{B} group is 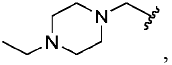 or
wherein one R^{B} group is haloalkyl, preferably
wherein one R^{B} group is -CF₃.

9. The compound of any of claims 1-6, or a pharmaceutically acceptable salt, solvate, hydrate, polymorph, co-crystal, tautomer, or stereoisomer thereof, wherein one R^{B} group is substituted or unsubstituted imidazolyl, preferably
wherein one R^{B} group is

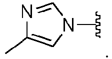

10. The compound of any of claims 1-6, or a pharmaceutically acceptable salt, solvate, hydrate, polymorph, co-crystal, tautomer, or stereoisomer thereof, wherein one R^{B} group is substituted or unsubstituted piperazinyl, preferably
wherein one R^{B} group is 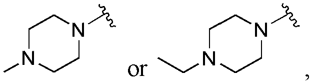 or
wherein one R^{B} group is substituted or unsubstituted morpholine, or
wherein two R^{B} groups are substituted or unsubstituted morpholine.

11. The compound of any of claims 1, 2, and 5-10, or a pharmaceutically acceptable salt, solvate, hydrate, polymorph, co-crystal, tautomer, or stereoisomer thereof,
wherein R^{Xa} is methyl or ethyl.

12. The compound of claim 1, wherein the compound is of the formula: 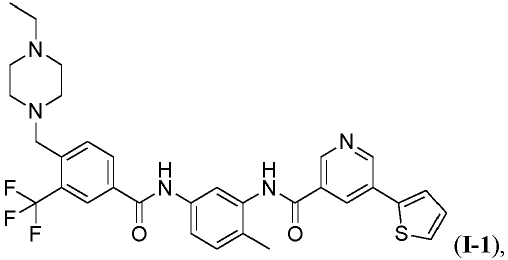 or a pharmaceutically acceptable salt thereof.

13. A pharmaceutical composition comprising a compound of any one of claims 1-12, or a pharmaceutically acceptable salt, solvate, hydrate, polymorph, co-crystal, tautomer, or stereoisomer thereof, and optionally a pharmaceutically acceptable excipient, preferably wherein the pharmaceutical composition further comprises an additional pharmaceutical agent, more preferably wherein the pharmaceutical composition further comprises one or more additional known chemotherapeutic agents.

14. A compound of any one of claims 1-12, or a pharmaceutically acceptable salt, solvate, hydrate, polymorph, co-crystal, tautomer, or stereoisomer thereof, or a pharmaceutical composition of claim 13, for use in treating a B cell neoplasm in a subject,
preferably wherein the compound, or a pharmaceutically acceptable salt, solvate, hydrate, polymorph, co-crystal, tautomer, or stereoisomer thereof, or pharmaceutical composition inhibits transforming growth factor b-activated kinase-1 (TAK1), hemopoietic cell kinase (HCK), or both TAK1 and HCK, more preferably
wherein the compound, or a pharmaceutically acceptable salt, solvate, hydrate, polymorph, co-crystal, tautomer, or stereoisomer thereof, or pharmaceutical composition inhibits hematopoetic progenitor kinase 1(HPK1), even more preferably
wherein the compound, or a pharmaceutically acceptable salt, solvate, hydrate, polymorph, co-crystal, tautomer, or stereoisomer thereof, or pharmaceutical composition is administered in combination with an inhibitor of Bruton's
tyrosine kinase (BTK), interleukin-1 receptor-associated kinase 1 (IRAK1), interleukin-1 receptor-associated kinase 4 (IRAK4), transforming growth factor b-activated kinase-1 (TAK1), or a Src family kinase, most preferably
wherein the BTK inhibitor is a benzonaphthyridinone, and/or wherein the B cell neoplasm is selected from the group consisting of Hodgkin's lymphoma and most non-Hodgkin's lymphomas, preferably
wherein the lymphoma is Waldenström's macroglobulinemia, diffuse large B cell lymphoma, Follicular lymphoma, mucosa-Associated Lymphatic Tissue lymphoma (MALT), Small cell lymphocytic lymphoma (overlaps with Chronic lymphocytic leukemia), Mantle cell lymphoma (MCL), Burkitt lymphoma, Mediastinal large B cell lymphoma, Nodal marginal zone B cell lymphoma (NMZL), Splenic marginal zone lymphoma (SMZL), Intravascular large B-cell lymphoma, Primary effusion lymphoma and lymphomatoid granulomatosis, more preferably
wherein the B cell neoplasm is Waldenström's macroglobulinemia, even more preferably
wherein the subject has a mutation at position 38182641 in chromosome 3p22.2, and/or
wherein the subject is receiving therapy for the B cell neoplasm, and/or
wherein the compound, or a pharmaceutically acceptable salt, solvate, hydrate, polymorph, co-crystal, tautomer, or stereoisomer thereof, or pharmaceutical composition is co-administered with one or more other chemotherapeutic agents.

15. The compound, pharmaceutically acceptable salt, solvate, hydrate, polymorph, co-crystal, tautomer, stereoisomer, or pharmaceutical composition for use of claim 14,
wherein the B cell neoplasm is Waldenström's macroglobulinemia.

## Patentansprüche

1. Verbindung der Formel **(I-al), (I-a2)** oder **(I-a3):** 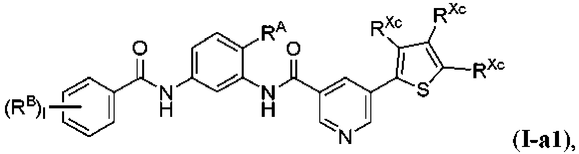 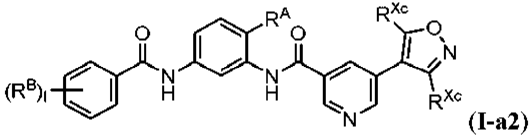 oder 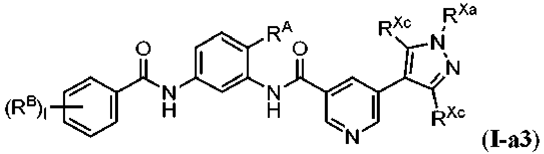
oder pharmazeutisch unbedenkliches Salz, Solvat, Hydrat, Polymorph, Co-Kristall, Tautomer oder Stereoisomer davon;
wobei:
R^{A} substituiertes oder unsubstituiertes C₁₋₆-Alkyl ist;
jede Instanz von R^{B} unabhängig aus der Gruppe ausgewählt ist, die aus optional substituiertem Alkyl, optional substituiertem Heterocyclyl und optional substituiertem Heteroaryl besteht;
R^{Xa} optional substituiertes C₁₋₆-Alkyl ist;
jede Instanz von R^{Xc} Wasserstoff ist; und
11 oder 2 ist;
Kohlenstoffatomsubstituenten unabhängig aus der Gruppe ausgewählt sind, die aus Folgendem besteht, Halogen, -CN, -NO₂, -N₃, -SO₂H, -SO₃H, -OH, -OR^{aa}, -ON(R^{bb})₂, -N(R^{bb})₂, - N(R^{bb})₃⁺X⁻, -N(OR^{cc})R^{bb}, -SH, -SR^{aa}, -SSR^{cc}, -C(=O)R^{aa}, -CO₂H, -CHO, -C(OR^{cc})₂, -CO₂R^{aa}, - OC(=O)R^{aa}, -OCO₂R^{aa}, -C(=O)N(R^{bb})₂, -OC(=O)N(R^{bb})₂, -NR^{bb}C(=O)R^{aa}, -NR^{bb}CO₂R^{aa}, - NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa}, - C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa}, - NR^{bb}SO₂R^{aa}, -SO₂N(R^{bb})₂, -SO₂R^{aa}, -SO₂OR^{aa}, -OSO₂R^{aa}, -S(=O)R^{aa}, -OS(=O)R^{aa}, -Si(R^{aa})₃, - OSi(R^{aa})₃ -C(=S)N(R^{bb})₂, -C(=O)SR^{aa}, -C(=S)SR^{aa}, -SC(=S)SR^{aa}, -SC(=O)SR^{aa}, -OC(=O)SR^{aa}, - SC(=O)OR^{aa}, -SC(=O)R^{aa}, -P(=O)₂R^{aa}, -OP(=O)₂R^{aa}, -P(=O)(R^{aa})₂, -OP(=O)(R^{aa})₂, - OP(=O)(OR^{cc})₂, -P(=O)₂N(R^{bb})₂, -OP(=O)₂N(R^{bb})₂, -P(=O)(NR^{bb})₂, -OP(=O)(NR^{bb})₂, - NR^{bb}P(=O)(OR^{cc})₂, -NR^{bb}P(=O)(NR^{bb})₂, -P(R^{cc})₂, -P(R^{cc})₃, -OP(R^{cc})₂, -OP(R^{cc})₃, -B(R^{aa})₂, - B(OR^{cc})₂, -BR^{aa}(OR^{cc}), C₁₋₁₀-Alkyl, C₁₋₁₀-Perhalogenalkyl, C₂₋₁₀-Alkenyl, C₂₋₁₀-Alkynyl, Hetero-C₁₋₁₀-alkyl, Hetero-C₂₋₁₀-alkenyl, Hetero-C₂₋₁₀-alkynyl, C₃₋₁₀-Carbocyclyl, 3-14-gliedrigem Heterocyclyl, C₆₋₁₄-Aryl und 5-14-gliedrigem Heteroaryl, wobei jedes Alkyl, Alkenyl, Alkynyl, Heteroalkyl, Heteroalkenyl, Heteroalkynyl, Carbocyclyl, Heterocyclyl, Aryl und Heteroaryl unabhängig mit 0, 1, 2, 3, 4 oder 5 R^{dd}-Gruppen substituiert ist;
oder zwei geminale Wasserstoffe an einem Kohlenstoffatom durch die Gruppe =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb} oder =NOR^{cc} ersetzt sind;
Stickstoffatomsubstituenten Stickstoffschutzgruppen sind;
jede Instanz der Stickstoffschutzgruppe unabhängig Folgendes ist, -OH, -OR^{aa}, -N(R^{cc})₂, - C(=O)R^{aa}, -C(=O)N(R^{cc})₂, -CO₂R^{aa}, -SO₂R^{aa}, -C(=NR^{cc})R^{aa}, -C(=NR^{cc})OR^{aa}, -C(=NR^{cc})N(R^{cc})₂, - SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, C₁₋₁₀-Alkyl, C₂₋₁₀-Alkenyl, C₂₋₁₀-Alkinyl, C₁₋₁₀-Heteroalkyl, C₂₋₁₀-Heteroalkenyl, C₂₋₁₀-Heteroalkinyl, C₃₋₁₀-Carbocyclyl, 3-14-gliedriges Heterocyclyl, C₆₋₁₄-Aryl oder 5-14-gliedriges Heteroaryl, wobei jedes von dem Alkyl, Alkenyl, Alkinyl, Heteroalkyl, Heteroalkenyl, Heteroalkinyl, Carbocyclyl, Heterocyclyl, Aralkyl, Aryl und Heteroaryl unabhängig mit 0, 1, 2, 3, 4 oder 5 R^{dd}-Gruppen substituiert ist;
jede Instanz von R^{aa} unabhängig aus Folgendem ausgewählt ist, C₁₋₁₀-Alkyl, C₁₋₁₀-Perhaloalkyl, C₂₋₁₀-Alkenyl, C₂₋₁₀-Alkinyl, Hetero-C₁₋₁₀-alkyl, Hetero-C₂₋₁₀-alkenyl, Hetero-C₂₋₁₀-alkinyl, C₃₋₁₀-Carbocyclyl, 3-14-gliedrigem Heterocyclyl, C₆₋₁₄-Aryl und 5-14-gliedrigem Heteroaryl oder zwei R^{aa}-Gruppen verbunden sind, um einen 3-14-gliedrigen Heterocyclyl- oder 5-14-gliedrigen Heteroarylring zu bilden, wobei jedes von dem Alkyl, Alkenyl, Alkinyl, Heteroalkyl, Heteroalkenyl, Heteroalkinyl, Carbocyclyl, Heterocyclyl, Aryl und Heteroaryl unabhängig mit 0, 1, 2, 3, 4 oder 5 R^{dd}-Gruppen substituiert ist;
jede Instanz von R^{bb} unabhängig aus Folgendem ausgewählt ist, Wasserstoff, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, - P(=O)(R^{aa})₂, -P(=O)₂N(R^{cc})₂, -P(=O)(NR^{cc})₂, C₁₋₁₀-Alkyl, C₁₋₁₀-Perhaloalkyl, C₂₋₁₀-Alkenyl, C₂₋₁₀-Alkinyl, Hetero-C₁₋₁₀-alkyl, Hetero-C₂₋₁₀-alkenyl, Hetero-C₂₋₁₀-alkinyl, C₃₋₁₀-Carbocyclyl, 3-14-gliedrigem Heterocyclyl, C₆₋₁₄-Aryl und 5-14-gliedrigem Heteroaryl, oder zwei R^{bb}-Gruppen verbunden sind, um ein 3-14-gliedriges Heterocyclyl oder einen 5- bis 14-gliedrigen Heteroarylring zu bilden, wobei jedes von dem Alkyl, Alkenyl, Alkinyl, Heteroalkyl, Heteroalkenyl, Heteroalkinyl, Carbocyclyl, Heterocyclyl, Aryl und Heteroaryl unabhängig mit 0, 1, 2, 3, 4 oder 5 R^{dd}-Gruppen substituiert ist;
jede Instanz von R^{cc} unabhängig aus Folgendem ausgewählt ist, Wasserstoff, C₁₋₁₀ -Alkyl, C₁₋₁₀-Perhaloalkyl, C₂₋₁₀-Alkenyl, C₂₋₁₀-Alkinyl, Hetero-C₁₋₁₀-alkyl, Hetero-C₂₋₁₀-alkenyl, Hetero-C₂₋₁₀-alkinyl, C₃₋₁₀-Carbocyclyl, 3-14-gliedrigem Heterocyclyl, C₆₋₁₄-Aryl und 5-14-gliedrigem Heteroaryl oder zwei R^{cc}-Gruppen verbunden sind, um einen 3-14-gliedrigen Heterocyclyl- oder 5-14-gliedrigen Heteroarylring zu bilden, wobei jedes von dem Alkyl, Alkenyl, Alkinyl, Heteroalkyl, Heteroalkenyl, Heteroalkinyl, Carbocyclyl, Heterocyclyl, Aryl und Heteroaryl unabhängig mit 0, 1, 2, 3, 4 oder 5 R^{dd}-Gruppen substituiert ist;
jede Instanz von R^{dd} unabhängig aus Folgendem ausgewählt ist, Halogen, -CN, -NO₂, -N₃, -SO₂H, -SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, -SSR^{ee}, - C(=O)R^{ee}, -CO₂H, -CO₂R^{ee}, -OC(=O)R^{ee}, -OCO₂R^{ee}, -C(=O)N(R^{ff})₂, -OC(=O)N(R^{ff})₂, - NR^{ff}C(=O)R^{ee}, -NR^{ff}CO₂R^{ee}, -NR^{ff}C(=O)N(R^{ff})₂, -C(=NR^{ff})OR^{ee}, -OC(=NR^{ff})R^{ee}, - OC(=NR^{ff})OR^{ee}, -C(=NR^{ff})N(R^{ff})₂, -OC(=NR^{ff})N(R^{ff})₂, -NR^{ff}C(=NR^{ff})N(R^{ff})₂,-NR^{ff}SO₂R^{ee}, - SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, -C(=S)N(R^{ff})₂, - C(=O)SR^{ee}, -C(=S)SR^{ee}, -SC(=S)SR^{ee}, -P(=O)₂R^{ee}, -P(=O)(R^{ee})₂, -OP(=O)(R^{ee})₂, -OP(=O)(OR^{ee})₂, C₁₋₆-Alkyl, C₁₋₆-Perhalogenalkyl, C₂₋₆-Alkenyl, C₂₋₆-Alkinyl, Hetero-C₁₋₆-alkyl, Hetero-C₂₋₆-alkenyl, Hetero-C₂₋₆-alkinyl, C₃₋₁₀-Carbocyclyl, 3-10 gliedrigem Heterocyclyl, C₆₋₁₀-Aryl und 5-10-gliedrigem Heteroaryl, wobei jedes von dem Alkyl, Alkenyl, Alkinyl, Heteroalkyl, Heteroalkenyl, Heteroalkinyl, Carbocyclyl, Heterocyclyl, Aryl und Heteroaryl unabhängig mit 0, 1, 2, 3, 4 oder 5 R^{gg}-Gruppen substituiert ist oder zwei geminale R^{dd}-Substituenten verbunden sind, um =O oder =S zu bilden;
jede Instanz von R^{ee} unabhängig aus Folgendem ausgewählt ist, C₁₋₆-Alkyl, C₁₋₆-Perhaloalkyl, C₂₋₆-Alkenyl, C₂₋₆-Alkinyl, Hetero-C₁₋₆-alkyl, Hetero-C₂₋₆-alkenyl, Hetero-C₂₋₆-alkinyl, C₃₋₁₀-Carbocyclyl, C₆₋₁₀-Aryl, 3-10-gliedrigem Heterocyclyl und 3-10-gliedrigem Heteroaryl, wobei jedes von dem Alkyl, Alkenyl, Alkinyl, Heteroalkyl, Heteroalkenyl, Heteroalkinyl, Carbocyclyl, Heterocyclyl, Aryl und Heteroaryl unabhängig mit 0, 1, 2, 3, 4 oder 5 R^{gg}-Gruppen substituiert ist;
jede Instanz von R^{ff} unabhängig aus Folgendem ausgewählt ist, Wasserstoff, C₁₋₆-Alkyl, C₁₋₆-Perhaloalkyl, C₂₋₆-Alkenyl, C₂₋₆-Alkinyl, Hetero-C₁₋₆-alkyl, Hetero-C₂₋₆-alkenyl, Hetero-C₂₋₆-alkinyl, C₃₋₁₀-Carbocyclyl, 3-10-gliedrigem Heterocyclyl, C₆₋₁₀-Aryl und 5-10-gliedrigem Heteroaryl oder zwei R^{ff}-Gruppen verbunden sind, um einen 3-10-gliedrigen Heterocyclyl- oder 5-10-gliedrigen Heteroarylring zu bilden, wobei jedes von dem Alkyl, Alkenyl, Alkinyl, Heteroalkyl, Heteroalkenyl, Heteroalkinyl, Carbocyclyl, Heterocyclyl, Aryl und Heteroaryl unabhängig mit 0, 1, 2, 3, 4 oder 5 R^{gg}-Gruppe substituiert ist;
jede Instanz von R^{gg} unabhängig Folgendes ist, Halogen, -CN, -NO₂, -N₃, -SO₂H, -SO₃H, -OH, -OC₁₋₆-Alkyl, -ON(C₁₋₆-Alkyl)₂, -N(C₁₋₆-Alkyl)₂, -N(C₁₋₆-Alkyl)₃⁺X⁻, -NH(C₁₋₆-Alkyl)₂⁺X⁻, -NH₂(C₁₋₆-Alkyl)⁺X⁻, -NH₃⁺X⁻, -N(OC₁₋₆-Alkyl)(C₁₋₆-Alkyl), -N(OH)(C₁₋₆-Alkyl), -NH(OH), - SH, -SC₁₋₆-Alkyl, -SS(C₁₋₆-Alkyl), -C(=O)(C₁₋₆-Alkyl), -CO₂H, -CO₂(C₁₋₆-Alkyl), -OC(=O)(C₁₋₆-Alkyl), -OCO₂(C₁₋₆alkyl), -C(=O)NH₂, -C(-O)N(C₁₋₆-Alkyl)₂, -OC(=O)NH(C₁₋₆-Alkyl), - NHC(=O)(C₁₋₆-Alkyl), -N(C₁₋₆-Alkyl)C(=O)(C₁₋₆-Alkyl), -NHCO₂(C₁₋₆-Alkyl), -NHC(=O)N(C₁₋₆-Alkyl)₂, -NHC(=O)NH(C₁₋₆-Alkyl), -NHC(=O)NH₂, -C(=NH)O(C₁₋₆-Alkyl),-OC(=NH)(C₁₋₆-Alkyl), -OC(=NH)OC₁₋₆-Alkyl, -C(=NH)N(C₁₋₆-Alkyl)₂, -C(=NH)NH(C₁₋₆-Alkyl), -C(=NH)NH₂, -OC(=NH)N(C₁₋₆-Alkyl)₂, -OC(NH)NH(C₁₋₆-Alkyl), -OC(NH)NH₂, -NHC(NH)N(C₁₋₆-Alkyl)₂, - NHC(=NH)NH₂, -NHSO₂(C₁₋₆-Alkyl), -SO₂N(C₁₋₆-Alkyl)₂, -SO₂NH(C₁₋₆-Alkyl), -SO₂NH₂-SO₂C₁₋₆-Alkyl, -SO₂OC₁₋₆-Alkyl, -OSO₂C₁₋₆-Alkyl, -SOC₁₋₆-Alkyl, -Si(C₁₋₆-Alkyl)₃, -OSi(C₁₋₆-Alkyl)₃ -C(=S)N(C₁₋₆-Alkyl)₂, C(=S)NH(C₁₋₆-Alkyl), C(=S)NH₂, -C(=O)S(C₁₋₆-Alkyl), - C(=S)SC₁₋₆-Alkyl, -SC(=S)SC₁₋₆-Alkyl, -P(=O)₂(C₁₋₆-Alkyl), -P(=O)(C₁₋₆-Alkyl)₂, -OP(=O)(C₁₋₆-Alkyl)₂, -OP(=O)(OC₁₋₆-Alkyl)₂, C₁₋₆-Alkyl, C₁₋₆-Perhaloalkyl, C₂₋₆-Alkenyl, C₂₋₆-Alkynyl, Hetero-C₁₋₆-alkyl, Hetero-C₂₋₆-alkenyl, Hetero-C₂₋₆-alkynyl, C₃₋₁₀-Carbocyclyl, C₆₋₁₀-Aryl, 3-10-gliedriges Heterocyclyl oder 5- 10-gliedriges Heteroaryl; oder zwei geminale R^{gg} -Substituenten verbunden sind, um =O oder =S zu bilden;
und X⁻ ein Gegenion ist.

2. Verbindung nach Anspruch 1 oder pharmazeutisch unbedenkliches Salz, Solvat, Hydrat, Polymorph, Co-Kristall, Tautomer oder Stereoisomer davon, wobei R^{A} Methyl ist.

3. Verbindung nach Anspruch 1 oder 2, wobei die Verbindung die folgende Formel aufweist: 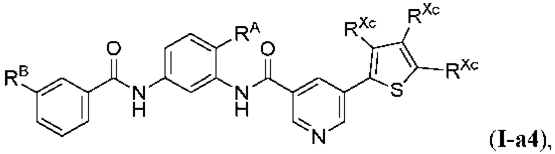 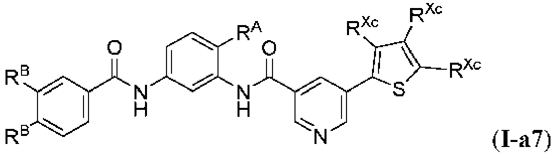 oder 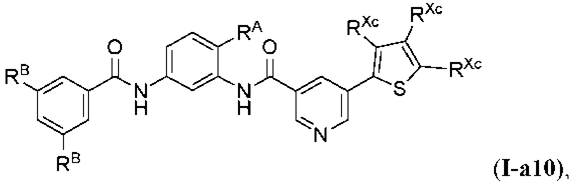 oder pharmazeutisch unbedenkliches Salz, Solvat, Hydrat, Polymorph, Co-Kristall, Tautomer oder Stereoisomer davon.

4. Verbindung nach Anspruch 1 oder 2, wobei die Verbindung die folgende Formel aufweist: 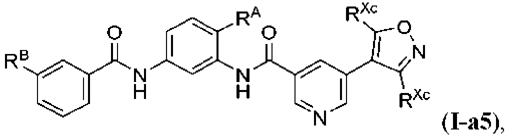 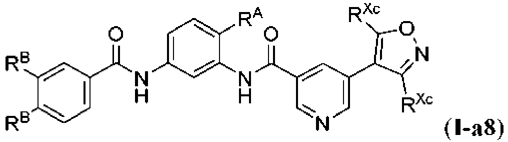 oder 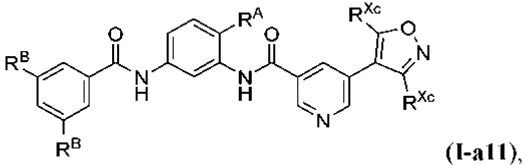 oder pharmazeutisch unbedenkliches Salz, Solvat, Hydrat, Polymorph, Co-Kristall, Tautomer oder Stereoisomer davon.

5. Verbindung nach Anspruch 1 oder 2, wobei die Verbindung die folgende Formel aufweist: 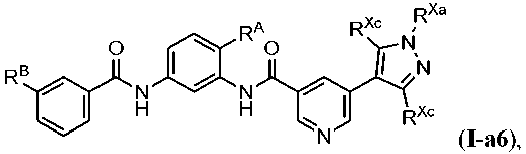 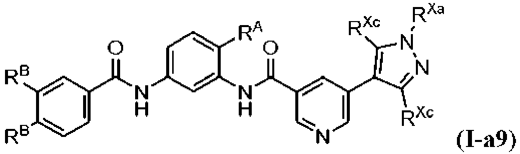 oder 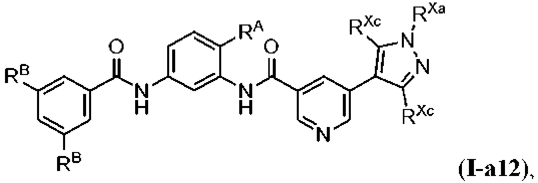 oder pharmazeutisch unbedenkliches Salz, Solvat, Hydrat, Polymorph, Co-Kristall, Tautomer oder Stereoisomer davon.

6. Verbindung nach Anspruch 1 oder 2 oder pharmazeutisch unbedenkliches Salz, Solvat, Hydrat, Polymorph, Co-Kristall, Tautomer oder Stereoisomer davon, wobei l 1 ist und R^{B} *meta zu* dem Anknüpfungspunkt des Amidlinkers ist; oder
wobei l 2 ist und zwei R^{B}-Gruppen *meta* zu dem Anknüpfungspunkt des Amidlinkers sind oder eine R^{B}-Gruppe *meta* zu dem Anknüpfungspunkt des Amidlinkers ist und die zweite R^{B}-Gruppe *para* zu dem Anknüpfungspunkt des Amidlinkers ist.

7. Verbindung nach einem der Ansprüche 1-6 oder pharmazeutisch unbedenkliches Salz, Solvat, Hydrat, Polymorph, Co-Kristall, Tautomer oder Stereoisomer davon, wobei eine R^{B}-Gruppe substituiertes oder unsubstituiertes C₁₋₆-Alkyl ist.

8. Verbindung nach Anspruch 7 oder pharmazeutisch unbedenkliches Salz, Solvat, Hydrat, Polymorph, Co-Kristall, Tautomer oder Stereoisomer davon, wobei eine R^{B}-Gruppe C₁₋₆-Alkyl ist, das mit einer -CN-Gruppe substituiert ist, bevorzugt
wobei eine R^{B}-Gruppe 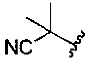 ist oder
wobei eine R^{B}-Gruppe substituiertes oder unsubstituiertes -CH₂-(Piperazinyl) ist, bevorzugt
wobei eine R^{B}-Gruppe 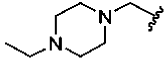 ist oder
wobei eine R^{B}-Gruppe Halogenalkyl ist, bevorzugt
wobei eine R^{B}-Gruppe -CF₃ ist.

9. Verbindung nach einem der Ansprüche 1-6 oder pharmazeutisch unbedenkliches Salz, Solvat, Hydrat, Polymorph, Co-Kristall, Tautomer oder Stereoisomer davon, wobei eine R^{B}-Gruppe substituiertes oder unsubstituiertes Imidazolyl, bevorzugt
wobei eine R^{B}-Gruppe 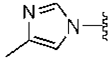 ist.

10. Verbindung nach einem der Ansprüche 1-6 oder pharmazeutisch unbedenkliches Salz, Solvat, Hydrat, Polymorph, Co-Kristall, Tautomer oder Stereoisomer davon, wobei eine R^{B}-Gruppe substituiertes oder unsubstituiertes Piperazinyl ist, bevorzugt
wobei eine R^{B}-Gruppe 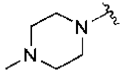 oder 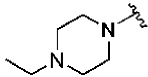 ist oder
wobei eine R^{B}-Gruppe substituiertes oder unsubstituiertes Morpholin ist oder
wobei zwei R^{B}-Gruppen substituiertes oder unsubstituiertes Morpholin sind.

11. Verbindung nach einem der Ansprüche 1, 2 und 5-10 oder pharmazeutisch unbedenkliches Salz, Solvat, Hydrat, Polymorph, Co-Kristall, Tautomer oder Stereoisomer davon, wobei R^{Xa} Methyl oder Ethyl ist.

12. Verbindung nach Anspruch 1, wobei die Verbindung die folgende Formel aufweist: 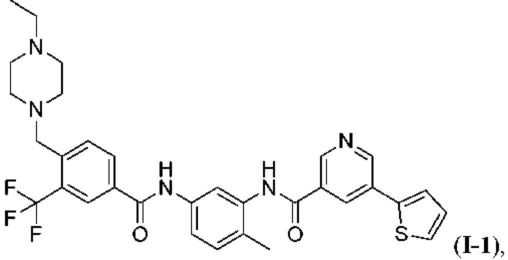 oder pharmazeutisch unbedenkliches Salz davon.

13. Pharmazeutische Zusammensetzung, umfassend eine Verbindung nach einem der Ansprüche 1-12 oder pharmazeutisch unbedenkliches Salz, Solvat, Hydrat, Polymorph, Co-Kristall, Tautomer oder Stereoisomer davon und optional einen pharmazeutisch unbedenklichen Hilfsstoff, wobei die pharmazeutische Zusammensetzung bevorzugt ferner ein zusätzliches pharmazeutisches Mittel umfasst, wobei die pharmazeutische Zusammensetzung stärker bevorzugt ferner ein oder mehrere zusätzliche bekannte chemotherapeutische Mittel umfasst.

14. Verbindung nach einem der Ansprüche 1-12 oder pharmazeutisch unbedenkliches Salz, Solvat, Hydrat, Polymorph, Co-Kristall, Tautomer oder Stereoisomer davon oder pharmazeutische Zusammensetzung nach Anspruch 13 zur Verwendung bei der Behandlung eines B-Zell-Neoplasmas bei einem Subjekt,
wobei die Verbindung oder pharmazeutisch unbedenkliches Salz, Solvat, Hydrat, Polymorph, Co-Kristall, Tautomer oder Stereoisomer davon oder pharmazeutische Zusammensetzung transformierende Wachstumsfaktor-b-aktivierte Kinase-1 (TAK1), hämopoetische Zellkinase (HCK) oder sowohl TAK1 als auch HCK hemmt, stärker bevorzugt
wobei die Verbindung oder ein pharmazeutisch unbedenkliches Salz, Solvat, Hydrat, Polymorph, Co-Kristall, Tautomer oder Stereoisomer davon oder eine pharmazeutische Zusammensetzung die hämatopoetische Vorläuferkinase 1 (HPK1) hemmt, noch bevorzugter
wobei die Verbindung oder ein pharmazeutisch unbedenkliches Salz, Solvat, Hydrat, Polymorph, Co-Kristall, Tautomer oder Stereoisomer davon oder pharmazeutische Zusammensetzung in Kombination mit einem Inhibitor von Brutons Tyrosinkinase (BTK), Interleukin-1-Rezeptor-assoziierter Kinase 1 (IRAK1), Interleukin-1-Rezeptor-assoziierter Kinase 4 (IRAK4), transformierendem Wachstumsfaktor b-aktivierter Kinase-1 (TAK1) oder einer Kinase der Src-Familie, besonders bevorzugt, verabreicht wird
wobei der BTK-Inhibitor ein Benzonaphthyridinon ist und/oder wobei das B-Zell-Neoplasma aus der Gruppe ausgewählt ist, die aus Hodgkin-Lymphomen und den meisten Non-Hodgkin-Lymphomen besteht, bevorzugt
wobei das Lymphom Folgendes ist, Waldenström-Makroglobulinämie, diffuses großzelliges B-Zelllymphom, follikuläres Lymphom, Mucosa-Associated Lymphatic Tissue Lymphoma (MALT), kleinzelliges lymphatisches Lymphom (Überlappungen mit chronischer lymphatischer Leukämie), Mantelzelllymphom (MCL), Burkitt-Lymphom, mediastinales großzelliges B-Zelllymphom, nodales Randzonen-B-Zelllymphom (NMZL), Milzrandzonenlymphom (SMZL), intravaskuläres großzelliges B-Zelllymphom, primäres Ergusslymphom und lymphomatoide Granulomatose, besonders bevorzugt
wobei das B-Zell-Neoplasma Waldenström-Makroglobulinämie ist, noch bevorzugter
wobei das Subjekt eine Mutation an Position 38182641 im Chromosom 3p22.2 aufweist und/oder
wobei das Subjekt Therapie für das B-Zell-Neoplasma erhält und/oder
wobei die Verbindung oder das pharmazeutisch unbedenkliche Salz, Solvat, Hydrat, Polymorph, Co-Kristall, Tautomer oder Stereoisomer davon oder die pharmazeutische Zusammensetzung zusammen mit einem oder mehreren anderen chemotherapeutischen Mitteln verabreicht wird.

15. Verbindung, pharmazeutisch unbedenkliches Salz, Solvat, Hydrat, Polymorph, Co-Kristall, Tautomer, Stereoisomer oder pharmazeutische Zusammensetzung zur Verwendung nach Anspruch 14, wobei das B-Zell-Neoplasma Waldenström-Makroglobulinämie ist.

## Revendications

1. Composé de formule **(I-al), (I-a2)** ou **(I-a3)** : 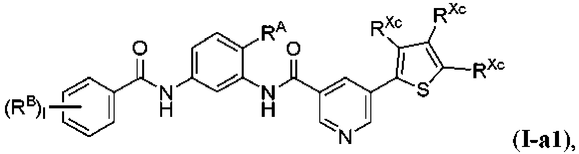 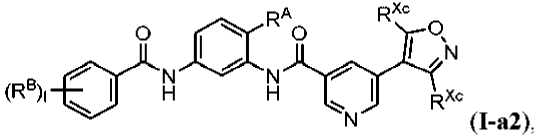 ou 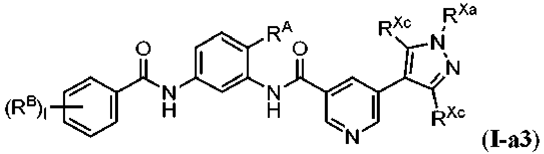
ou sel, solvate, hydrate, polymorphe, co-cristal, tautomère ou stéréoisomère pharmaceutiquement acceptable de celui-ci ;
dans laquelle :
R^{A} représente un groupe C₁₋₆ alkyle substitué ou non substitué ;
chaque instance de R^{B} est indépendamment choisie dans le groupe constitué par : les groupes alkyle éventuellement substitué, hétérocyclyle éventuellement substitué et hétéroaryle éventuellement substitué ;
R^{Xa} représente un groupe C₁₋₆ alkyle éventuellement substitué ;
chaque instance de R^{Xc} représente un atome d'hydrogène ; et
l vaut 1 ou 2 ;
les substituants d'atomes de carbone sont choisis indépendamment dans le groupe constitué par un atome d'halogène, les groupes -CN, -NO₂, -N₃, -SO₂H, -SO₃H, -OH, -OR^{aa}, -ON(R^{bb})₂, - N(R^{bb})₂, -N(R^{bb})₃⁺X⁻ -N(OR^{cc})R^{bb}, -SH, -SR^{aa}, -SSR^{cc}, -C(=O)R^{aa}, -CO₂H, -CHO, -C(OR^{cc})₂, - CO₂R^{aa}, -OC(=O)R^{aa}, -OCO₂R^{aa}, -C(=O)N(R^{bb})₂, -OC(=O)N(R^{bb})₂, -NR^{bb}C(=O)R^{aa}, - NR^{bb}CO₂R^{aa}, -NR^{bb}C(=O)N(R^{bb})₂, -C(=NR^{bb})R^{aa}, -C(=NR^{bb})OR^{aa}, -OC(=NR^{bb})R^{aa}, - OC(=NR^{bb})OR^{aa}, -C(=NR^{bb})N(R^{bb})₂, -OC(=NR^{bb})N(R^{bb})₂, -NR^{bb}C(=NR^{bb})N(R^{bb})₂, - C(=O)NR^{bb}SO₂R^{aa}, -NR^{bb}SO₂R^{aa}, -SO₂N(R^{bb})₂, -SO₂R^{aa}, -SO₂OR^{aa}, -OSO₂R^{aa}, -S(=O)R^{aa}, - OS(=O)R^{aa}, -Si(R^{aa})₃, -OSi(R^{aa})₃ -C(=S)N(R^{bb})₂, -C(=O)SR^{aa}, -C(=S)SR^{aa}, -SC(=S)SR^{aa}, - SC(=O)SR^{aa}, -OC(=O)SR^{aa}, -SC(=O)OR^{aa}, -SC(=O)R^{aa}, -P(=O)₂R^{aa}, -OP(=O)₂R^{aa}, -P(=O)(R^{aa})₂, -OP(=O)(R^{aa})₂, -OP(=O)(OR^{CC})₂, -P(=O)₂N(R^{bb})₂, -OP(=O)₂N(R^{bb})₂, -P(=O)(NR^{bb})₂, -OP (=O)(NR^{bb})₂, -NR^{bb}P(=O)(OR^{cc})₂, -NR^{bb}P(=O)(NR^{bb})₂, -P(R^{cc})₂, -P(R^{cc})₃, -OP(R^{cc})₂, -OP(R^{cc})₃, - B(R^{aa})₂, -B(OR^{cc})₂, -BR^{aa}(OR^{cc}), C₁₋₁₀ alkyle, C₁₋₁₀ perhalogénoalkyle, C₂₋₁₀ alcényle, C₂₋₁₀ alcynyle, hétéroC₁₋₁₀ alkyle, hétéroC₂₋₁₀ alcényle, hétéroC₂₋₁₀ alcynyle, C₃₋₁₀ carbocyclyle, hétérocyclyle de 3 à 14 chaînons, C₆₋₁₄ aryle et hétéroaryle de 5 à 14 chaînons, chaque groupe alkyle, alcényle, alcynyle, hétéroalkyle, hétéroalcényle, hétéroalcynyle, carbocyclyle, hétérocyclyle, aryle et hétéroaryle étant indépendamment substitué par 0, 1, 2, 3, 4 ou 5 groupes R^{dd} ;
ou deux atomes d'hydrogène géminés sur un atome de carbone sont remplacés par le groupe =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb} ou =NOR^{cc} ;
les substituants d'atomes d'azote sont des groupes protecteurs d'azote ;
chaque instance du groupe protecteur d'azote représente indépendamment un groupe -OH, -OR^{aa}, -N(R^{cc})₂, -C(=O)R^{aa}, -C(=O)N(R^{cc})₂, -CO₂R^{aa}, -SO₂R^{aa}, -C(=NR^{cc})R^{aa}, -C(=NR^{cc})OR^{aa}, - C(=NR^{cc})N(R^{cc})₂, -SO₂N(R^{CC})₂, -SO₂R^{CC}, -SO₂OR^{CC}, -SOR^{aa}, -C(=S)N(R^{cc})₂, -C(=O)SR^{cc}, - C(=S)SR^{cc}, C₁₋₁₀ alkyle, C₂₋₁₀ alcényle, C₂₋₁₀ alcynyle, C₁₋₁₀ hétéroalkyle, C₂₋₁₀ hétéroalcényle, C₂₋₁₀ hétéroalcynyle, C₃₋₁₀ carbocyclyle, hétérocyclyle de 3 à 14 chaînons, C₆₋₁₄ aryle, ou hétéroaryle de 5 à 14 chaînons, chacun des groupes alkyle, alcényle, alcynyle, hétéroalkyle, hétéroalcényle, hétéroalcynyle, carbocyclyle, hétérocyclyle, aralkyle, aryle, et hétéroaryle étant indépendamment substitué par 0, 1, 2, 3, 4 ou 5 groupes R^{dd} ;
chaque instance de R^{aa} est, indépendamment, choisie parmi un groupe C₁₋₁₀ alkyle, C₁₋₁₀ perhalogénoalkyle, C₂₋₁₀ alcényle, C₂₋₁₀ alcynyle, hétéroC₁₋₁₀ alkyle, hétéroC₂₋₁₀ alcényle, hétéroC₂₋₁₀ alcynyle, C₃₋₁₀ carbocyclyle, hétérocyclyle de 3 à 14 chaînons, C₆₋₁₄ aryle et hétéroaryle de 5 à 14 chaînons, ou deux groupes R^{aa} sont joints pour former un noyau hétérocyclyle de 3 à 14 chaînons ou hétéroaryle de 5 à 14 chaînons, chacun des groupes alkyle, alcényle, alcynyle, hétéroalkyle, hétéroalcényle, hétéroalcynyle, carbocyclyle, hétérocyclyle, aryle et hétéroaryle étant indépendamment substitué par 0, 1, 2, 3, 4 ou 5 groupes R^{dd} ;
chaque instance de R^{bb} est, indépendamment, choisie parmi un atome d'hydrogène, les groupes -OH, -OR^{aa}, -N(R^{cc})₂, -CN, -C(=O)R^{aa}, -C(=O)N(R^{cc})₂, -CO₂R^{aa}, -SO₂R^{aa}, - C(=NR^{cc})OR^{aa}, -C(=NR^{cc})N(R^{cc})₂, -SO₂N(R^{CC})₂, -SO₂R^{CC}, -SO₂OR^{CC}, -SOR^{aa}, -C(=S)N(R^{cc})₂, - C(=O)SR^{cc}, -C(=S)SR^{ee}, -P(=O)₂R^{aa}, -P(=O)(R^{aa})₂, -P(=O)₂N(R^{ee})₂, -P(=O)(NR^{ee})₂, C₁₋₁₀ alkyle, C₁₋₁₀ perhalogénoalkyle, C₂₋₁₀ alcényle, C₂₋₁₀ alcynyle, hétéroC₁₋₁₀ alkyle, hétéroC₂₋₁₀ alcényle, hétéroC₂₋₁₀ alcynyle, C₃₋₁₀ carbocyclyle, hétérocyclyle de 3 à 14 chaînons, C₆₋₁₄ aryle et hétéroaryle de 5 à 14 chaînons, ou deux groupes R^{bb} sont joints pour former un noyau hétérocyclyle de 3 à 14 chaînons ou hétéroaryle de 5 à 14 chaînons, chacun des groupes alkyle, alcényle, alcynyle, hétéroalkyle, hétéroalcényle, hétéroalcynyle, carbocyclyle, hétérocyclyle, aryle et hétéroaryle étant indépendamment substitué par 0, 1, 2, 3, 4 ou 5 groupes R^{dd} ;
chaque instance de R^{cc} est, indépendamment, choisie parmi un atome d'hydrogène, un groupe C₁₋₁₀ alkyle, C₁₋₁₀ perhalogénoalkyle, C₂₋₁₀ alcényle, C₂₋₁₀ alcynyle, hétéroC₁₋₁₀ alkyle, hétéroC₂₋₁₀ alcényle, hétéroC₂₋₁₀ alcynyle, C₃₋₁₀ carbocyclyle, hétérocyclyle de 3 à 14 chaînons, C₆₋₁₄ aryle et hétéroaryle de 5 à 14 chaînons, ou deux groupes R^{cc} sont joints pour former un noyau hétérocyclyle de 3 à 14 chaînons ou hétéroaryle de 5 à 14 chaînons, chacun des groupes alkyle, alcényle, alcynyle, hétéroalkyle, hétéroalcényle, hétéroalcynyle, carbocyclyle, hétérocyclyle, aryle et hétéroaryle étant indépendamment substitué par 0, 1, 2, 3, 4 ou 5 groupes R^{dd} ;
chaque instance de R^{dd} est, indépendamment, choisie parmi un atome d'halogène, les groupes -CN, -NO₂, -N₃, -SO₂H, -SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, -SSR^{ee}, -C(=O)R^{ee}, -CO₂H, -CO₂R^{ee}, -OC(=O)R^{ee}, -OCO₂R^{ee}, -C(=O)N(R^{ff})₂, - OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee}, - OC(=NR^{ff})R^{ee}, -OC(=NR^{ff})OR^{ee}, -C(=NR^{ff})N(R^{ff})₂, -OC(=NR^{ff})N(R^{ff})₂, -NR^{ff}C(=NR^{ff})N(R^{ff})₂, - NR^{ff}SO₂R^{ee}, -SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, - C(=S)N(R^{ff})₂, -C(=O)SR^{ee}, -C(=S)SR^{ee}, -SC(=S)SR^{ee}, -P(=O)₂R^{ee}, -P(=O)(R^{ee})₂, -OP(=O)(R^{ee})₂, - OP(=O)(OR^{ee})₂, C₁₋₆ alkyle, C₁₋₆ perhalogénoalkyle, C₂₋₆ alcényle, C₂₋₆ alcynyle, hétéroC₁₋₆alkyle, hétéroC₂₋₆alcényle, hétéroC₂₋₆ alcynyle, C₃₋₁₀ carbocyclyle, hétérocyclyle de 3 à 10 chaînons, C₆₋₁₀ aryle et hétéroaryle de 5 à 10 chaînons, chacun des substituants alkyle, alcényle, alcynyle, hétéroalkyle, hétéroalcényle, hétéroalcynyle, carbocyclyle, hétérocyclyle, aryle et hétéroaryle étant indépendamment substitué par 0, 1, 2, 3, 4 ou 5 groupes R^{gg}, ou deux substituants R^{dd} géminaux sont joints pour former un groupe =O ou =S ;
chaque instance de R^{ee} est, indépendamment, choisie parmi les groupes C₁₋₆ alkyle, C₁₋₆ perhalogénoalkyle, C₂₋₆ alcényle, C₂₋₆ alcynyle, hétéroC₁₋₆ alkyle, hétéroC₂₋₆ alcényle, hétéroC₂₋₆ alcynyle, C₃₋₁₀ carbocyclyle, C₆₋₁₀ aryle, hétérocyclyle de 3 à 10 chaînons et hétéroaryle de 3 à 10 chaînons, chacun des groupes alkyle, alcényle, alcynyle, hétéroalkyle, hétéroalcényle, hétéroalcynyle, carbocyclyle, hétérocyclyle, aryle et hétéroaryle étant indépendamment substitué par 0, 1, 2, 3, 4 ou 5 groupes R^{gg} ;
chaque instance de R^{ff} est, indépendamment, choisie parmi un atome d'hydrogène, un groupe C₁₋₆ alkyle, C₁₋₆ perhalogénoalkyle, C₂₋₆ alcényle, C₂₋₆ alcynyle, hétéroC₁₋₆ alkyle, hétéroC₂₋₆ alcényle, hétéroC₂₋₆ alcynyle, C₃₋₁₀ carbocyclyle, hétérocyclyle de 3 à 10 chaînons, C₆₋₁₀ aryle et hétéroaryle de 5 à 10 chaînons, ou deux groupes R^{ff} sont joints pour former un noyau hétérocyclyle de 3 à 10 chaînons ou hétéroaryle de 5 à 10 chaînons, chacun des groupes alkyle, alcényle, alcynyle, hétéroalkyle, hétéroalcényle, hétéroalcynyle, carbocyclyle, hétérocyclyle, aryle et hétéroaryle étant indépendamment substitué par 0, 1, 2, 3, 4 ou 5 groupes R^{gg} ;
chaque instance de R^{gg} représente, indépendamment, un atomes d'halogène, les groupes - CN, -NO₂, -N₃, -SO₂H, -SO₃H, -OH, -OC₁₋₆ alkyle, -ON(C₁₋₁₀ alkyle)₂, -N(C₁₋₆ alkyle)₂, -N(C₁₋₆ alkyle)₃⁺X⁻, -NH(C₁₋₆ alkyle)₂⁺X⁻, -NH₂(C₁₋₆ alkyle)⁺X⁻, -NH₃X⁻, -N(OC₁₋₆ alkyle)(C₁₋₆ alkyle), - N(OH)(C₁₋₆ alkyle), -NH(OH), -SH, -SC₁₋₆ alkyle, -SS(C₁₋₆ alkyle), -C(=O)(C₁₋₆ alkyle), -CO₂H, -CO₂(C₁₋₆ alkyle), -OC(=O)(C₁₋₆ alkyle), -OCO₂(C₁₋₆ alkyle), -C(=O)NH₂, -C(=O)N(C₁₋₆alkyle)₂, -OC(=O)NH(C₁₋₆ alkyle), -NHC(=O)(C₁₋₆ alkyle), -N(C₁₋₆ alkyle)C(=O)(C₁₋₆ alkyle), -NHCO₂(C₁₋₆ alkyle), -NHC(=O)N(C₁₋₆ alkyle)₂, -NHC(=O)NH(C₁₋₆ alkyle), -NHC(=O)NH₂, -C(=NH)O(C₁₋₆ alkyle),-OC(=NH)(C₁₋₆ alkyle), -OC(=NH)OC₁₋₆ alkyle, -C(=NH)N(C₁₋₆ alkyle)₂, - C(=NH)NH(C₁₋₆ alkyle), -C(=NH)NH₂, -OC(=NH)N(C₁₋₆alkyle)₂, -OC(NH)NH(C₁₋₆ alkyle), - OC(NH)NH₂, -NHC(NH)N(C₁₋₆ alkyle)₂, -NHC(=NH)NH₂, -NHSO₂(C₁₋₆ alkyle), -SO₂N(C₁₋₆ alkyle)₂, -SO₂NH(C₁₋₆ alkyle), -SO₂NH₂-SO₂C₁₋₆ alkyle, -SO₂OC₁₋₆ alkyle, - OSO₂C₁₋₆ alkyle, - SOC₁₋₆ alkyle, -Si(C₁₋₆ alkyle)₃, -OSi(C₁₋₆ alkyle)₃ -C(=S)N(C₁₋₆ alkyle)₂, C(=S)NH(C₁₋₆ alkyle), C(=S)NH₂, -C(=O)S(C₁₋₆alkyle), -C(=S)SC₁₋₆alkyle, -SC(=S)SC₁₋₆ alkyle, -P(=O)₂(C₁₋₆ alkyle), - P(=O)(C₁₋₆ alkyle)₂, -OP(=O)(C₁₋₆ alkyle)₂, -OP(=O)(OC₁₋₆ alkyle)₂, C₁₋₆ alkyle, C₁₋₆ perhalogénoalkyle, C₂₋₆ alcényle, C₂₋₆ alcynyle, hétéroC₁₋₆ alkyle, hétéroC₂₋₆ alcényle, hétéroC₂₋₆ alcynyle, C₃₋₁₀ carbocyclyle, C₆₋₁₀ aryle, hétérocyclyle de 3 à 10 chaînons ou hétéroaryle de 5 à 10 chaînons ; ou deux substituants R^{gg} géminaux sont joints pour former un groupe =O ou =S ; et X⁻ représente un contre-ion.

2. Composé selon la revendication 1, ou sel, solvate, hydrate, polymorphe, co-cristal, tautomère ou stéréoisomère pharmaceutiquement acceptable de celui-ci, dans lequel R^{A} représente un groupe méthyle.

3. Composé selon la revendication 1 ou 2, dans lequel le composé répond à la formule : 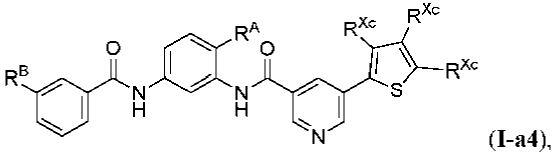 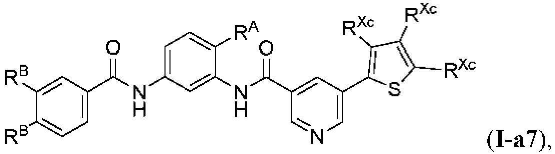 ou 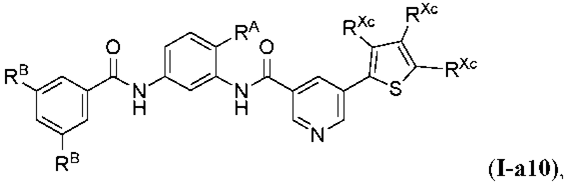 ou sel, solvate, hydrate, polymorphe, co-cristal, tautomère ou stéréoisomère pharmaceutiquement acceptable de celui-ci.

4. Composé selon la revendication 1 ou 2, ledit composé répondant à la formule : 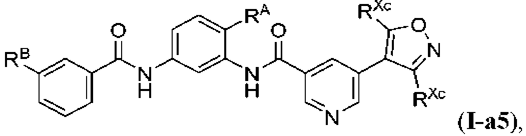 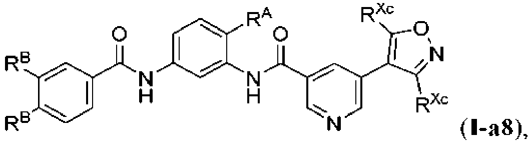 ou 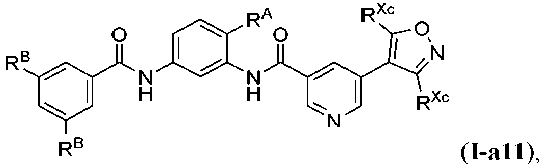 ou sel, solvate, hydrate, polymorphe, co-cristal, tautomère ou stéréoisomère pharmaceutiquement acceptable de celui-ci.

5. Composé selon la revendication 1 ou 2, ledit composé répondant à la formule : 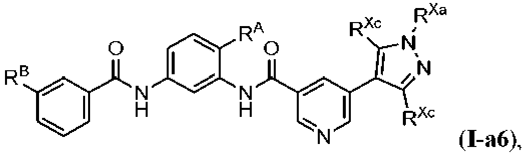 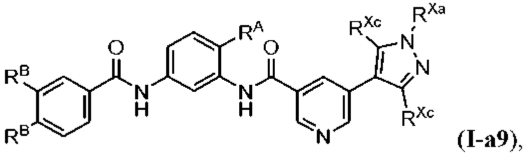 ou 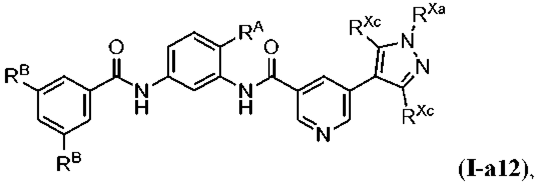 ou sel, solvate, hydrate, polymorphe, co-cristal, tautomère ou stéréoisomère pharmaceutiquement acceptable de celui-ci.

6. Composé selon la revendication 1 ou 2, ou sel, solvate, hydrate, polymorphe, co-cristal, tautomère ou stéréoisomère pharmaceutiquement acceptable de celui-ci, dans lequel 1 vaut 1 et R^{B} représente ***méta*** au point de fixation du lieur amide ; ou
dans lequel l vaut 2, et deux groupes R^{B} sont ***méta*** au point de fixation du lieur amide, ou un groupe R^{B} est ***méta*** au point de fixation du lieur amide, et le second groupe R^{B} est ***para*** au point de fixation du lieur amide.

7. Composé selon l'une quelconque des revendications 1 à 6, ou sel, solvate, hydrate, polymorphe, co-cristal, tautomère ou stéréoisomère pharmaceutiquement acceptable de celui-ci, dans lequel un groupe R^{B} représente un groupe C₁₋₆ alkyle substitué ou non substitué.

8. Composé selon la revendication 7, ou sel, solvate, hydrate, polymorphe, co-cristal, tautomère ou stéréoisomère pharmaceutiquement acceptable de celui-ci, dans lequel un groupe R^{B} représente un groupe C₁₋₆ alkyle substitué par un groupe -CN, de préférence
dans lequel un groupe R^{B} représente 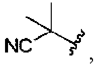 ou
dans lequel un groupe R^{B} représente un groupe CH₂-(pipérazinyle) substitué ou non substitué, de préférence
dans lequel un groupe R^{B} représente 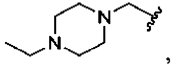 ou
dans lequel un groupe R^{B} représente un groupe halogénoalkyle, de préférence
dans lequel un groupe R^{B} représente un groupe -CF₃.

9. Composé selon l'une quelconque des revendications 1 à 6, ou sel, solvate, hydrate, polymorphe, co-cristal, tautomère ou stéréoisomère pharmaceutiquement acceptable de celui-ci, dans lequel un groupe R^{B} représente un groupe imidazolyle substitué ou non substitué
dans lequel un groupe R^{B} représente

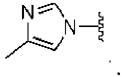

10. Composé selon l'une quelconque des revendications 1 à 6, ou sel, solvate, hydrate, polymorphe, co-cristal, tautomère ou stéréoisomère pharmaceutiquement acceptable de celui-ci, dans lequel un groupe R^{B} représente un groupe pipérazinyle substitué ou non substitué, de préférence
dans lequel un groupe R^{B} représente 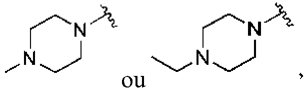 ou
dans lequel un groupe R^{B} représente un groupe morpholine substitué ou non substitué, ou
dans lequel deux groupes R^{B} représentent un groupe morpholine substitué ou non substitué.

11. Composé selon l'une quelconque des revendications 1, 2 et 5 à 10, ou sel, solvate, hydrate, polymorphe, co-cristal, tautomère ou stéréoisomère pharmaceutiquement acceptable de celui-ci,
dans lequel R^{Xa} représente un groupe méthyle ou éthyle.

12. Composé selon la revendication 1, dans lequel le composé répond à la formule : 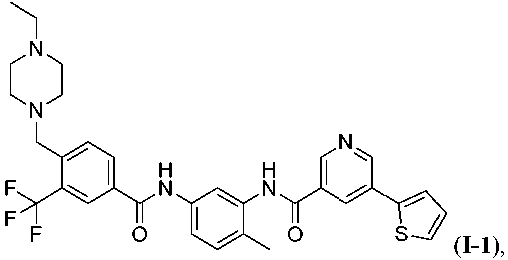 ou sel pharmaceutiquement acceptable de celui-ci.

13. Composition pharmaceutique comprenant un composé selon l'une quelconque des revendications 1 à 12, ou sel, solvate, hydrate, polymorphe, co-cristal, tautomère ou stéréoisomère pharmaceutiquement acceptable de celui-ci, et éventuellement un excipient pharmaceutiquement acceptable, de préférence ladite composition pharmaceutique comprenant en outre un agent pharmaceutique supplémentaire, plus préférablement ladite composition pharmaceutique comprenant en outre un ou plusieurs agents chimiothérapeutiques connus supplémentaires.

14. Composé selon l'une quelconque des revendications 1 à 12, ou sel, solvate, hydrate, polymorphe, co-cristal, tautomère ou stéréoisomère pharmaceutiquement acceptable de celui-ci, ou composition pharmaceutique selon la revendication 13, destiné à être utilisé dans le traitement d'une tumeur des lymphocytes B chez un sujet,
de préférence ledit composé, ou un sel, solvate, hydrate, polymorphe, co-cristal, tautomère ou stéréoisomère pharmaceutiquement acceptable de celui-ci, ou une composition pharmaceutique inhibant la kinase-1 activée par le facteur de croissance transformant b (TAK1), la kinase des cellules hématopoïétiques (HCK), ou à la fois TAK1 et HCK, plus préférablement
ledit composé, ou un sel, solvate, hydrate, polymorphe, co-cristal, tautomère ou stéréoisomère pharmaceutiquement acceptable de celui-ci, ou une composition pharmaceutique inhibant la kinase progénitrice hématopoïétique 1 (HPK1), encore plus préférablement
ledit composé, ou un sel, solvate, hydrate, polymorphe, co-cristal, tautomère ou stéréoisomère pharmaceutiquement acceptable de celui-ci, ou une composition pharmaceutique étant administré en combinaison avec un inhibiteur de la tyrosine kinase de Bruton (BTK), de la kinase 1 associée au récepteur de l'interleukine-1 (IRAK1), de la kinase 4 associée au récepteur de l'interleukine-1 (IRAK4), de la kinase-1 activée par le facteur de croissance transformant b (TAK1) ou d'une kinase de la famille Src, de préférence
ledit inhibiteur de la BTK étant une benzonaphtyridinone, et/ou adite tumeur des lymphocytes B étant choisie dans le groupe constitué par un lymphome hodgkinien et la plupart des lymphomes non hodgkiniens, de préférence
ledit lymphome étant la macroglobulinémie de Waldenström, le lymphome diffus à grandes lymphocytes B, le lymphome folliculaire, le lymphome du tissu lymphatique associé à la muqueuse (MALT), le lymphome lymphocytaire à petites cellules (coïncidant avec la leucémie lymphocytaire chronique), le lymphome à cellules du manteau (MCL), le lymphome de Burkitt, le lymphome à grands lymphocytes B médiastinal, le lymphome à lymphocytes B de la zone marginale ganglionnaire (NMZL), le lymphome splénique de la zone marginale (SMZL), le lymphome à grands lymphocytes B intravasculaire, le lymphome à épanchement primaire et la granulomatose lymphomatoïde, plus préférablement
ladite tumeur à lymphocytes B étant la macroglobulinémie de Waldenström, encore plus préférablement
ledit sujet présentant une mutation en position 38182641 dans le chromosome 3p22.2, et/ou
ledit sujet recevant un traitement pour la tumeur à lymphocytes B, et/ou
ledit composé, ou un sel, solvate, hydrate, polymorphe, co-cristal, tautomère ou stéréoisomère pharmaceutiquement acceptable de celui-ci, ou une composition pharmaceutique étant co-administré avec un ou plusieurs autres agents chimiothérapeutiques.

15. Composé, sel, solvate, hydrate, polymorphe, co-cristal, tautomère, stéréoisomère pharmaceutiquement acceptable ou composition pharmaceutique destiné à être utilisé selon la revendication 14, ladite tumeur à lymphocytes B étant la macroglobulinémie de Waldenström.
